(19) Europäisches Patentamt
European Patent Office
Office européen des brevets

(11) EP 1 460 062 A1

(12) **EUROPEAN PATENT APPLICATION**
published in accordance with Art. 158(3) EPC

(43) Date of publication:
**22.09.2004 Bulletin 2004/39**

(21) Application number: **02793399.3**

(22) Date of filing: **26.12.2002**

(51) Int Cl.[7]: **C07D 207/24**

(86) International application number:
**PCT/JP2002/013581**

(87) International publication number:
**WO 2003/057668 (17.07.2003 Gazette 2003/29)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **28.12.2001 JP 2001400051**

(71) Applicant: **Takeda Chemical Industries, Ltd.**
**Osaka-shi, Osaka 541-0045 (JP)**

(72) Inventors:
- **YAMAOKA, Masayoshi**
  **Mino-shi, Osaka 562-0043 (JP)**
- **IKEURA, Yoshinori**
  **Kashiba-shi, Nara 639-0251 (JP)**
- **HASHIMOTO, Tadatoshi**
  **Ibaraki-shi, Osaka 567-0828 (JP)**
- **TARUI, Naoki**
  **Nara-shi, Nara 631-0061 (JP)**

(74) Representative: **von Kreisler, Alek, Dipl.-Chem.**
**Deichmannhaus am Dom,**
**Postfach 10 22 41**
**50462 Köln (DE)**

(54) **NITROGENOUS CYCLIC KETONE DERIVATIVE, PROCESS FOR PRODUCING THE SAME, AND USE**

(57) A novel compound represented by the formula (I):

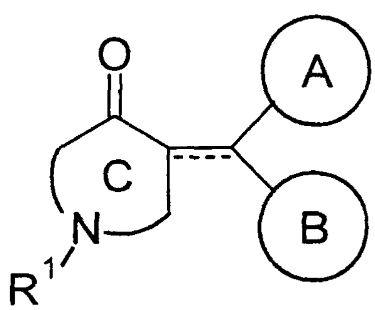

(I)

wherein rings A and B each represents an optionally substituted aromatic ring, or rings A and B may be bonded to each other through linking between bonds or substituents thereof to form a ring; ring C represents a nitrogenous saturated heterocycle optionally having one or more substituents besides the oxo (provided that 2,3-dioxopyrrolidine ring is excluded); $R^1$ represents hydrogen, an optionally substituted hydrocarbon group, or an optionally substituted heterocyclic group; and

--------

indicates a single bond or a double bond. It has high antagonistic activity against a tachykinin receptor, especially an SP receptor.

EP 1 460 062 A1

## Description

Technical Field

The present invention relates to a novel nitrogenous cyclic ketone derivative having excellent tachykinin receptor antagonistic activity, a process for producing the same, and use.

Background Art

Tachykinin is a general name of a series of neuropeptides, and includes, in mammals, substance P (SP), neurokinin-A and neurokinin-B. These peptides are known to exert various physiological activities by binding to respective receptors present in a living body (neurokinin-1, neurokinin-2, neurokinin-3).

Among them, SP is one of the oldest and most extensively investigated neuropeptide, and is a peptide composed of 11 amino acids which was identified in an equine intestinal tract extract for the first time in 1931, and whose structure was determined in 1971.

SP is distributed widely over the central and peripheral nervous systems and, in addition to the function as a primary sensory neuron transmitter, it has various physiological activities such as vasodilating activity, vascular permeability-enhancing activity, smooth muscle contracting activity, neurocyte exciting activity, salivating activity, diuretic activity, immunological activity, and the like. In particular, it is known that SP released from a terminal of a spinal occipital horn by a pain impulse transmits the pain signal to a secondary neuron, and that SP released from a peripheral terminal induces an inflammatory reaction in its receptor. Accordingly, SP is considered to be involved in various disease conditions (for example, pain, headache, especially migraine, Alzheimer's disease, multiple sclerosis, cardiovascular modulation, a chronic inflammatory disease such as rheumatoid arthritis, a respiratory disease such as asthma and allergic rhinitis, bowel inflammatory disease such as ulcerative colitis and Crohn's disease, ocular damage and ocular inflammation, proliferative vitreoretinopathy, irritable bowel syndrome, pollakiuria, psychosis, vomiting and the like) [see Review: Physiological Reviews, Vol.73, p.229-308 (published 1993); Journal of Autonomic Pharmacology, Vol.13, p.23-93 (published 1993)].

Currently, as compounds having SP receptor antagonistic activity, EP-A-436,334 describes a compound represented by the formula:

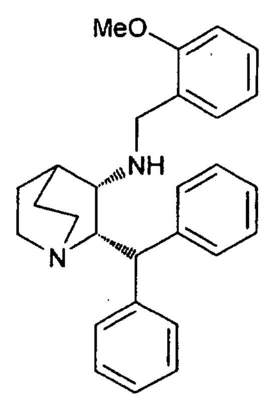

and the like; WO92/17449 describes a compound represented by the formula:

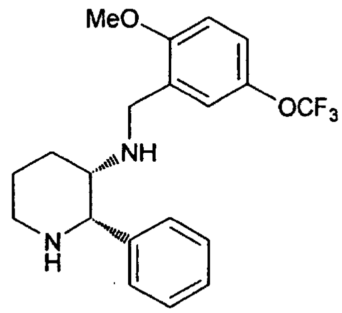

and the like; WO95/16679 describes a compound represented by the formula:

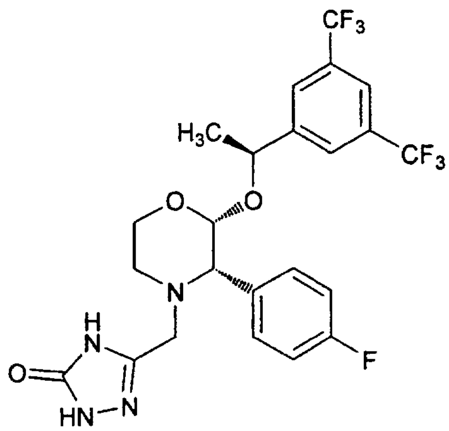

and the like; and JP-A 9-263585 describes a heterocyclic compound represented by the formula:

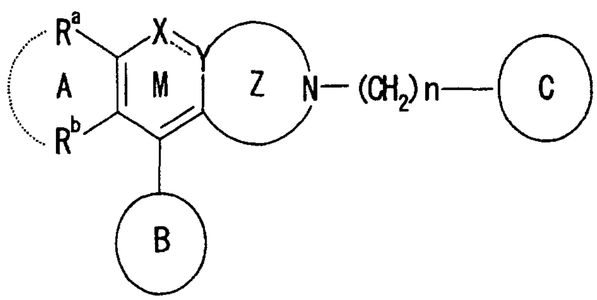

wherein ring M is a heterocyclic ring having -N=C<, -CO-N< or -CS-N< as its partial structure:

-X = Y <

; Ra and Rb are taken together to form ring A, or may be same or different and each is a hydrogen atom or a substituent on ring M; each of rings A and B is an optionally substituted homocyclic or heterocyclic ring, and at least one is an optionally substituted heterocyclic ring; ring C is an optionally substituted homocyclic or heterocyclic ring; ring Z is an optionally substituted nitrogenous heterocyclic ring; and n is an integer of 1 to 6, or a salt thereof, and the like.

Objects of the Invention

Currently, there is no compound found as an agent for treating various disease conditions described above which has excellent tachykinin receptor antagonistic activity (especially SP receptor antagonistic activity) and which is sufficiently satisfactory in terms of the safety and sustained activity. Accordingly, it is desired to develop a compound whose chemical structure is different from those of the above known compounds and which has excellent tachykinin receptor antagonistic activity and can serve as a therapeutic agent capable of exhibiting a sufficiently satisfactory clinical effect.
Accordingly, the objects of the present invention are to provide a novel compound having high tachykinin receptor antagonistic activity, especially SP receptor antagonistic activity, a process for producing the same, etc.
The other objects of the present invention are to provide a pharmaceutical composition having high tachykinin receptor antagonistic activity, especially SP receptor antagonistic activity, a tachykinin receptor antagonist, an abnormal urination improving agent, etc.

Summary of the Invention

In view of the above circumstances, the present inventors have studied intensively. As a result, the present inventors have found that a nitrogenous cyclic ketone derivative represented by the following formula (I) or its salt has unexpectedly potent tachykinin receptor antagonistic activity (especially SP receptor antagonistic activity) based on its characteristic chemical structure, and can serve as a sufficiently satisfactory medicine. The present invention has been completed based on these findings.
That is, the present invention provides:

(1) A compound represented by the formula (I):

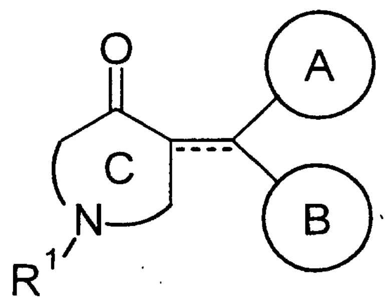

(I)

wherein each of rings A and B represents an optionally substituted aromatic ring, or rings A and B may be bonded to each other through linking between bonds or substituents thereof to form a ring; ring C represents a nitrogenous saturated heterocyclic ring optionally having one or more substituents besides the oxo (provided that 2,3-dioxopyrrolidine ring is excluded); $R^1$ represents a hydrogen atom, an optionally substituted hydrocarbon group or an optionally substituted heterocyclic group; and

--------

represents a single bond or a double bond, or a salt thereof;
(2) The compound according to the above (1), wherein each of rings A and B represents an optionally substituted benzene ring, or the substituents on rings A and B may be taken together to form a dibenzotricyclic ring;
(3) The compound according to the above (1), which is represented by the formula (Ia):

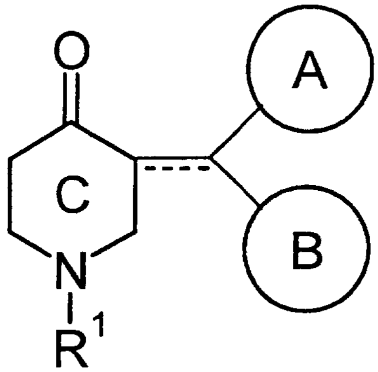

(Ia)

wherein each symbol is as defined in the above (1), or a salt thereof;

(4) The compound according to the above (1), which is represented by the formula (Ib):

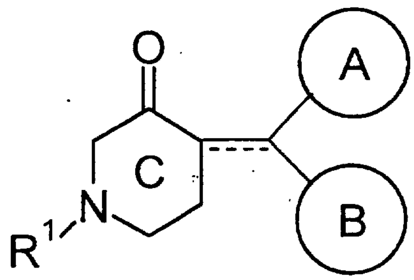

(Ib)

wherein each symbol is as defined in claim 1 or a salt thereof;

(5) The compound according to the above (1), which is represented by the formula (Ic):

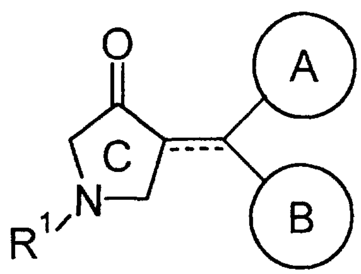

(Ic)

wherein each symbol is as defined in the above (1), or a salt thereof;

(6) The compound according to the above (1), wherein each of rings A and B is a benzene ring which may have one to three substituents selected from a halogen atom, a $C_{1-6}$ alkyl group and a $C_{1-6}$ alkoxy group, or the ring formed by binding between the bonds or substituents of rings A and B is a dibenzsuberane ring, dibenzosuberene ring, xanthene ring or thioxanthene ring;

(7) The compound according to the above (1), wherein $R^1$ is an optionally substituted benzyl;

(8) A prodrug of the compound according to the above (1), or a salt thereof;

(9) A process for producing the compound according to the above (1), which comprises reacting a compound represented by the formula (II):

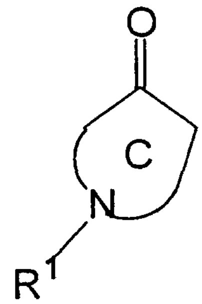

(II)

wherein each symbol is as defined in the above (1), or a salt thereof with a compound represented by the formula (III) :

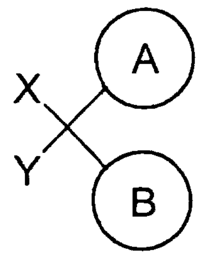

(III)

wherein each of X and Y represents a hydrogen atom, a hydroxyl group or a halogen atom, and other symbols are as defined in the above (1), provided that, when X is a hydrogen atom, then Y represents a hydroxyl group or a halogen atom, while X is a halogen atom, then Y is a halogen atom, or a reactive derivative or a salt thereof;
(10) A pharmaceutical composition comprising the compound according to the above (1), or a salt or a prodrug thereof;
(11) The pharmaceutical composition according to the above (10), which is a tachykinin receptor antagonist;
(12) The pharmaceutical composition according to the above (11), which is an agent for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder;
(13) A method for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder, which comprises administering an effective amount of the compound according to the above (1), or a salt or a prodrug to thereof to a mammal; and
(14) Use of the compound according to the above (1), or a salt or a prodrug thereof for manufacturing an agent for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder.

Further, the present invention also provides:

(15) The pharmaceutical composition according to the above (10), which is a substance P receptor antagonist;
(16) The pharmaceutical composition according to the above (10), which is an agent for preventing or treating a disease associated with substance P;
(17) The pharmaceutical composition according to the above (10), which is an abnormal urination improving agent;
(18) The pharmaceutical composition comprising a combination of an effective amount of the compound according

to the above (1) or a salt thereof or a prodrug thereof with an effective amount of one or more agents selected from the group consisting of a diabetes treating agent, diabetic complication treating agent, anti-hyperlipidemic agent, hypotensive agent, anti-obesity agent, diuretic, chemotherapeutic agent, immunotherapeutic agent, cachexia improving agent, anti-inflammatory agent, saccharificaiton inhibitor, nerve regeneration promoting agent, anti-depressant, anti-epileptic agent, anti-arrhythmic agent, acetylcholine receptor ligand, endothelin receptor antagonistic agent, monoamine intake inhibitor, narcotic and anti-narcotic analgesic, GABA receptor agonist, GABA intake inhibitor, $\alpha_2$ receptor agonist, local analgesic, protein kinase C inhibitor, anti-anxiety agent, phosphodiesterase inhibitor, dopamine receptor agonist or antagonist, serotonin receptor agonist or antagonist, serotonin intake inhibitor, sleepiness inducing agent, anti-cholinergic agent, $\alpha_1$ receptor blocker, muscle relaxant, calcium channel opener, calcium channel blocker, Alzheimer's disease preventing or treating agent, Parkinson's disease preventing or treating agent, multiple sclerosis preventing or treating agent, histamine $H_1$ receptor inhibitor, proton pump inhibitor, anti-thrombotic agent, NK-2 receptor antagonist, NK-3 receptor antagonist, antioxidant, sodium channel inhibitor and angiotensin II receptor antagonist;

(19) A method for preventing or treating pollakiuria or incontinence of urine comprising administering to a mammal a combination of an effective amount of the compound according to the above (1) or a salt thereof or a prodrug thereof with an effective amount of one or more agents selected from the group consisting of a diabetes treating agent, diabetic complication treating agent, anti-hyperlipidemic agent, hypotensive agent, anti-obesity agent, diuretic, chemotherapeutic agent, immunotherapeutic agent, cachexia improving agent, anti-inflammatory agent, saccharification inhibitor, nerve regeneration promoting agent, anti-depressant, anti-epileptic agent, anti-arrhythmic agent, acetylcholine receptor ligand, endothelin receptor antagonistic agent, monoamine intake inhibitor, narcotic and anti-narcotic analgesic, GABA receptor agonist, GABA intake inhibitor, $\alpha_2$ receptor agonist, local analgesic, protein kinase C inhibitor, anti-anxietic agent, phosphodiesterase inhibitor, dopamine receptor agonist or antagonist, serotonin receptor agonist or antagonist, serotonin intake inhibitor, sleepiness inducing agent, anti-cholinergic agent, $\alpha_1$ receptor blocker, muscle relaxant, calcium channel opener, calcium channel blocker, Alzheimer's disease preventing or treating agent, Parkinson's disease preventing or treating agent, multiple sclerosis preventing or treating agent, histamine $H_1$ receptor inhibitor, proton pump inhibitor, anti-thrombotic agent, NK-2 receptor antagonist, NK-3 receptor antagonist, antioxidant, sodium channel inhibitor and angiotensin II receptor antagonist;

(20) A method for preventing or treating asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder, comprising administering to a mammal a combination of an effective amount of the compound according to the above (1) or a salt thereof or a prodrug thereof with an effective amount of one or more agents selected from the group consisting of a diabetes treating agent, diabetic complication treating agent, anti-hyperlipidemic agent, hypotensive agent, anti-obesity agent, diuretic, chemotherapeutic agent, immunotherapeutic agent, cachexia improving agent, anti-inflammatory agent, saccharification inhibitor, nerve regeneration promoting agent, anti-depressant, anti-epileptic agent, anti-arrhythmic agent, acetylcholine receptor ligand, endothelin receptor antagonistic agent, monoamine intake inhibitor, narcotic and anti-narcotic analgesic, GABA receptor agonist, GABA intake inhibitor, $\alpha_2$ receptor agonist, local analgesic, protein kinase C inhibitor, anti-anxietic agent, phosphodiesterase inhibitor, dopamine receptor agonist or antagonist, serotonin receptor agonist or antagonist, serotonin intake inhibitor, sleepiness inducing agent, anti-cholinergic agent, $\alpha_1$ receptor blocker, muscle relaxant, calcium channel opener, calcium channel blocker, Alzheimer's disease preventing or treating agent, Parkinson's disease preventing or treating agent, multiple sclerosis preventing or treating agent, histamine $H_1$ receptor inhibitor, proton pump inhibitor, anti-thrombotic agent, NK-2 receptor antagonist, NK-3 receptor antagonist, antioxidant, sodium channel inhibitor and angiotensin II receptor antagonist.

Detailed Description of the Invention

As used herein, a $C_{1-6}$ alkyl may be a straight or branched alkyl and includes, for example, methyl, ethyl, propyl, isopropyl, butyl, isobutyl, sec-butyl, tert-butyl, pentyl, hexyl and the like.

A $C_{2-6}$ alkenyl may be a straight or branched alkenyl, and includes, for example, vinyl, allyl, isopropenyl, butenyl, isobutenyl, sec-butenyl and the like.

Examples of a $C_{3-6}$ cycloalkyl include cyclopropyl, cyclobutyl, cyclopentyl, cyclohexyl and the like.

Examples of a $C_{6-14}$ aryl include phenyl, 1-naphthyl, 2-naphthyl, biphenyl, 2-anthryl and the like.

Examples of a halogen atom include fluorine, chlorine, bromine, iodine and the like.

In the compounds represented by the formulas (I), (Ia), (Ib) and (Ic) shown above (hereinafter sometimes referred to as the compounds (I), (Ia), (Ib) and (Ic), respectively), each of rings A and B represents an optionally substituted aromatic ring, or rings A and B may be taken together via the binding between their bonds or substituents

to form a ring.

Examples of the "aromatic ring" in the "optionally substituted aromatic ring" represented by rings A and B include an aromatic hydrocarbon ring, aromatic heterocyclic ring and the like.

The "aromatic hydrocarbon ring" may for example be a monocyclic to tricyclic aromatic hydrocarbon ring having 6 to 14 carbon atoms, typically benzene ring, naphthalene ring, indene ring, anthracene ring and the like.

The "aromatic heterocyclic ring" may for example be a 5- to 14-membered, preferably 5- to 10-membered aromatic heterocyclic ring containing one or more (for example, 1 to 4) heteroatoms selected from nitrogen atoms, sulfur atoms and oxygen atoms in addition to carbon atoms. Specific examples thereof include aromatic heterocyclic rings such as thiophene, benzothiophene, benzofuran, benzimidazole, benzoxazole, benzothiazole, benzisothiazole, naphtho[2,3-b]thiophene, furane, phenoxathine, pyrrole, imidazole, pyrazole, oxadiazole, pyridine, pyrazine, pyrimidine, pyridazine, indole, isoindole, 1H-indazole, purine, 4H-quinolidine, isoquinoline, quinoline, phthaladine, naphthylidine, quinoxaline, quinazoline, cinnoline, carbazole, β-carboline, phenanthridine, acridine, phenadine, thiazole, isothiazole, phenothiazine, isoxazole, furazane, phenoxadine, phthalimide and the like, as well as rings formed by fusion of any of these rings (preferably a monocyclic ring) with one or more (preferably 1 or 2) aromatic rings (for example, benzene ring, etc.).

The "aromatic ring" in the "optionally substituted aromatic ring" represented by rings A and B is preferably a monocyclic aromatic hydrocarbon ring (i.e., benzene ring) or a monocyclic aromatic heterocyclic ring, more preferably a benzene ring.

Examples of the substituent on rings A and B include (1) a halogen atom (e.g., fluorine, chlorine, bromine, iodine and the like), (2) $C_{1-3}$ alkylenedioxy (e.g., methylenedioxy, ethylenedioxy and the like), (3) nitro, (4) cyano, (5) optionally halogenated $C_{1-6}$ alkyl, (6) optionally halogenated $C_{2-6}$ alkenyl, (7) optionally halogenated $C_{2-6}$ alkynyl, (8) optionally halogenated $C_{3-6}$ cycloalkyl, (9) $C_{6-14}$ aryl (e.g., phenyl, 1-naphthyl, 2-naphthyl, biphenylyl, 2-anthryl and the like), (10) optionally halogenated $C_{1-6}$ alkoxy, (11) optionally halogenated $C_{1-6}$ alkylthio or mercapto, (12) hydroxy, (13) amino, (14) mono-$C_{1-6}$ alkylamino (e.g., methylamino, ethylamino and the like), (15) mono-$C_{1-6}$ arylamino (e.g., phenylamino, 1-naphthylamino, 2-naphthylamino and the like), (16) di-$C_{1-6}$ alkylamino (e.g., dimethylamino, diethylamino and the like), (17) di-$C_{6-14}$ arylamino (e.g., diphenylamino and the like), (18) acyl, (19) acylamino, (20) acyloxy, (21) optionally substituted 5- to 7-membered saturated cyclic amino, (22) 5- to 10-membered aromatic heterocyclic group (e.g., 2- or 3-thienyl, 2-,3- or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl and the like), (23) sulfo, (24) $C_{6-14}$ aryloxy (e.g., phenyloxy, naphthyloxy and the like) and the like.

Each of rings A and B may have 1 to 3, preferably 1 to 2 substituents in any substitutable positions, and when there are two or more substituents, each substituent may be the same or different.

The "optionally halogenated $C_{1-6}$ alkyl group" described above may for example be a $C_{1-6}$ alkyl group (for example, methyl, ethyl, propyl, isopropyl, butyl, isobutyl, sec-butyl, tert-butyl, pentyl, hexyl and the like) which may have 1 to 5, preferably 1 to 3 halogen atoms (for example, fluorine, chlorine, bromine, iodine and the like), and the like. Specific examples thereof include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

The "optionally halogenated $C_{2-6}$ alkenyl group" may for example be a $C_{2-6}$ alkenyl group (for example, vinyl, allyl, isopropenyl, butenyl, isobutenyl, sec-butenyl and the like) which may have 1 to 5, preferably 1 to 3 halogen atoms (for example, fluorine, chlorine, bromine, iodine and the like), and the like. Specific examples thereof include vinyl, allyl, isopropenyl, butenyl, isobutenyl, sec-butenyl, 3,3,3-trifluoro-1-propenyl, 4,4,4-trifluoro-1-butenyl and the like.

The "optionally halogenated $C_{2-6}$ alkynyl group" described above may for example be a $C_{2-6}$ alkynyl group (for example, ethynyl, propargyl, butynyl, 1-hexynyl and the like) which may have 1 to 5, preferably 1 to 3 halogen atoms (for example, fluorine, chlorine, bromine, iodine and the like), and the like. Specific examples thereof include ethynyl, propargyl, butynyl, 1-hexynyl, 3,3,3-trifluoro-1-propynyl, 4,4,4-trifluoro-1-butynyl and the like.

The "optionally halogenated $C_{3-6}$ cycloalkyl group" described above may for example be a $C_{3-6}$ cycloalkyl group (for example, cyclopropyl, cyclobutyl, cyclopentyl, cyclohexyl and the like) which may have 1 to 5, preferably 1 to 3 halogen atoms (for example, fluorine, chlorine, bromine, iodine and the like), and the like. Specific examples thereof include cyclopropyl, cyclobutyl, cyclopentyl, cyclohexyl, 4,4-dichlorocyclohexyl, 2,2,3,3-tetrafluorocyclopentyl, 4-chlorocyclohexyl and the like.

The "optionally halogenated $C_{1-6}$ alkoxy group" described above may for example be a $C_{1-6}$ alkoxy group (for example, methoxy, ethoxy, propoxy, isopropoxy, butoxy, isobutoxy, sec-butoxy, pentyloxy, hexyloxy and the like) which may have 1 to 5, preferably 1 to 3 halogen atoms (for example, fluorine, chlorine, bromine, iodine and the like), and the like. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like.

The "optionally halogenated $C_{1-6}$ alkylthio group" described above may for example be a $C_{1-6}$ alkylthio group (for example, methylthio, ethylthio, propylthio, isopropylthio, butylthio, sec-butylthio, tert-butylthio and the like) which

may have 1 to 5, preferably 1 to 3 halogen atoms (for example, fluorine, chlorine, bromine, iodine and the like), and the like. Specific examples thereof include methylthio, difluoromethylthio, trifluoromethylthio, ethylthio, propylthio, isopropylthio, butylthio, 4,4,4-trifluorobutylthio, pentylthio, hexylthio and the like.

Examples of the "acyl" described above include formyl, carboxy, carbamoyl, $C_{1-6}$ alkyl-carbonyl (e.g., acetyl, propionyl and the like), $C_{3-6}$ cycloalkyl-carbonyl (e.g., cyclopropylcarbonyl, cyclopentylcarbonyl, cyclohexylcarbonyl and the like), $C_{1-6}$ alkoxy-carbonyl (e.g., methoxycarbonyl, ethoxycarbonyl, propoxycarbonyl, tert-butoxycarbonyl and the like), $C_{6-14}$ aryl-carbonyl (e.g., benzoyl, 1-naphtoyl, 2-naphthoyl and the like), $C_{7-16}$ aralkyl-carbonyl (e.g., phenylacetyl, phenylpropionyl and the like), $C_{6-14}$ aryloxy-carbonyl (e.g., phenoxycarbonyl and the like), $C_{7-16}$ aralkyloxy-carbonyl (e.g., benzyloxycarbonyl, phenethyloxycarbonyl and the like), 5-or 6-membered heterocyclic carbonyl (e.g., nicotinoyl, isonicotinoyl, 2-thenoyl, 3-thenoyl, 2-furoyl, 3-furoyl, morpholinocarbonyl, thiomorpholinocarbonyl, piperidinocarbonyl, 1-pyrrolidinylcarbonyl and the like), mono-$C_{1-6}$ alkyl-carbamoyl (e.g., methylcarbamoyl, ethylcarbamoyl and the like), di-$C_{1-6}$ alkyl-carbamoyl (e.g., dimethylcarbamoyl, diethylcarbamoyl, ethylmethylcarbamoyl and the like), $C_{6-14}$ aryl-carbamoyl (e.g., phenylcarbamoyl, 1-naphthylcarbamoyl, 2-naphthylcarbamoyl and the like), thiocarbamoyl, 5-or 6-membered heterocyclic carbamoyl (e.g., 2-pyridylcarbamoyl, 3-pyridylcarbamoyl, 4-pyridylcarbamoyl, 2-thienylcarbamoyl, 3-thienylcarbamoyl and the like), $C_{1-6}$ alkylsulfonyl (e.g., methylsulfonyl, ethylsulfonyl and the like), $C_{6-14}$ arylsulfonyl (e.g., phenylsulfonyl, 1-naphthylsulfonyl, 2-naphthylsulfonyl and the like), $C_{1-6}$ alkylsulfinyl (e.g., methylsulfinyl, ethylsulfinyl and the like), $C_{6-14}$ arylsulfinyl (e.g., phenylsulfinyl, 1-naphthylsulfinyl, 2-naphthylsulfinyl and the like) and the like.

Examples of the "acylamino" described above include formylamino, $C_{1-6}$ alkyl-carbonylamino (e.g., acetylamino and the like), $C_{6-14}$ aryl-carbonylamino (e.g., phenylcarbonylamino, naphthylcarbonylamino and the like), $C_{1-6}$ alkoxy-carbonylamino (e.g., methoxycarbonylamino, ethoxycarbonylamino, propoxycarbonyamino, butoxycarbonylamino and the like), $C_{1-6}$ alkylsulfonylamino (e.g., methylsulfonylamino, ethylsulfonylamino and the like), $C_{6-14}$ arylsulfonylamino (e.g., phenylsulfonylamino, 2-naphthylsulfonylamino, 1-naphtylsulfonylamino and the like) and the like.

Examples of the "acyloxy group" described above include $C_{1-6}$ alkyl-carbonyloxy (e.g., acetoxy, propionyloxy and the like), $C_{6-14}$ aryl-carbonyloxy (e.g., benzoyloxy, naphthylcarbonyloxy and the like), $C_{1-6}$ alkoxy-carbonyloxy (e.g., methoxycarbonyloxy, ethoxycarbonyloxy, propoxycarbonyloxy, butoxycarbonyloxy and the like), mono-$C_{1-6}$ alkyl-carbamoyloxy (e.g., methylcarbamoyloxy, ethylcarbamoyloxy and the like), di-$C_{1-6}$ alkyl-carbamoyloxy (e.g., dimethylcarbamoyloxy, diethylcarbamoyloxy and the like), $C_{6-14}$ aryl-carbamoyloxy (e.g., phenylcarbamoyloxy, naphtylcarbamoyloxy and the like), nicotinoyloxy and the like.

Examples of the "5- to 7-membered saturated cyclic amino" in the "optionally substituted 5- to 7-membered saturated cyclic amino" described above include morpholino, thiomorpholino, piperazin-1-yl, piperidino, pyrrolidin-1-yl and the like. Examples of the "substituent" in the "optionally substituted 5- to 7-membered saturated cyclic amino" include $C_{1-6}$ alkyl (e.g., methyl, ethyl, propyl, isopropyl, butyl, isobutyl, sec-butyl, tert-butyl, pentyl, hexyl, etc.), $C_{6-14}$ aryl (e.g., phenyl, 1-naphthyl, 2-naphthyl, biphenylyl, 2-anthryl, etc.), 5- to 10-mebered aromatic heterocyclic group (e.g., 2- or 3-thienyl, 2-, 3-or 4-pyridyl, 2-, 3-, 4-, 5- or 8-quinolyl, 1-, 3-, 4- or 5-isoquinolyl, 1-, 2- or 3-indolyl, 2-benzothiazolyl, 2-benzo[b]thienyl, benzo[b]furanyl, etc.) and the like and, 1 to 3 of which may be used.

Examples of the ring which may be formed together with rings A and B as a result of the binding between their bonds or substituents include fluorene ring, indenopyridine ring, indenothiophene ring, indenofurane ring, thienocyclopentapyridine ring, xanthene ring, thioxanthene ring, curomenopyridine ring, thienochromene ring, furochromene ring, thienopyranopyridine ring, dibenzosuberane ring, dibenzosuberene ring, benzocycloheptapyridine ring, benzocycloheptathiophene ring, benzocycloheptafurane ring, thienocycloheptapyridine ring and the like.

Preferably, each of rings. A and B is a benzene ring which may be substituted by 1 to 3 halogen atoms, preferably fluorine atoms.

$R^1$ represents a hydrogen atom, an optionally substituted hydrocarbon group or an optionally substituted heterocyclic group.

The "hydrocarbon group" in the "optionally substituted hydrocarbon group" represented by $R^1$ may for example be an aliphatic hydrocarbon group, a monocyclic saturated hydrocarbon group and an aromatic hydrocarbon group, with those having 1 to 16 carbon atoms being preferred. Specifically, an alkyl group, an alkenyl group, an alkynyl group, a cycloalkyl group, an aryl group, an aralkyl group, etc. are used.

For example, the "alkyl group" is preferably a lower alkyl group. For, example, a $C_{1-6}$ alkyl group such as methyl, ethyl, propyl, isopropyl, butyl, isobutyl, sec-butyl, tert-butyl, pentyl, hexyl, etc. is generally used.

For example, the "alkenyl group" is preferably a lower alkenyl group. For example, a $C_{2-6}$ alkenyl group such as vinyl, 1-propenyl, allyl, isopropenyl, butenyl, isobutenyl, etc. is generally used.

For example, the "alkynyl group" is preferably a lower alkynyl group. For example, a $C_{2-6}$ alkynyl group such as ethynyl, propargyl, 1-propynyl, etc. is generally used.

For example, the "cycloalkyl group" is preferably a lower cycloalkyl group. For example, a $C_{3-6}$ cycloalkyl group such as cyclopropyl, cyclobutyl, cyclopentyl, cyclohexyl, etc. is generally used.

For example, the "aryl group" is preferably a $C_{6-14}$ aryl such as phenyl, 1-naphthyl, 2-naphthyl, biphenylyl,

2-anthryl and the like, and a phenyl group, etc. are generally used.

For example, the "aralkyl group" is preferably a $C_{7-16}$ aralkyl group such as benzyl, phenylethyl, phenylpropyl, phenylbutyl, $\alpha$-naphthylmethyl, diphenylmethyl and the like, and benzyl, etc. are generally used.

Examples of the substituent which may be possessed by the "hydrocarbon group" in the "optionally substituted hydrocarbon group" include a halogen atom (for example, fluorine, chlorine, bromine, iodine and the like), a nitro group, a cyano group, a hydroxyl group, an optionally halogenated lower alkyl group (for example, an optionally halogenated $C_{1-6}$ alkyl group such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl and the like), an optionally halogenated lower alkoxy group (for example, a $C_{1-6}$ alkoxyl group such as methoxy, ethoxy, propoxy, isopropoxy, butoxy, isobutoxy, pentyloxy, hexyloxy and the like), an amino group, a mono-lower alkylamino group (for example, a mono-$C_{1-6}$ alkylamino group such as methylamino, ethylamino and the like), a di-lower alkylamino group (for example, a di-$C_{1-6}$ alkylamino group such as dimethylamino, diethylamino and the like), a mono-lower alkylamino-lower alkoxy group (for example, a mono-$C_{1-6}$ alkylamino-$C_{1-6}$ alkoxy group such as methylaminoethoxy and the like), a di-lower alkylamino-lower alkoxy group (for example, a di-$C_{1-6}$ alkylamino-$C_{1-6}$ alkoxy group such as diethylaminoethoxy and the like), a $C_{1-3}$ alkylenedioxy (for example, methylenedioxy, ethylenedioxy, propylenedioxy), a carboxyl group, a lower alkylcarbonyl group (for example, a $C_{1-6}$ alkyl-carbonyl group such as acetyl, propionyl and the like), a lower alkoxycarbonyl group (for example, a $C_{1-6}$ alkoxy-carbonyl group such as methoxycarbonyl, ethoxycarbonyl, propoxycarbonyl, butoxycarbonyl and the like), a carbamoyl group, a thiocarbamoyl group, a mono-lower alkyl carbamoyl group (for example, a mono-$C_{1-6}$ alkyl-carbamoyl group such as methyl carbamoyl, ethylcarbamoyl and the like), a di-lower alkylcarbamoyl group (for example, a di-$C_{1-6}$ alkylcarbamoyl group such as dimethylcarbamoyl, diethylcarbamoyl and the like), an arylcarbamoyl group (for example, a $C_{6-10}$ aryl-carbamoyl group such as phenylcarbamoyl, naphthylcarbamoyl and the like), an optionally substituted aryl group (for example, a $C_{6-10}$ aryl group such as phenyl, naphthyl and the like), an aryloxy group (for example, a $C_{6-10}$ aryloxy group such as phenyloxy, naphthyloxy and the like), an optionally halogenated lower alkylcarbonylamino group (for example, an optionally halogenated $C_{1-6}$ alkylcarbonylamino group such as acetylamino, trifluoroacetylamino and the like), an oxo group, a 5- to 14-membered (preferably 5- to 10-membered) (monocyclic to tricyclic, preferably monocyclic or bicyclic) heterocyclic group containing 1 to 4 (preferably 1 to 3) heteroatoms of 1 or 2 species selected from nitrogen, oxygen and sulfur atoms in addition to carbon atoms (for example, a 5-membered cyclic group containing 1 to 4 heteroatoms selected from oxygen, sulfur or nitrogen atoms in addition to carbon atoms such as 2- or 3-thienyl, 2- or 3-furyl, 1-, 2- or 3-pyrroryl, 1-, 2- or 3-pyrrolidinyl, 2-, 4- or 5-oxazolyl, 3-, 4- or 5-isoxazolyl, 2-, 4- or 5-thiazolyl, 3-, 4-or 5-isothiazolyl, 3-, 4- or 5-pyrazolyl, 2-, 3- or 4-pyrazolidinyl, 2-, 4- or 5-imidazolyl, 1, 2, 3-triazolyl, 1,2,4-triazolyl, 1H- or 2H-tetrazolyl and the like, a 6-membered cyclic group containing 1 to 4 heteroatoms selected from oxygen, sulfur or nitrogen atoms in addition to carbon atoms such as 2-, 3- or 4-pyridyl, N-oxide-2-, 3-or 4-pyridyl, 2-, 4- or 5-pyrimidinyl, N-oxide-2-, 4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl N-oxide-3 or 4-pyridazinyl and the like, a bicyclic or tricyclic fused ring group containing 1 to 4 heteroatoms selected from oxygen, sulfur or nitrogen atoms in addition to carbon atoms (preferably, a group formed by fusion of the 5- to 6-membered ring described above with one or two 5- to 6-membered cyclic groups containing 1 to 4 heteroatoms selected from oxygen, sulfur or nitrogen atoms in addition to carbon atoms) such as indolyl, benzofuryl, benzothiazolyl, benzoxazolyl, benzimidazolyl, quinolyl, isoquinolyl, phthalazinyl, quinazolinyl, quinoxalinyl, indolidinyl, quinolidinyl, 1,8-naphthylidinyl, dibenzofuranyl, carbazolyl, acridinyl, phenanthridinyl, chromanyl, phenothiazinyl, phenoxazinyl and the like, preferably a 5- to 7-membered (preferably 5- or 6-membered) heterocyclic group containing 1 to 3 heteroatoms selected from oxygen, sulfur or nitrogen atoms in addition to carbon atoms), a tricyclic hydrocarbon group (for example, 5H-dibenzo[a,d]cyclohepten-5-yl and the like), an imino group, a thioxo group, $C_{1-6}$ alkylthio (e.g., methylthio), carboxy $C_{1-6}$ alkyl (e.g., carboxymethyl), $C_{1-6}$ alkylsulfinyl (e.g., methylsulfinyl), mono-$C_{1-6}$ alkylsulfonylamino (e.g., methylsulfonylamino), di-$C_{1-6}$ alkylsulfonylamino (e.g., N,N-di(methylsulfonyl)amino) and the like. The "hydrocarbon group" in the "optionally substituted hydrocarbon group" may have 1 to 5, preferably 1 to 3 substituents described above at any substitutable positions in the hydrocarbon group, and when there are two or more substituents, each substituent may be the same or different.

Examples of the "heterocyclic group" in the "optionally substituted heterocyclic group" represented by $R^1$ include a 5- to 14-membered (preferably 5- to 10-membered) (monocyclic to tricyclic, preferably monocyclic or bicyclic) heterocyclic group containing 1 to 4 (preferably 1 to 3) heteroatoms of 1 or 2 species selected from nitrogen, oxygen and sulfur atoms in addition to carbon atoms. For example, a 5-membered cyclic group containing 1 to 4 heteroatoms selected from oxygen, sulfur or nitrogen atoms in addition to carbon atoms such as 2- or 3-thienyl, 2- or 3-furyl, 1-, 2- or 3-pyrroryl, 1-, 2- or 3-pyrrolidinyl, 2-, 4- or 5-oxazolyl, 3-, 4- or 5-isoxazolyl, 2-, 4- or 5-thiazolyl, 3-, 4-or 5-isothiazolyl, 3-, 4- or 5-pyrazolyl, 2-, 3- or 4-pyrazolidinyl, 2-, 4- or 5-imidazolyl, 1, 2, 3-triazolyl, 1,2,4-triazolyl, 1H- or 2H-tetrazolyl and the like, a 6-membered cyclic group containing 1 to 4 heteroatoms selected from oxygen, sulfur or nitrogen atoms in addition to carbon atoms such as 2-, 3- or 4-pyridyl, N-oxide-2-, 3- or 4-pyridyl, 2-, 4- or 5-pyrimidinyl, N-oxide-2-,

4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl N-oxide 3- or 4- pyridazinyl and the like, a bicyclic or tricyclic fused ring group containing 1 to 4 heteroatoms selected from oxygen, sulfur or nitrogen atoms in addition to carbon atoms (preferably, a group formed by fusion of the 5- to 6-membered ring described above with one or two 5- to 6-membered cyclic groups containing 1 to 4 heteroatoms selected from oxygen, sulfur or nitrogen atoms in addition to carbon atoms) such as indolyl, benzofuryl, benzothiazolyl, benzoxazolyl, benzimidazolyl, quinolyl, isoquinolyl, phthalazinyl, quinazolinyl, quinoxalinyl, indolidinyl, quinolidinyl, 1,8-naphthylidinyl, dibenzofuranyl, carbazolyl, acridinyl, phenanthridinyl, chromanyl, phenothiazinyl, phenoxazinyl and the like are used. Among them, a 5- to 7-membered (preferably 5- or 6-membered) heterocyclic group containing 1 to 3 heteroatoms selected from oxygen, sulfur or nitrogen atoms in addition to carbon atoms are preferred.

Examples of the substituent which may be possessed by the "heterocyclic group" in the "optionally substituted heterocyclic group" include a halogen atom (for example, fluorine, chlorine, bromine, iodine and the like), a lower alkyl group (for example, a $C_{1-6}$ alkyl group such as methyl, ethyl, propyl, isopropyl, butyl, isobutyl, sec-butyl, tert-butyl, pentyl, hexyl and the like), a cycloalkyl group (for example, a $C_{3-6}$ cycloalkyl group such as cyclopropyl, cyclobutyl, cyclopentyl, cyclohexyl and the like), a lower alkynyl group (for example, a $C_{2-6}$ alkynyl group such as ethynyl, 1-propynyl, propargyl and the like), a lower alkenyl group (for example, a $C_{2-6}$ alkenyl group such as vinyl, allyl, isopropenyl, butenyl, isobutenyl and the like), an aralkyl group (for example, a $C_{7-11}$ aralkyl group such as benzyl, $\alpha$-methylbenzyl, phenethyl and the like), an aryl group (for example, a $C_{6-10}$ aryl group such as phenyl, naphthyl and the like, preferably phenyl group and the like), a lower alkoxy group (for example, a $C_{1-6}$ alkoxy group such as methoxy, ethoxy, propoxy, isopropoxy, butoxy, isobutoxy, sec-butoxy, tert-butoxy and the like), an aryloxy group (for example, a $C_{6-10}$ aryloxy group such as phenoxy and the like), a lower alkanoyl group (for example, a $C_{1-6}$ alkyl-carbonyl group such as formyl, acetyl, propionyl, butyryl, isobutyryl and the like), arylcarbonyl (for example, a $C_{6-10}$ aryl-carbonyl group such as benzoyl, naphthoyl and the like), a lower alkanoyloxy group (for example, a $C_{1-6}$ alkyl-carbonyloxy group such as formyloxy, acetyloxy, propionyloxy, butyryloxy, isobutyryloxy and the like), an arylcarbonyloxy group (for example, a $C_{6-10}$ aryl-carbonyloxy group such as benzoyloxy, naphthoyloxy and the like), a carboxyl group, a lower alkoxycarbonyl group (for example, a $C_{1-6}$ alkoxy-carbonyl group such as methoxycarbonyl, ethoxycarbonyl, propoxycarbonyl, isopropoxycarbonyl, butoxycarbonyl isobutoxycarbonyl, tertbutoxycarbonyl and the like), aralkyloxycarbonyl (for example, a $C_{7-11}$ aralkyloxycarbonyl group such as benzyloxycarbonyl group and the like), a carbamoyl group, a mono-, di- or tri-halogeno-lower alkyl group (for example, a mono-, di- or tri-halogeno-$C_{1-4}$ alkyl group such as chloromethyl, dichloromethyl, trifluoromethyl, 2,2,2-trifluoroethyl and the like), an oxo group, an amidino group, an imino group, an amino group, a mono-lower alkylamino group (for example, a mono-$C_{1-4}$ alkylamino group such as methylamino, ethylamino, propylamino, isopropylamino, butylamino and the like), a di-lower alkylamino group (for example, a di-$C_{1-4}$ alkylamino group such as dimethylamino, diethylamino, dipropylamino, diisopropylamino, dibutylamino, methylethylamino and the like), a 3- to 6-membered cyclic amino group which may contain 1 to 3 heteroatoms selected from oxygen, sulfur and nitrogen atoms in addition to carbon atoms and one nitrogen atom (for example a 3- to 6-membered cyclic amino group such as aziridinyl, azetidinyl, pyrrolidinyl, pyrrolinyl, pyrrolyl, imidazolyl, pyrazolyl, imidazolidinyl, piperidinyl, morpholinyl, dihydropyridyl, pyridyl, N-methylpiperazinyl, N-ethylpiperazinyl and the like), an alkylenedioxy group (for example, a $C_{1-3}$ alkylenedioxy group such as methylenedioxy, ethylene dioxy and the like), a hydroxy group, a nitro group, a cyano group, a mercapto group, a sulfo group, a sulfino group, a phosphono group, a sulfamoyl group, a monoalkylsulfamoyl group (for example, a mono-$C_{1-6}$ alkylsulfamoyl group such as N-methylsulfamoyl, N-ethylsulfamoyl, N-propylsulfamoyl, N-isopropylsulfamoyl, N-butylsulfamoyl and the like), a dialkylsulfamoyl group (for example, a di-$C_{1-6}$ alkylsulfamoyl group such as N,N-dimethylsulfamoyl, N,N-diethylsulfamoyl, N,N-dipropylsulfamoyl, N,N-dibutylsulfamoyl and the like), an alkylthio group (for example a $C_{1-6}$ alkylthio group such as methylthio, ethylthio, propylthio, isopropylthio, butylthio, sec-butylthio, tert-butylthio and the like), an arylthio group (for example a $C_{6-10}$ arylthio group such as phenylthio, naphthylthio and the like), a lower alkylsulfinyl group (for example a $C_{1-6}$ alkylsulfinyl group such as methylsulfinyl, ethylsulfinyl, propylsulfinyl, butylsulfinyl and the like), an arylsulfinyl group (for example, a $C_{6-10}$ arylsulfinyl group such as phenylsulfinyl, naphthylsulfinyl and the like), a lower alkylsulfonyl group (such as a $C_{1-6}$ alkylsulfoyl group such as methylsulfonyl, ethylsulfonyl, propylsulfonyl,. butylsulfonyl and the like), an arylsulfonyl group (for example, a $C_{6-10}$ arylsulfonyl group such as phenylsulfonyl, naphthylsulfonyl and the like).

The "heterocyclic group" in the "optionally substituted heterocyclic group" may have 1 to 5, preferably 1 to 3 substituents described above at any substitutable positions in the heterocyclic group, and when there are two or more substituents, each substituent may be the same or different.

$R^1$ is preferably an optionally substituted benzyl, i.e., a group represented by the formula:

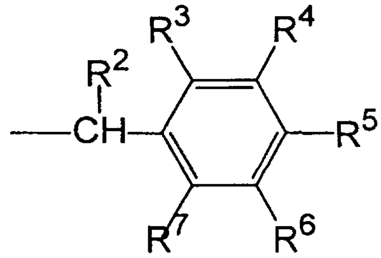

wherein $R^2$ is a hydrogen atom, $C_{1-6}$ alkyl or optionally substituted $C_{6-14}$ aryl, $R^3$, $R^4$, $R^5$, $R^6$ and $R^7$ may be the same or different and each represents a hydrogen atom, hydroxy, a halogen atom, an optionally halogenated $C_{1-6}$ alkyl group, $C_{6-14}$ aryl, $C_{6-14}$ aryloxy, optionally halogenated $C_{1-6}$ alkoxy, a substituted aminoethoxy group, $C_{1-3}$ alkylenedioxy (for example, methylenedioxy), $C_{1-6}$ alkylthio, nitro, amino, cyano, carboxyl, carboxamide, mono- or di-$C_{1-6}$ alkylamino-$C_{1-6}$ alkoxy (for example, diethylaminoethoxy), $C_{1-6}$ alkoxycarbonyl (for example, methoxycarbonyl), carboxy $C_{1-6}$ alkyl (for example, carboxymethyl), carbamoyl, $C_{1-6}$ alkylsulfinyl (for example, methylsulfinyl), mono- or di-$C_{1-6}$ alkylsulfomylamino (for example, di(methylsulfonyl)amino), heterocyclic group (for example, 1H-pyrrol-1-yl and the like), N-(optionally halogenated $C_{1-6}$ alkyl)-$C_{1-6}$ alkylsulfonylamino (for example, N-2,2,2-trifluoroethyl-N-methylsulfonylamino), optionally halogenated $C_{1-6}$ alkyl-carbonylamino (for example, acetoamino, trifluoroacetoamino), alkoxycarbonylalkyl (for example, methoxycarbonylmethyl) or aroylalkoxycarbonylalkyl (for example, benzoylmethoxycarbonylmethyl).

Examples of each of the alkyl, aryl and halogen atom represented by $R^2$, $R^3$, $R^4$, $R^5$, $R^6$ and $R^7$ include a group similar to those in the substituents on rings A and B described above. The substituent on the aryl is, for example, a group similar to those in the substituents on rings A and B described above. Examples of the aliphatic acyl include formyl, carboxy, carbamoyl, $C_{1-6}$ alkyl-carbonyl (e.g., acetyl, propionyl and the like), $C_{3-6}$ cycloalkyl-carbonyl (e.g., cyclopropylcarbonyl, cyclopentylcarbonyl, cyclohexylcarbonyl and the like), $C_{1-6}$ alkoxy-carbonyl (e.g., methoxycarbonyl, ethoxycarbonyl, propoxycarbonyl, tertbutoxycarbonyl and the like), mono-$C_{1-6}$ alkyl-carbamoyl (e.g., methylcarbamoyl, ethylcarbamoyl and the like), di-$C_{1-6}$ alkyl-carbamoyl (e.g., dimethylcarbamoyl, diethylcarbamoyl, ethylmethylcarbamoyl and the like), thiocarbamoyl, $C_{1-6}$ alkylsulfonyl (e.g., methylsulfonyl, ethylsulfonyl and the like) and the like. Examples of the substituted amino include mono-$C_{1-6}$ alkylamino (e.g., methylamino, ethylamino and the like), mono-$C_{6-14}$ arylamino (e.g., phenylamino, 1-naphthylamino, 2-naphthylamino and the like), di-$C_{1-6}$ alkylamino (e.g., dimethylamino, diethylamino and the like), di-$C_{6-14}$ arylamino (e.g., diphenylamino and the like), optionally substituted 5- to 7-membered saturated cyclic amino described above and the like.

Ring C represents a nitrogenous saturated heterocyclic ring which may be substituted in addition to the oxo.

Examples of the nitrogenous saturated heterocyclic ring include a 5- to 7-membered saturated heterocyclic ring such as pyrrolidine, piperidine, perhydroazepine and the like, provided that a 2,3-dioxopyrrolidine ring is excluded.

Examples of the substituent on the nitrogenous saturated heterocyclic ring include a group similar to the "optionally substituted hydrocarbon group" in $R^1$ described above. Preferred examples thereof include (1) $C_{1-6}$ alkyl which may have one or more substituents selected from (a) hydroxyl, (b) oxo, (c) $C_{1-6}$ alkoxy, (d) phenyl-$C_{1-3}$ alkoxy, (e) phenyl, (f) cyano, (g) halogen atom, (h) $-NR^8R^9$ wherein each of $R^8$ and $R^9$ independently represents a hydrogen atom, $C_{1-6}$ alkyl, hydroxy-$C_{1-6}$ alkyl or phenyl, (i) $-NR^8COR^9$ wherein $R^8$ and $R^9$ are as defined above, (j) $-NR^8CO_2R^9$ wherein $R^8$ and $R^9$ are as defined above, (k) $-CONR^8R^9$ wherein $R^8$ and $R^9$ are as defined above, (1) $-COR^8$ wherein $R^8$ is as defined above, (m) $-CO_2R^8$ wherein $R^8$ is as defined above, (2) $C_{2-6}$ alkenyl which may have one or more substituents selected from (a) to (m) in (1) described above, (3) $C_{2-6}$ alkynyl, (5) a phenyl group which may have one or more substituents selected from (a) to (m) in (1) described above, and the like. Ring C may have 1 to 4, preferably 1 to 3 substituents at any substitutable positions, and when there are two or more substituents, each substituent may be the same or different.

In the formula (I),

--------

represents a single bond or a double bond. A single bond is preferred.

Preferably, the compound (I) is the compound represented by the formulas (Ia), (Ib) and (Ic). More preferably, it is the compound represented by the following formulas (Ia'), (Ib') and (Ic'):

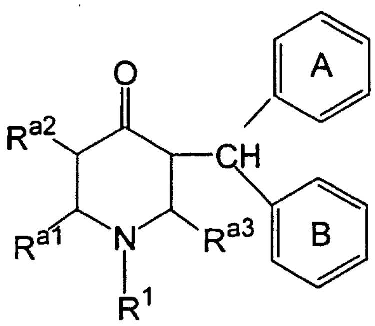

(Ia')

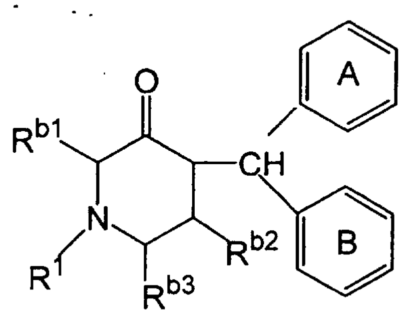

(Ib')

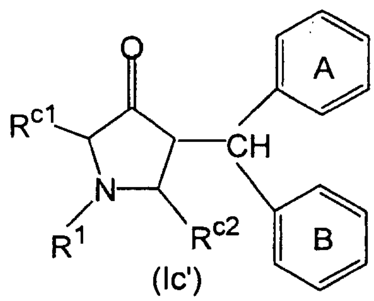

(Ic')

In the formula (Ia'), $R^{a1}$, $R^{a2}$ and $R^{a3}$ are the same or different, and each represents a hydrogen atom or an optionally substituted hydrocarbon group. As the hydrocarbon group, the group exemplified with respect to the above ring C can be used. Preferably, $R^{a1}$, $R^{a2}$ and $R^{a3}$ are hydrogen atoms.

In the formula (Ib'), $R^{b1}$, $R^{b2}$ and $R^{b3}$ are the same or different, and each represents a hydrogen atom or an optionally substituted hydrocarbon group. As the hydrocarbon group, the group exemplified with respect to the above ring C can be used. Preferably, $R^{b1}$, $R^{b2}$ and $R^{b3}$ are hydrogen atoms.

In the formula (Ic'), $R^{c1}$ and $R^{c2}$ are the same or different, and each represents. a hydrogen atom or an optionally substituted hydrocarbon group. As the hydrocarbon group, the group exemplified with respect to the above ring C can be used. Preferably, $R^{c1}$ and $R^{c2}$ are hydrogen atoms.

Examples of the salts of the compounds (I), (Ia), (Ib) and (Ic) included metal salts, ammonium salts, salts with organic bases, salts with inorganic acids, salts with organic acids, salts with basic or acidic amino acids and the like. Preferred examples of the metal salt include an alkaline metal salt such as a sodium salt, a potassium salt and the like; an alkaline earth metal salt such as a calcium salt, a magnesium salt, a barium salt and the like; an aluminum salt; and the like. Preferred examples of the salt with an organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferred examples of the salt with an inorganic acid include a salt with hydrochloric acid, hydrobromic acid, nitric acid, sulfuric acid, phosphoric acid and the like. Preferred examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferred examples of the salt with a basic amino acid include a salt with arginine, lysine, ornithine and the like. Preferred examples of the salt with an acidic amino acid include a salt with aspartic acid, glutamic acid and the like.

Among them, pharmaceutically acceptable salts are preferred. For example, when the compound has an acidic functional group in its molecule, examples thereof include an inorganic salt such as an alkaline metal salt (e.g., sodium salt, potassium salt and the like) and an alkaline earth metal salt (e.g., calcium salt, magnesium salt, barium salt and the like), an ammonium salt and the like. When the compound has a basic functional group in its molecule, examples thereof include a salt with an inorganic acid such as hydrochloric acid, hydrobromic acid, nitric acid, sulfuric acid, phosphoric acid and the like as well as a salt with an organic acid such as acetic acid, phthalic acid, fumaric acid, oxalic acid, tartaric acid, maleic acid, citric acid, succinic acid, methanesulfonic acid, p-toluenesulfonic acid and the like.

The prodrug of the compound (I) of the present invention or a salt thereof means a compound which is converted into the compound (I) under physiological conditions in a living body by. a reaction with an enzyme or gastric acid, that is, a compound which is converted into the compound (I) by an enzymatic oxidation, reduction, hydrolysis, etc., and a compound which is converted into the compound (I) by hydrolysis, etc., with gastric acid, etc.

Examples of the prodrug of the compound (I) of the present invention include a compound obtained by subjecting an amino group in the compound (I) to acylation, alkylation or phosphorylation (e.g., a compound obtained by subjecting an amino group in the compound (I) of the present invention to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxyl group in the compound (I) of the present invention to acylation, alkylation, phosphorylation or boration (e.g., a compound obtained by subjecting an hydroxyl group in the compound (I) of the present invention to acetylation, palmitoylation, propanoylation, pivaloylation, succinylation, fumarylation, alanylation, dimethylaminomethylcarbonylation, etc.); a compound obtained by subjecting a carboxyl group in the compound (I) of the present invention to esterification or amidation (e.g., a compound obtained by subjecting a carboxyl group in the compound (I) of the present invention to ethyl esterification, phenyl esterification, carboxymethyl esterification, dimethylamino esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation) and the like. Any of these compounds can be produced from the compound (I) of the present invention by a method known per se.

The prodrug of the compound (I) of the present invention may also be a compound which is converted into the compound (I) under physiological conditions such as those described in "IYAKUHIN no KAIHATSU (Development of Medicines)", Vol.7, Design of Molecules, p.163-198, Published by HIROKAWA SHOTEN (1990).

The present invention includes a solvate, for example, a hydrate of the compound represented by the formula (I) or a salt thereof. Further, the compound represented by the formula (I) may be labeled with an isotope (for example, $^{3}H$, $^{14}C$, $^{35}S$, $^{125}I$ and the like) and the like.

The compound (I) of the present invention or its salt may be present as a stereoisomer based on its double bond or an amide structure such as a cis- or trans-isomer as well as an optical isomer based on its asymmetric carbon such as an R- or S-form, and the present invention includes both of a mixture of these isomers and a single compound.

The compound (I) of the present invention or its salt can be produced for example by reacting a compound represented by the formula:

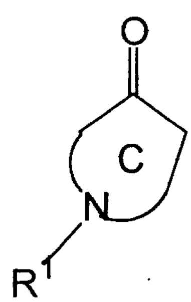

(II)

wherein each symbol is as defined above (hereinafter referred to as the compound (II)) or a salt thereof with a compound represented by the formula:

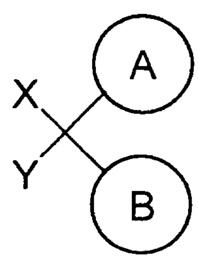

(III)

wherein each of X and Y represents a hydrogen atom, a hydroxyl group or a halogen atom, and other symbols are as

defined above, provided that when X is a hydrogen atom, then Y is a hydroxyl group or a halogen atom, while when X is a halogen atom, then Y is a halogen atom (hereinafter referred to as the compound (III)) or a reactive derivative thereof or a salt thereof in the presence of a trialkylsilyl triflate. If necessary, an acid catalyst such as a Lewis acid may be added.

The trialkylsilyl triflate is not specifically limited, but trimethylsilyl trifluoromethanesulfonate is preferred. The amount of the trialkylsilyl triflate is 1 to 10 molar equivalents, preferably 1 to 4 molar equivalents per mole of the compound (II) or a salt thereof.

The acid catalyst is not limited particularly and a mineral acid or a Lewis acid may be used. Preferred examples thereof include a zinc salt such as zinc chloride, zinc bromide, zinc iodide and the like, an aluminum salt such as aluminum chloride, an iron salt such as iron chloride, a halogenated boron such as boron trifluoride, with a zinc salt such as zinc chloride being especially preferred. The amount of a catalyst to be used is 0.001 to 1 molar equivalent, preferably 0.1 to 1 molar equivalent per mole of the compound (II) or a salt thereof. When a benzhydrol derivative is used as the compound (III) (in case that X is a hydrogen atom and Y is a hydroxyl group in the compound (III)), a catalyst is not required particularly, but may be added under certain circumstances. The benzhydrol derivative used in this step is known or can be readily produced from a corresponding benzophenone derivative by a known method [for example, Chem. Ber. 103, 2041-2051 (1970)]. The amount of the compound (III) to be used is 1 to 5 molar equivalents, preferably 1 to 3 molar equivalents per mole of the compound (II) or a salt thereof. Usually, the reaction is carried out in a solvent which is inert to the reaction. Preferred examples of the solvent to be used include aprotic solvents such as halogenated hydrocarbons such as dichloromethane, dichloroethane, chloroform and the like; nitriles such as acetonitrile and the like; esters such as ethyl acetate and the like; ethers such as dimethoxyethane, tetrahydrofurane, dioxane and the like; hydrocarbons such as benzene, toluene and the like; amides such as dimethylformamide, hexamethylphosphoramide and the like; sulfoxides such as dimethylsulfoxide and the like. In particular, halogenated hydrocarbons such as dichloromethane, dichloroethane and the like are preferred. The reaction temperature is usually -78°C to the boiling point of the solvent, with a temperature from -50°C to room temperature being especially preferred. The time required for the reaction may vary depending on the type of the compound (II) or a salt thereof, the type of the compound (III), the reaction temperature and other reaction conditions, and is usually 1 to 96 hours, preferably 1 to 24 hours.

In this reaction, an enol ether of the compound (II) may be employed as the starting compound instead of the compound (II). Examples of the enol ether include alkyl enol ether, silyl enol ether and the like, with silyl enol ether such as trimethylsilyl enol ether, triethylsilyl enol ether and triisopropylsilyl enol ether being preferred.

Alternatively, the compound (I) of the present invention or its salt can be produced by reacting a compound represented by the formula:

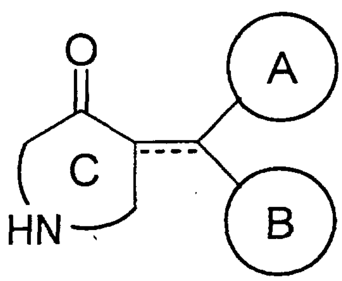

(IV)

wherein each symbol is as defined above (hereinafter referred to as the compound (IV)) or a salt thereof with a reactive derivative of a compound represented by the formula: $R^1$-OH wherein $R^1$ is as defined above, or a salt thereof, which is an alkylating or acylating agent.

The compound (IV) to be used as the starting compound can be produced by subjecting the compound (I) which is obtained by the method described above and has, as $R^1$, a group employed as a protective group such as acyl, alkyl, benzyl and the like to a known deprotection reaction or a reaction analogous thereto depending on the type of $R^1$ [for example, the method described in Protective Groups in Organic Synthesis, 3rd edition, Theodara W.Greene, Peter G.M.Wuts, Wiley-Interscience, 1999].

As the reactive derivative of the compound represented by the formula: $R^1$-OH or a salt thereof, for example, a compound represented by the formula: $R^1$-L wherein L is a leaving group and $R^1$ is as defined above (hereinafter simply referred to as the reactive derivative) or a salt thereof can be used.

Examples of the leaving group represented by L include a hydroxy group, a halogen atom (for example, chlorine atom, bromine atom, iodine atom and the like), a substituted sulfonyloxy group (for example, a $C_{1-6}$ alkylsulfonyloxy group such as methanesulfonyloxy, ethanesulfonyloxy and the like; a $C_{6-14}$ arylsulfonyloxy group such as

benzenesulfonyloxy, p-toluenesulfonyloxy and the like; a $C_{7-16}$ aralkylsulfonyloxy group such as benzylsulfonyloxy group), an acyloxy group (acetoxy, benzoyloxy and the like), an oxy group substituted with a heterocyclic ring or an aryl group (succinyl imide, benzotriazole, quinoline, 4-nitrophenyl and the like), a heterocyclic group (imidazole, alkylimidazole) and the like.

The reaction using the reactive derivative described above as the alkylating agent can be carried out by reacting the reactive derivative in the presence of a base in a solvent. Examples of the solvent include alcohols such as methanol, ethanol, propanol and the like, ethers such as dimethoxyethane, dioxane tetrahydroethane and the like, ketones such as acetone and the like, amides such as N,N-dimethylformamide and the like, sulfoxides such as dimethylsulfoxide and the like. These solvents may be appropriately mixed. Examples of the base include organic bases such as trimethylamine, triethylamine, N-methylmorpholine, pyridine, picoline, N,N-dimethylaniline and the like, and inorganic bases such as potassium carbonate, sodium carbonate, potassium hydroxide, sodium hydroxide and the like. Further, examples of the reactive derivative include optionally substituted alkane halides (for example, chloride, bromide, iodide and the like), sulfate esters, sulfonate esters (for example, methanesulfonate, p-toluenesulfonate, benzenesulfonate and the like) and the like, with a halide being preferred especially.

The amount of the reactive derivative to be used is, for example, 1 to 5 equivalents, preferably 1 to 3 equivalents per mole of the substrate. The reaction temperature is usually -10°C to 200°C, preferably about 0°C to 110°C, and the reaction time is usually 0.5 to 48 hours, preferably 0.5 to 16 hours.

When the leaving group L in the reactive derivative described above is a hydroxyl group, the reaction can also be carried out by reacting an organophosphorous compound in the presence of a base according to, for example, JP-A 58-43979. Examples of the organophosphorous compound to be used here include alkyl o-phenylenephosphates such as methyl o-phenylenephosphate, ethyl o-phenylenephosphate (EPPA) and the like, and aryl o-phenylenephosphates such as phenyl o-phenylenephosphate, p-chlorophenyl o-phenylenephosphate and the like, with EPPA being especially preferred. Examples of the base include alkylamines such as trimethylamine, triethylamine, diisopropylethylamine, tri(n-butyl)amine, di(n-butyl)amine, diisobutylamine, dicyclohexylamine and the like, and cyclic amines such as pyridine, 2,6-lutidine and the like, with an organic tertiary amine such as diisopropylethylamine being preferred. The amounts of the reactive derivative, base and organophosphorous compound described above vary depending on the types of the compound (IV), reactive derivative, base and solvent described above as well as other reaction conditions. Usually, each of them is used in an amount of about 1 to about 10 moles, preferably about 1 to about 5 moles per mole of the compound (IV). The reaction is usually carried out in a solvent inert to the reaction. Examples of the solvent to be used include aprotic solvents, for example, halogenated hydrocarbons such as dichloromethane, dichloroethane, chloroform and the like; nitriles such as acetonitrile and the like; esters such as ethyl acetate and the like; ethers such as dimethoxyethane, tetrahydrofurane, dioxane and the like; hydrocarbons such as benzene, toluene and the like; amides such as dimethylformamide, hexamethylphosphoramide and the like; sulfoxides such as dimethylsulfoxide and the like, with halogenated hydrocarbons such as dichloromethane, dichloroethane and the like being preferred.

The reaction temperature is usually -78C° to 200°C, preferably about -20°C to about 150°C, and the reaction time varies depending on the type of the compound (IV), reactive derivative, base and solvent to be used as well as other reaction conditions, and, for example, about 1 to about 72 hours, preferably about 1 to about 24 hours.

For example, the reaction using the reactive derivative described above as the acylating agent is usually carried out in a solvent optionally with a base for promoting the reaction advantageously, while the reaction varies depending on the types of the reactive derivative, base and solvent. Examples of the solvent include hydrocarbons such as benzene, toluene and the like, ethers such as ethyl ether, dioxane, tetrahydrofuran and the like, esters such as ethyl acetate and the like, halogenated hydrocarbons such as chloroform, dichloromethane and the like, amides such as N,N-dimethylformamide and the like, and aromatic hydrocarbons such as pyridine and the like. Examples of the base include bicarbonates such as sodium bicarbonate, potassium bicarbonate and the like, carbonates such as sodium carbonate, potassium carbonate and the like, acetates such as sodium acetate and the like, tertiary amines such as triethylamine, and aromatic amines such as pyridine and the like.

Examples of the reactive derivative as the acylating agent described above to be used include acid anhydrides, mixed anhydrides, acid halides (for example, chloride, bromide), heterocyclic ring or aryl (succinimide, benzotriazole, quinoline, 4-nitrophenyl and the like) esters, heterocyclic ring (imidazole, alkylimidazole) amides and the like.

The amount of the reactive derivative to be used is usually 1 to 10 molar equivalents, preferably 1 to 3 molar equivalents per mole of a substrate. The reaction temperature is usually -10°C to 150°C, preferably about 0°C to 100°C, and the reaction time is usually 15 minutes to 24 hours, preferably 30 minutes to about 16 hours.

The compound (I) or its salt can also be produced by reacting the compound (IV) with an aldehyde in the presence of a reducing agent such as a metal hydride. Examples of the metal hydride as the reducing agent include sodium borohydride, lithium borohydride, zinc borohydride, sodium cyanoborohydride, sodium triacetoxyborohydride, lithium cyanoborohydride, lithium aluminum hydride and the like. The preferred metal halide includes sodium borohydride, sodium cyanoborohydride, sodium triacetoxyborohydride and the like. The amount of the reducing agent to be used is, for example, 1 to 50 equivalents, preferably 1 to 10 equivalents per mole of the substrate. As the aldehyde,

formalin or a $C_{1-5}$ alkyl-aldehyde (e.g., acetoaldehyde) is used and the amount thereof is 1 to 100 equivalents, preferably 1 to 20 equivalents per mole of the substrate. As a reaction solvent, for example, lower alcohols such as methanol, ethanol and the like, ethers such as dioxane, tetrahydrofuran, and the like, and hydrocarbons such as benzene, toluene and the like are exemplified. They can be used alone or in combination with each other. The reaction temperature is usually -80°C to 40°C, preferably 50°C to about 25°C, and the reaction time is usually 5 minutes to 48 hours, preferably 1 hour to 24 hours.

If necessary, the compound thus obtained can be subjected to oxidation, reduction, alkylation, acylation, substitution, condensation and the like or a combination thereof by a known method or a method analogous thereto, whereby effecting further modification of the structure.

The starting compound is a known compound or can be produced by a method known per se. For example, the compound (II) can be produced by reacting a compound represented by the formula:

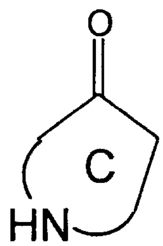

(V)

wherein ring C is as defined above (hereinafter referred to as the compound (V)) and the reactive derivative described above with an organophosphorous compound in the presence of a base according to the method described, for example, in JP-A 58-43979. Examples of the organophosphorous compound to be used here include alkyl o-phenylenephosphates such as methyl o-phenylenephosphate, ethyl o-phenylenephosphate (EPPA) and the like, aryl o-phenylenephosphates such as phenyl o-phenylenephosphate, p-chlorophenyl o-phenylenephosphate and the like, with EPPA being especially preferred. Examples of the base to be used include alkylamines such as trimethylamine, triethylamine, diisopropylethylamine, tri(n-butyl)amine, di(n-butyl)amine, diisopropylbutylamine, dicyclohexylamine and the like, cyclic amines such as pyridine, 2,6-lutidine and the like, with an organic tertiary amine such as diisopropylethylamine being preferred. The amounts of the reactive derivative, base and organophosphorous compound described above vary depending on the types of the compound (V), reactive derivative, base and solvent used as well as other reaction conditions, and usually each amount is about 1 to about 10 moles, preferably about 1 to about 5 moles per mole of the compound (V). The reaction is carried out usually in a solvent inert to the reaction. Examples of the solvent include aprotic solvents such as halogenated hydrocarbons, for example, dichloromethane, dichloroethane, chloroform and the like; nitriles such as acetonitrile and the like; esters such as ethyl acetate and the like; ethers such as dimethoxyethane, tetrahydrofuran, dioxane and the like; hydrocarbons such as benzene, toluene and the like; amides such as dimethylformamide, hexamethylphosphoramide and the like; sulfoxides such as dimethylsulfoxide and the like, with halogenated hydrocarbons such as dichloromethane, dichloroethane and the like being preferred.

The reaction temperature is usually about -78C° to about 200°C, preferably about -20°C to about 150°C, and the reaction time varies depending on the type of the compound (V), reactive derivative, base and solvent used as well as other reaction conditions, and for example is about 1 to about 72 hours, preferably about 1 to about 24 hours.

In each of the reactions for synthesizing the desired compound and starting compounds, when the starting compound has an amino group, a carboxy group or a hydroxy group as its substituent, such a group may be protected by a protective group such as that generally used in a peptide chemistry. In this case, the desired compound can be obtained by deprotection if necessary after the reaction.

As such a protective group, there is, for example, that described in Protective Groups in Organic Synthesis, 3rd edition, Theodara W.Greene, Peter G.M.Wuts, Wiley-Interscience, 1999. Examples of the protective group for an amino group include a $C_{1-6}$ alkyl-carbonyl group (for example, formyl, acetyl, propionyl groups, etc.), a phenylcarbonyl group, a $C_{1-6}$ alkyl-oxycarbonyl group (for example, methoxycarbonyl, ethoxycarbonyl groups, etc.), an aryloxycarbonyl group (for example, phenyloxycarbonyl group, etc.), a $C_{7-10}$ aralkyl-carbonyl group (for example, benzyloxycarbonyl group, etc.), a benzyl group, a benzhydryl group, a trityl group, a phthaloyl group and the like, any of which may be substituted. Examples of the substituent include a halogen atom (for example, fluorine, chlorine, bromine, iodine atoms), a $C_{1-6}$ alkyl-carbonyl group (for example, acetyl, propionyl, butylcarbonyl groups, etc.), a nitro group and the like, and the number of the substituents is about 1 to 3.

Examples of the protective group for a carboxy group include a $C_{1-6}$ alkyl group (for example, methyl, ethyl, n-propyl, i-propyl, n-butyl, tert-butyl groups, etc.), a phenyl group, a trityl group, a silyl group and the like, any of which

may be substituted. Examples of the substituent include a halogen atom (for example, fluorine, chlorine, bromine, iodine atoms), a $C_{1-6}$ alkylcarbonyl group (for example, formyl, acetyl, propionyl, butylcarbonyl groups, etc.), a nitro group and the like, and the number of the substituents is about 1 to 3.

Examples of the protective group for a hydroxy group include a $C_{1-6}$ alkyl group (for example, methyl, ethyl, n-propyl, i-propyl, n-butyl, tert-butyl groups, etc.), a phenyl group, a $C_{7-10}$ aralkyl group (for example, benzyl group, etc.), a $C_{1-6}$ alkylcarbonyl group (for example, formyl, acetyl, propionyl groups, etc.), an aryloxycarbonyl group (for example, phenyloxycarbonyl group, etc.), a $C_{7-10}$ aralkyl-carbonyl group (for example, benzyloxycarbonyl group, etc.), a pyranyl group, a furanyl group, a silyl group and the like, any of which may be substituted. Examples of the substituent include a halogen atom (for example, fluorine, chlorine, bromine, iodine atoms), a $C_{1-6}$ alkyl group, a phenyl group, a $C_{7-10}$ aralkyl group, a nitro group and the like, and the number of the substituents is about 1 to 4.

The deprotection can be carried out by a method known per se or a method described in Protective Groups in Organic Synthesis, 3rd edition, Theodara W.Greene, Peter G.M.Wuts, Wiley-Interscience, 1999, or a method analogous thereto. For example, treatment with an acid, a base, reduction, UV, hydrazine, phenylhydrazine, sodium N-methyldithiocarbamate, tetrabutylammonium fluoride, Palladium acetate, etc. can be used.

When the compound (I) is obtained in a free form by the reaction described above, according to a conventional method, it can be converted into a salt with an inorganic acid (for example, hydrochloric acid, sulfuric acid, hydrobromic acid and the like), an organic acid (for example, methanesulfonic acid, benzensulfonic acid, toluenesulfonic acid, oxalic acid, fumaric acid, maleic acid, tartaric acid and the like), an inorganic base (for example, an alkaline metal such as sodium, potassium and the like, an alkaline earth metal such as calcium, magnesium and the like, aluminum or ammonium) or an organic base (for example, trimethylamine, triethylamine, pyridine, picoline, ethanolamine, diethanolamine, triethanolamine, dicyclohexylamine or N,N'-dibenzylethylenediamine and the like). When the compound (I) is obtained as a salt, according to a conventional method, it can be converted into a free form or another salt.

When the starting compound can form a salt in the reaction described above, the compound may be used as a salt. As such a salt, there is, for example, a salt exemplified as a salt of the compound (I).

The compound (I) of the present invention obtained by these methods can be isolated and purified by a conventional method such as concentration, solvent extraction, recrystallization, distillation, column chromatography and the like.

When the compound (I) contains an optical isomer, a stereoisomer, a position isomer or a rotational isomer, these isomers are included in the compound (I), and can be obtained as an isolated product by a synthesis or separation method known per se (for example, concentration, solvent extraction, column chromatography, recrystallization and the like). For example when an optical isomer of the compound (I) exists, the optical isomer resolved from the compound is also included in the compound (I).

The optical isomer can be produced by a method known per se. Specifically, the optical isomer is obtained by using an optically active synthesis intermediate or by optically resolving a racemate of the end product according to a conventional method.

As the optical resolution, a method known per se, for example, fractional recrystallization, chiral column procedure, diastereomer procedure and the like can be used.

1) Fractional recrystallization

A method for obtaining a free optical isomer, wherein a salt of a racemate is formed with an optically active compound (for example, (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine or the like), which is then separated by fractional recrystallization, if necessary followed by neutralization to obtain the free optical isomers.

2) Chiral column method

A method for separating a racemate or a salt by applying it to a column for optical isomer separation (chiral column). For example, in case of a liquid chromatography, a mixture of the optical isomers is added to a chiral column such as ENANTIO-OVM (manufactured by TOSOH CORPORATION) or CPI COMPANY CHIRAL series manufactured by DAICEL CHEMICAL INDUSTRIES, LTD., and eluted with water, various buffers (e.g., phosphate buffer), organic solvents (e.g., ethanol, methanol, isopropanol, acetonitrile, trifluoroacetic acid, diethylamine and the like) alone or in a mixture to separate into optical isomers. Alternatively, in case of a gas chromatography, a chiral column such as CP-Chirasil-DeX CB (GL Sciences, Inc.) or the like is used to separate into optical isomers.

3) Diastereomer method

A method for obtaining an optical isomer, wherein a racemate is subjected to a chemical reaction with an

optically active reagent to form a diastereomer mixture, which is then separated into single substances by a conventional method (for example, fractional recrystallization, chromatography method and the like), from either of which an optically active reagent portion is cleaved by a chemical treatment such as hydrolysis to obtain the desired optical isomer. For example, in case where the compound (I) has hydroxy or primary or secondary amino in its molecule, the compound and an optically active organic acid (for example, MTPA [α-methoxy-α-(trifluoromethyl)phenylacetic acid], (-)-menthoxyacetic acid and the like) are subjected to a condensation reaction to obtain the diastereomers in the forms of esters or amides, respectively. On the other hand, when the compound (I) contains a carboxylate group, the compound and an optically active amine or alcohol reagent are subjected to a condensation reaction to obtain the diastereomers in the form of amides or esters, respectively. The diastereomer separated is converted into an optical isomer of the starting compound by subjecting it to acid hydrolysis or basic hydrolysis.

The compound (I) or its salt may be crystals.

The crystals of the compound (I) or its salt (hereinafter sometimes referred to as the crystals of the present invention) can be produced by subjecting the compound (I) or its salt to a crystallization method known per se to crystallize it.

Examples of the crystallization method include crystallization from a solution, crystallization from vapor, crystallization from a melt and the like.

As the "crystallization from a solution", a generally employing method is to change an unsaturated state to a supersaturated state by altering a factor involved in the solubility of the compound (solvent composition, pH, temperature, ion strength, redox conditions and the like), or an amount of a solvent. Specifically, for example, there are concentration, gradual cooling, reaction (diffusion, electrolysis), hydrothermal growth, flux method and the like. Examples of the solvent to be used include aromatic hydrocarbons (e.g., benzene, toluene, xylene and the like), halogenated hydrocarbons (e.g., dichloromethane, chloroform and the like), saturated hydrocarbons (e.g., hexane, heptane, cyclohexane and the like), ethers (e.g., diethyl ether, diisopropyl ether, tetrahydrofuran, dioxane and the like), nitriles (e.g., acetonitrile and the like), ketones (e.g., acetone and the like), sulfoxides (e.g., dimethyl sulfoxide and the like), acid amides (e.g., N,N-dimethylformamide and the like), esters (e.g., ethyl acetate and the like), alcohol (e.g., methanol, ethanol, isopropyl alcohol and the like), water and the like. These solvents can be used alone or in combination in a suitable ratio (e.g., 1:1 to 1:100 (by volume)).

Examples of the "crystallization from vapor" include a vaporization method (sealed tube method, gas flow method), gas phase reaction method, chemical transportation method and the like.

Examples of the "crystallization from a melt" include a normal freezing method (pulling method, temperature gradient method, Bridgeman method), a zone melting method (zone leveling method, float zone method), special growth method (VLS method, liquid phase epitaxy method) and the like.

As the preferred crystallization method, there are a method wherein the compound (I) or its salt is dissolved in a suitable solvent (e.g., an alcohol such as methanol, ethanol, etc.) at a temperature of 20 to 120°C, and the resultant solution is cooled to a temperature below the dissolution temperature (for example, 0 to 50°C, preferably 0 to 20°C), and the like.

The crystals of the present invention thus obtained can be isolated for example by filtration or the like.

As used herein, the melting point means that measured with a trace amount melting point meter (Yanaco. LTD. Co., Ltd., model MP-500D) or a DSC (differential scanning calorimeter) device (SEIKO, EXSTAR 6000) and the like.

Further, as used herein, the peaks in a powder X-ray diffraction method are those measured with a model RINT 2100 device (RIGAKU DENKI) and the like by using as a radiation source a Cu-K α1 beam (tube voltage: 40KV; tube current: 50 mA).

In general, sometimes, the melting point and the powder X-ray diffraction peaks vary depending on the device and measurement conditions. The crystals herein may be those having a melting point and powder X-ray diffraction peaks which are different from those specified herein in so far as they are within an ordinary error range.

The crystals of the present invention are excellent in physicochemical characteristics (e.g., melting point, solubility, stability and the like) and biological characteristics (e.g., pharmacokinetics (absorption, distribution, metabolism, excretion), manifestation of pharmacological activity and the like) and is extremely useful as a medicine.

The compound (I) of the present invention or a salt or prodrug thereof (hereinafter sometimes simply referred to as the compound of the present invention) has, in addition to an inhibitory effect of an increasing bronchial blood vessel permeability induced by capsicin, excellent tachykinin receptor antagonistic activity, especially substance P receptor antagonistic activity and neurokinin A receptor antagonistic activity. The compound of the present invention has low toxicity and is safe.

Accordingly, the compound of the present invention having excellent SP receptor antagonistic activity, neurokinon A receptor antagonistic activity, etc. can be used as a safe medicine to a mammal (for example, mouse, rat, hamster, rabbit, cat, dog, cattle, sheep, monkey, human and the like) in prevention or treatment of various diseases such as substance P-related diseases, for example, inflammation or allergic diseases (for example, atopic dermatitis,

dermatitis, herpes, psoriasis, asthma, bronchitis, chronic occlusive pulmonary disease, expectoration, rhinitis, rheumatoid arthritis, osteoarthritis, osteoporosis, multiple sclerosis, conjunctivitis, cystitis and the like), pain, migraine, neuralgia, itching, cough, further central nervous system diseases [for example, schizophrenia, Parkinson's disease, depression, anxiety neurosis, obsessive-compulsive neurosis, panic disorder, dementia (for example, Alzheimer's disease and the like) and the like], digestive organ diseases [for example, irritative bowel syndrome, ulcerative colitis, Crohn's disease, urease-positive helical gram negative microorganism (for example, *Helicobacter pylori* and the like)-induced abnormality (for example, gastiritis, gastric ulcer and the like) and the like], vomitting, abnormal urination (for example, pollakiuria, incontinence of urine and the like), circulatory diseases (for example, angina pectoris, hypertension, cardiac insufficiency, thrombosis and the like) and an immune abnormality and the like. In particular, the compound of the present invention is useful as a tachykinin receptor antagonist, an agent for improving an abnormal urination such as pollakiuria and incontinence of urine as well as an agent for treating such an abnormal urination.

Further, the compound of the present invention is useful as an agent for preventing or treating depression, anxiety, manic-depressive psychosis, schizophrenia, mania, migraine, cancer, HIV infection, cardiovascular disorder, solar dermatitis, hypogonadism, ataxia, cognitive disorder or circadian rhythm disorder.

A pharmaceutical preparation containing the compound of the present invention may be any dosage form such as a solid preparation including powder, granules, tablet, capsule, suppository and the like as well as a liquid preparation including syrup, emulsion, injectable preparation, suspension and the like.

The pharmaceutical preparation of the present invention can be produced by a conventional method such as mixing, kneading, granulating, tablet compression, coating, sterilization, emulsification and the like depending on a particular dosage form of the preparation. For producing the preparation, for example, respective sections in Japanese Pharmacopeia Preparation General Rules can be referred to.

In the pharmaceutical preparation of the present invention, the content of the compound of the present invention or a salt thereof varies depending on the dosage form of the preparation but, usually, it is about 0.01 to 100% by weight, preferably about 0.1 to 50% by weight, more preferably about 0.5 to 20% by weight based on the entire weight of the preparation.

When the compound of the present invention is used as the pharmaceutical preparation described above, it can be administered orally or parenterally as it is or in a solid dosage form such as a powder, fine particles, granules, tablet, capsule and the like or in a liquid dosage form such as an injectable preparation which is obtained by mixing with a pharmaceutically acceptable carrier, for example, an excipient (for example, starch, lactose, sugar, calcium carbonate, calcium phosphate and the like), a binder (for example, starch, gum arabic, carboxymethylcellulose, hydroxypropyl cellulose, crystalline cellulose, arginic acid, gelatin, polyvinyl pyrrolidone and the like), a lubricant (for example, stearic acid, magnesium stearate, calcium stearate, talc and the like), a disintegrant (for example, calcium carboxymethylcellulose, talc and the like), a diluent (for example, water for injection, physiological saline and the like), if necessary, together with additives (stabilizer, preservative, colorant, flavor, solubilizer, emulsifier, buffer, isotonicity and the like), and the like, according to a conventional method.

The dose varies depending on the type of the compound of the present invention or a pharmaceutically acceptable salt thereof, administration route, disease conditions and age of a patient, but a daily oral dose in an adult having an abnormal urination may be, for example, about 0.005 to 50 mg, preferably about 0.05 to 10 mg, more preferably about 0.2 to 4 mg as the compound of the present invention per kg body weight, which may be divided into 1 to 3 times a day.

The compound of the present invention can also be used in combination with a suitable amount of another pharmacologically active component by appropriately compounding or using them together.

The combined use of the compound of the present invention and a medicine to be used together results in, for example, the following excellent effects.

(1) The dose of the compound of the present invention or a medicine to be used together can be reduced when compared with that when each is administered alone. More specifically, when the compound of the present invention is used together with an anticholinergic agent or an NK-2 receptor antagonist, the dose of the anticholinergic agent or the NK-2 receptor antagonist becomes lower than that when administered alone, whereby enabling reduction in side effects such as xerostomia, etc.

(2) A medicine to be used together with the compound of the present invention can be selected according to the condition (mild, severe, etc.) of a patient.

(3) A medicine to be used together whose functional mechanism is different from that of the compound of the present invention can be selected so that a treatment period becomes longer.

(4) A medicine to be used together whose functional mechanism is different from that of the compound of the present invention can be selected so that a long lasting effect is realized.

(5) A synergistic effect can be obtained by the combined use of the compound of the present invention and a medicine to be used together.

Examples of the medicine to be compounded or used together with the compound of the present invention (hereinafter referred a medicine to be used together) include the following agents:

(1) Diabetes treating agents

An insulin preparation [e.g., an animal insulin preparation derived from bovine or porcine pancreas; a human insulin preparation synthesized by gene engineering technique using *E. coli* or yeast; zinc insulin: zinc insulin protamine; an insulin fragment or derivative (e.g., INS-1 etc.) and the like], an insulin sensitivity-enhancing agent (e.g., pioglitazone hydrochloride, troglitazone, rosiglitazone or its maleate, JTT-501, MCC-555, YM-440, GI-262570, KRP-297, FK-614, CS-011 and the like), an α-glycosidase inhibitor (e.g., voglibose, acarbose, miglitol, emiglitate and the like), a biganide agent (e.g., fenformin, metformin, buformin and the like), or a sulfonylurea agent (e.g., tolbutamid, glibenclamid, gliclazide, chlorpropamide, tolazamide, acetohexamide, glyclopyramide, glimepiride and the like), or other insulin secretion-promoting agents (e.g., repaglinide, senaglinide, mitiglinide or its calcium salt hydrate, GLP-1, nateglinide and the like), a dipeptyl peptidase IV inhibitor (e.g., NVP-DPP-278, PT-100, P32/98 and the like), a β3 agonist (e.g., CL-316243, SR-58611-A, UL-TG-307, AJ-9677, AZ40140 and the like), an amylin agonist (e.g., pramlintide and the like), a phosphotyrosine phosphatase inhibitor (e.g., vanadic acid and the like), a gluconeogenesis inhibitor (e.g., glycogen phosphorylase inhibitor, glucose-6-phosphatase inhibitor, glucagon antagonist and the like), SGLT (sodium-glucose cotransporter) inhibitor (e.g.., T-1095 and the like), and the like.

(2) Diabetic complication treating agent

An aldose reductase inhibitor (e.g., tolrestat, epalrestat, zenarestat, zopolrestat, fidarestat (SK-860), minalrestat (ARI-509), CT-112 and the like), a neurotrophic factor (e.g., NGF, NT-3 and the like), an AGE inhibitor (e.g., ALT-945, Pimagedine, pyratoxathine, N-phenacylthiazolium bromide (ALT-766), EXO-226 and the like), an active oxygen scavenger (e.g., thioctic acid), a cerebrovascular dilator (e.g., thiopride and the like) and the like.

(3) Antihyperlipidemic agent

A statin compound which is a cholesterol synthesis inhibitor (e.g., pravastatin, simvastatin, lovastatin, atorvastatin, fluvastatin, serivastatin or its salt (e.g., sodium salt, etc.) and the like), a squalene synthesis inhibiting- or triglyceride reducing-fibrate compound (e.g., bezafibrate, clofibrate, simfibrate, clinofibrate and the like), and the like.

(4) Hypotensive agent

An angiotensin converting enzyme inhibitor (e.g., captopril, enalapril, delapril and the like) or an angiotensin II antagonist (e.g., losartan, candesartan cilexetil and the like), a calcium antagonist (e.g., manidipine, nifedipine, amlodin, efonidipine, nicardipine and the like), clonidine, and the like.

(5) Anti-obesic

A central anti-obesic agent (e.g., dexfenfluramine, fenfluramine, phentermine, sibutramine, amfepramone, dexamphetamine, mazindol, phenylpropanolamine, clobenzorex and the like), a pancreatic lipase inhibitor (e.g., orlistat and the like), a β3 agonist (e.g., CL-316243, SR-58611-A, UL-TG-307, AJ-9677, AZ40140 and the like), a peptidic apetite-suppressing agent (e.g., leptin, CNTF (ciliary neurotrophic factor) and the like), a cholecystokinin agonist (e. g., rinchitript, FPL-15849 and the like) and the like.

(6) Diuretic

A xanthine derivative (e.g., teobromine sodium salicylate, teobromine calcium salicylate and the like), a thiazide preparation (e.g., ethiazide, cyclopenthiazide, trichlormethiazide, hydrochlorothiazide, hydroflumethiazide, benzylhydrochlorothiazide, penflutizide, polythiazide, methyclothiazide and the like), an anti-aldosterone preparation (e. g., spironolactone, triamterene and the like), a carbonate dehydrogenase inhibitor (e.g., acetazolamide and the like), a chlorbenzensulfonamide preparation (e.g., chlorthalidone, mefruside, indapamide and the like), azosemide, isosorbide, ethacrynic acid, piretanide, bumetanide furosemide and the like.

(7) Chemotherapeutic agent

An alkylating agent (e.g., cyclophosphamide, iphosphamide and the like), a metabolism antagonist (e.g.,

methotrexate, 5-fluorouracil and the like), an anticancer antibiotic (e.g., mitomycin, adriamycin and the like), a plant derived anticancer agent (e.g., vincristine, vindesine, taxol and the like), cisplatin, carboplatin, ethopoxide and the like, with 5-fluorouracil derivatives such as furtulon or neo furtulon being preferred.

(8) Immunotherapeutic agent

A microbial or bacterial component (e.g., muramyldipeptide derivative, picibanil and the like), an immunoenhancing polysaccharide (e.g., lentinan, schizophyllan, krestin and the like), a cytokine obtained by gene engineering technique (e.g., interferon, interleukin (IL) and the like), a colony stimulating factor (e.g., granulocyte colony stimulating factor, erythropoietin and the like), and the like with IL-1, IL-2 and IL-12 being preferred.

(9) Agent proven in animal model or clinically to have cachexia improving activity

A progesterone derivative (e.g., megesterol acetate) [Journal of Clinical Oncology, Vol.12, p.213-225, 1994], a metoclopramide agent, a tetrahydrocannabinol agent [ibid], a fat metabolism-improving agent (e.g., eicosapentaenoic acid and the like) [British Journal of Cancer, Vol.68, p.314-318, 1993], a growth hormone, IGF-1, an antibody against a cachexia-inducing factor such as TNF-$\alpha$, LIF, IL-6, or oncostatin M, and the like.

(10) Anti-inflammatory

A steroid agent (e.g., dexamethasone and the like), sodium hyaluronate, a cyclooxygenase inhibitor (e.g., indomethacin, ketoprofen, loxoprofen, meloxicam, ampiroxicam, celecoxib, rofecoxib and the like), and the like.

(11) Others

A glycosylation inhibitor (e.g., ALT-711 and the like), a neuranagenesis promoter (e.g., Y-128, VX853, prosaptide and the like), a CNS agonist (e.g., anti-depressant such as desipramine, amitriptyline, imipramine, floxetine, paroxetine, doxepin, carbamazepine and the like), an anti-epileptic agent (e.g., lamotrigine), an anti-arrhythmic agent (e.g., mexiletine), an acetylcholine receptor ligand (e.g., ABT-594), an endothelin receptor antagonist (e.g., ABT-627), a monoamine intake inhibitor (e.g., tramadol), an indoleamine intake inhibitor (e.g., fluoxetine, paroxetine), an opioid analgesic (e.g., morphine), a GABA receptor agonist (e.g., gabapentin), a GABA intake inhibitor (e.g., tiagabin), an $\alpha_2$ receptor agonist (e.g., clonidine), a local anesthesia (e.g., capsicin), a protein kinase C inhibitor (e.g., LY-333531), an anxiolytic agent (e.g., benzodiazepines), a phosphodiesterase inhibitor (e.g., sildenafil), a dopamine receptor agonist (e.g., apomorphine), an anti-cholinergic agent, an $\alpha_1$ receptor blocker (e.g., tamusulosin), a muscle relaxant (e.g., baclofen), a potassium channel opener (e.g., nicorandil), a calcium channel blocker (e.g., nifedipine), an Alzheimer's disease preventing and treating agent (e.g., donepezil, rivastigmine, galanthamine), a Parkinson's disease treating agent (e.g., L-dopa), an antithrombotic agent (e.g., aspirin, cilostazol), an NK-2 receptor antagonist, an HIV infection treating agent (saquinavir, zidovudine, lamivudine, nevirapine), a chronic obstructive pulmonary disease treating agent (salmeterol, tiotropium bromide, silomirasut) and the like.

Examples of the anticholinergic agent include atropin, scopolamin, homatropine, tropicamido, cyclopentorate, butylscopolamin bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or its salt (e.g., atropin sulfate, scopolamine hydrobromide, homatropine hydrobromide, cyclopentorate hydrochloride, flavoxate hydrochloride, pirenzepine hydrochloride, trihexyphenidyl hydrochloride, oxybutynin chloride, tolterodine, tartarate and the like), with oxybutynin, propiverine, darifenacin , tolterodine, temiverine, trospium chloride or its salt (e.g., oxybutynin chloride, tolterodine tartarate) being preferred. An acetylcholine esterase inhibitor (e.g., distigmine and the like) may also be used.

Examples of the NK-2 receptor antagonist include GR94800, GR159897, MEN10627, MEN11420 (nepadutant), SR144190, SR48968 (saredutant) or salts thereof.

The pharmceutical compositions for the combined used of the compound of the present invention and a medicine to be used together includes any of (1) a pharmaceutical composition comprising the compound of the present invention and a medicine to be used together, and (2) separately prepared preparations of the compound of the present invention and a medicine to be used together. Hereinafter, they are abbreviated as the combination preparations of the present invention.

The combination preparations of the present invention can be administered orally or parenterally in the form of, for example, solid preparations such as powders, granules, tablets, capsules and the like, liquid preparations such as syrups, emulsions, injectable preparations (including subcutaneous injection, intravenous injection, intramuscular injection and dripping infusion) and the like, sustained-release preparations such as subliqual tablets, buccal tablets,

troches, microcapsules and the like, oral quick disintegrating preparations, as well as suppositories, which are obtained by using the effective components of the compound of the present invention and a medicine to be used together as they are or mixing them with a pharmaceutically acceptable carrier separately or simultaneously.

As the above pharmaceutically acceptable carrier, various conventional organic and inorganic carrier materials to be used as pharmaceutical raw materials are used. They are compounded as excipients, lubricants, binders and disintegrants in solid preparations, solvents, dissolution aids, suspending agents, isotonicities, buffering agents and soothing agents in liquid preparations, and the like. Furthermore, if necessary, other additives such as preservatives, antioxidants, colorants, sweeteners can also be used.

Preferred examples of the above excipient include lactose, sugar, D-mannitol, starch, crystalline cellulose, light silicic anhydride, calcium carbonate, calcium phosphate and the like.

Preferred examples of the above lubricant include stearic acid, magnesium stearate, calcium stearate, talc, colloidal silica and the like.

Preferred examples of the above binder include crystalline cellulose, sugar, D-mannitol, dextrin, hydroxypropyl cellulose, hydroxypropylmethyl cellulose, polyvinyl pyrrolidone, gum arabic, gelatin and the like.

Preferred examples of the above disintegrant include starch, carboxymethylcellulose, calcium carboxymethylcellulose, sodium croscarmellose, sodium carboxymethyl starch and the like.

Preferred examples of the above solvent include water for injection, physiological saline, alcohol, propylene glycol, macrogol, sesame oil, corn oil and the like.

Preferred examples of the above dissolution aid include polyethylene glycol, propylene glycol, D-mannitol, benzyl benzoate, ethanol, tris aminomethane, cholesterol, triethanolamine, sodium carbonate, sodium citrate and the like.

Preferred examples of the above suspending agent include surfactants such as stearyl triethanolamine, sodium lauryl sulfate, lauryl aminopropionic acid, lecithin, benzalkonium chloride, benzethonium chloride, glycerin monostearate and the like; hydrophilic polymers such as polyvinyl alcohol, polyvinyl pyrrolidone, sodium carboxymethylcellulose, methylcellulose, hydroxymethyl cellulose, hydroxyethyl cellulose, hydroxypropyl cellulose and the like.

Preferred examples of the above isotonicity include sodium chloride, glycerin, D-mannitol and the like.

Preferred examples of the above buffering agent include a buffer solution of phosphate, acetate, carbonate, citrate and the like.

Preferred examples of the above soothing agent include benzyl alcohol and the like.

Preferred examples of the above preservative include p-hydroxy benzoate esters, chlorobutanol, benzyl alcohol, phenethyl alcohol, dehydroacetic acid, sorbic acid and the like.

Preferred examples of the above antioxidant include sulfite, ascorbic acid and the like.

These preparations can be produced by a method known per se which is generally employed in production steps of a pharmaceutical preparation.

For example, the compound of the present invention or a medicine to be used together can be prepared in the form of an injectable preparation by compounding it with a dispersant (e.g., Tween 80 (manufactured by ATLAS POWDER, USA), HCO60 (manufactured by NIKKO CHEMICALS), polyethylene glycol, carboxymethylcellulose, sodium alginate, hydroxypropylmethyl cellulose, dextrin and the like), a stabilizer (e.g., ascorbic acid, sodium pyrosulfite), a surfactant (e.g., polysorbate 80, macrogol and the like), a solubilizing agent (e.g., glycerin, ethanol and the like), a buffering agent (e.g., phosphoric acid or its alkali metal salt, citric acid or its alkali metal salt, and the like), an isotonicity (e.g., sodium chloride, potassium chloride, mannitol, sorbitol, glucose and the like), a pH adjuster (e.g., hydrochloric acid, sodium hydroxide and the like), a preservative (e.g., ethyl p-oxybenzoate, benzoic acid, methylparabene, propylparabene, benzyl alcohol and the like), a solubilizer (e.g., concentrated glycerin, meglumine and the like), a solubilizing aid (e.g., propylene glycol, sugar and the like), a soothing agent (e.g., glucose, benzyl alcohol and the like) and the like to obtain an aqueous injectable preparation, or dissolving, suspending or emulsifying in a vegetable oil such as olive oil, sesame oil, cottonseed oil, corn oil or the like, or in a solubilizing aid such as propylene glycol or the like to obtain an oily injectable preparation.

In order to obtain an oral dosage preparation, according to a method known per se, the compound of the present invention or a medicine to be used together is subjected to compression molding, for example, with addition of an excipient (e.g., lactose, sugar, starch and the like), a disintegrant (e.g., starch, calcium carbonate and the like), a binder (e.g., starch, gum arabic, carboxymethylcellulose, polyvinyl pyrrolidone, hydroxypropyl cellulose and the like) or a lublicant (e.g., talc, magnesium stearate, polyethylene glycol 6000 and the like) and the like, if necessary, followed by subjecting to taste masking, and coating for entric property or long lasting by a method per se known to obtain the oral dosage preparation. Examples of the coating agent to be used include hydroxypropylmethyl cellulose, ethylcellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid/acrylic acid copolymer manufactured by Rohm, German), a colorant (e.g., colcothar, titanium dioxide and the like) and the like. The oral dosage preparation may be any of a quick-release preparation and a sustained-release

preparation.

For example, in order to obtain a suppository, according to a method known per se, the compound of the present invention or a medicine to be used together can be formulated in the form of an oily or aqueous solid, semisolid or liquid suppository. Examples of an oily substrate to be used in the above composition include a higher fatty acid glyceride [e.g., cocoa butter, witepsols (manufactured by Dynamit Nobel, Germany)], a medium fatty acid [e.g., miglyol (manufactured by Dynamit Nobel, Germany) and the like], or a vegetable oil (e.g.., sesame oil, soybean oil, cottonseed oil and the like). Examples of an aqueous substrate include polyethylene glycol and propylene glycol, and examples of an aqueous gel substrate include natural gums, cellulose derivatives, vinyl polymers, acrylic acid polymers and the like.

Examples of the above sustained-release preparation include sustained-release microcapsules and the like.

While'sustained-release microcapsules can be obtained by a method known per se, preferably, a sustained-release preparation is prepared, for example, by the method of [2] hereinafter and administered.

Preferably, the compound of the present invention is formulated as an oral dosage preparation such as a solid preparation (e.g., powder, granules, tablet, capsule) or a rectal dosage preparation such as a suppository. An oral dosage preparation is especially preferred.

A medicine to be used together can be formulated in the above dosage form depending on the kind of the medicine.

Hereinafter, [1] an injectable preparation of the compound of the present invention or a medicine to be used together and its production, [2] a sustained-release or quick-release preparation of the compound of the present invention or a medicine to be used together and its production and [3] a sublingual, buccal or oral quick disintegrating preparations of the compound of the present invention or a medicine to be used together and its production will be specifically illustrated.

[1] Injectable preparation and its production

An injectable preparation obtained by dissolving the compound of the present invention or a medicine to be used together in water is preferred. The injectable preparation may contain a benzoate and/or a salicylate.

The injectable preparation is obtained by dissolving the compound of the present invention or a medicine to be used together and optionally a benzoate and/or a salicylate in water.

Examples of the above benzoate and/or salicylate include an alkali metal salt such as sodium and potassium salts, an alkaline earth metal salt such as calcium and magnesium salts, an ammonium salt, a meglumine salt, a salt of an organic acid such as trometamol, and the like.

The concentration of the compound of the present invention or a medicine to be used together in the injectable preparation is about 0.5 to 50 w/v %, preferably about 3 to 20 w/v %. The concentration of a benzoate and/or a salicylate is 0.5 to 50 w/v %, preferably 3 to 20 w/v %.

Further, additives generally used in an injectable preparation such as a stabilizer (ascorbic acid, sodium pyrosulfite and the like), a surfactant (polysorbate 80, macrogol and the like), a solubilizing agent (glycerin, ethanol and the like), a buffering agent (phosphoric acid and its alkali metal salt, citric acid and its alkali metal salt and the like), an isotonicity (sodium chloride, potassium chloride and the like), a dispersing agent (hydroxypropylmethyl cellulose, dextrin), a pH adjuster (hydrochloric acid, sodium hydroxide and the like), a preservative (ethyl p-oxybenzoate, benzoic acid and the like), a solubilizer (concentrated glycerin, meglumine and the like), a solubilizing aid (propylene glycol, sugar and the like), a soothing agent (glucose, benzyl alcohol and the like) are appropriately added to the preparation. Any of these additives is added in an amount generally used in an injectable preparation.

The pH of the injectable preparation is adjusted to 2 to 12, preferably 2.5 to 8.0 with addition of a pH adjuster.

The injectable preparation is obtained by dissolving the compound of the present invention or a medicine to be used together, optionally a benzoate and/or salicylate, and, if necessary, the above additives in water. These components may be dissolved in any order according to the same manner as that for producing a conventional injectable preparation.

An injectable aqueous solution is preferably warmed, and used as an injectable preparation after sterilization by filtration or autoclaved according to the same manner as that of a conventional injectable preparation.

An aqueous injectable preparation is preferably autoclaved at 100 to 121°C for 5 to 30 minutes.

Further, the preparation may be a solution to which antibacterial activity is given so that it can be divided to administer plural times.

[2] Sustained-release or quick-release preparation and its production

A sustained-release preparation comprising a core containing the compound of the present invention or a medicine to be used together which is optionally coated with a water-insoluble material or a swelling polymer is preferred. For example, a sustained-release oral preparation in a single daily dosage form is preferred.

Examples of the water-insoluble material used for the coating agent include cellulose ethers such as ethyl cellulose, butyl cellulose and the like, cellulose esters such as cellulose acetate, cellulose propionate and the like, polyvinyl esters such as polyvinyl acetate, polyvinyl butyrate and the like, acrylic acid polymers such as an acrylic acid/methacrylic acid copolymer, a methyl methacrylate copolymer, an ethoxyethyl methacrylate/cinnamoethyl methacrylate/aminoalkyl methacrylate copolymer, a polyacrylic acid, a polymethacrylic acid, a metacrylic acid alkylamide copolymer, a poly(methyl methacrylate), a polymethacrylate, an aminoalkyl methacrylate copolymer, a poly(methacrylic anhydride), a glycidyl methacrylate copolymer, in particular, a series of Eudragits (Rohm & Pharma) such as Eudragit RS-100, RL-100, RS-30D, RL-30D, RL-PO, RS-PO (ethyl acrylate/methyl methacrylate/chlorotrimethyl methacrylate/ethyl ammonium copolymer) and Eudragit NE-30D (methyl methacrylate/ethyl acrylate copolymer) and the like, hydrogenated oils such as hydrogenated castor oil (e.g., LUBRI WAX (Freund Industrial Co., Ltd.) and the like), waxes such as carnauba wax, a fatty acid glycerin ester, paraffin and the like, polyglycerin fatty acid esters, and the like.

The swelling polymer is preferably a polymer having an acidic cleavable group and exhibiting pH-dependent swelling, and a polymer having an acidic cleavable group which undergoes a less swelling at acidic pH such as in the stomach but is swollen extensively at neutral pH such as in small and large intestines is preferred.

Examples of such a polymer having an acidic cleavable group and exhibiting pH-dependent swelling include a crosslinked polyacrylic acid polymer such as Carbomers 934P, 940, 941, 974P, 980, 1342 and the like, polycarbophil and calcium polycarbophil (all manufactured by BF Goodrich Chemicals), Hivis Wakos 103, 104, 105 and 304 (all manufactured by Wako Pure Chemical Industries, Ltd.), and the like.

The coating agent used in the sustained-release preparation may further contain a hydrophilic material.

Examples of the hydrophilic material include a polysaccharide which may have a sulfate group such as pullulan, dextrin, alkali metal alginates and the like, a polysaccharide having a hydroxyalkyl group or a carboxyalkyl group such as hydroxypropyl cellulose, hydroxypropylmethyl cellulose, sodium carboxymethylcellulose and the like, methyl cellulose, polyvinyl pyrrolidone, polyvinyl alcohol, polyethylene glycol and the like.

The water-insoluble material content in the coating agent of the sustained-release preparation is about 30 to about 90% (w/w), preferably about 35 to about 80% (w/w), more preferably about 40 to about 75% (w/w), and the swelling polymer content is about 3 to about 30% (w/w), preferably about 3 to about 15% (w/w). The coating agent may further contain the hydrophilic material, the content of which in the coating agent is about 50% (w/w) or less, preferably about 5 to about 40% (w/w), more preferably about 5 to about 35% (w/w). As used herein, the above % (w/w) means a % by weight based on the coating agent composition which is the remainder of the coating agent solution after removing any solvent (e.g., water, a lower alcohol such as methanol, ethanol and the like).

The sustained-release preparation is produced by preparing a core containing a drug as exemplified hereinafter, followed by coating the resultant core with a coating agent solution obtained by heat-melting a water-insoluble material or a swelling polymer or by dissolving or dispersing such a material in a solvent.

I. Preparation of core containing drug

While the shape of a core containing a drug to be coated with a coating agent (hereinafter sometimes simply referred to as a core) is not specifically limited, preferably, it is prepared in the shape of particles such as granules, fine particles or the like.

When the core is granules or fine particles, they have a mean particle size of preferably about 150 to 2,000 μm, more preferably about 500 to 1,400 μm.

The core can be prepared by a conventional method. For example, a drug is mixed with a suitable excipient, binder, disintegrant, lubricant, stabilizer and the like, and then subjected to wet extruding granulation, fluidized bed granulation or the like.

The drug content in the core is about 0.5 to about 95% (w/w), preferably about 5.0 to about 80% (w/w), more preferably about 30 to about 70% (w/w).

Examples of the excipient contained in the core include a saccharide such as sugar, lactose, mannitol, glucose, and the like, starch, crystalline cellulose, calcium phosphate, corn starch and the like. Among them, crystalline cellulose and corn starch are preferred.

Examples of the binder to be used include polyvinyl alcohol, hydroxypropyl cellulose, polyethylene glycol, polyvinyl pyrrolidone, Pluronic F68, gum arabic, gelatin, starch and the like. Examples of the disintegrant include calcium carboxymethyl cellulose (ECG505), sodium croscarmellose (Ac-Di-Sol), crosslinked polyvinyl pyrrolidone (crospovidone), a low substituted hydroxypropyl cellulose (L-HPC) and the like. Among them, hydroxypropyl cellulose, polyvinyl pyrrolidone and a low substituted hydroxypropyl cellulose are preferred. Examples of the lubricant and the anticoagulant include talc, magnesium stearate and its inorganic salts, and examples of the glidant include polyethylene glycol and the like. Examples of the stabilizer include an acid such as tartaric acid, citric acid, succinic acid, fumaric acid, maleic acid and the like.

In addition to the method described above, the core can be prepared by using other methods such as an agitating granulation method, a pan coating method, a fluidized bed coating method and a melt cooling granulation method, wherein a drug or a mixture thereof with an excipient, a lublicant and the like is added to inert carrier particles as seeds for the core by portions with spraying a binder dissolved in a suitable solvent such as water, a lower alcohol (e.g., methanol, ethanol, etc.) or the like. Examples of the inert carrier particles include those prepared from sugar, lactose, starch, crystalline cellulose and waxes, and, preferably, they have a mean particle size of about 100 μm to about 1,500 μm.

In order to separate the drug contained in the core from a coating agent, the surface of the core may be covered with a protective material. Examples of the protective material include the above hydrophilic material and water-insoluble material. The preferred protective material is polyethylene glycol or a polysaccharide having a hydroxyalkyl group or a carboxyalkyl group, more preferably, hydroxypropylmethyl cellulose and hydroxypropyl cellulose. The protective material may contain, as a stabilizer, an acid such as tartaric acid, citric acid, succinic acid, fumaric acid, maleic acid and the like, and a glidant such as talc. When the protective material is used, the amount thereof to be coated is about 1 to about 15% (w/w), preferably about 1 to about 10% (w/w), more preferably about 2 to about 8%. (w/w) based on the core.

The protective material can be coated by a conventional coating method and, specifically, the core is spray-coated with the protective material by a fluidized bed coating method, a pan coating method and the like.

II. Coating of core with coating agent

The core obtained as described in the above I is coated with a coating agent solution prepared by melt-heating the above water-insoluble material, pH-dependent swelling polymer and hydrophilic material or dissolving or dispersing them in a solvent to obtain a sustained-release preparation.

As a coating method of the core with the coating agent solution, there are, for example, spray coating and the like.

The composition ratio of the water-insoluble material, swelling polymer and hydrophilic material in the coating agent solution can be selected appropriately so that contents of the respective components in the coating become the above contents.

The amount of the coating agent is about 1 to about 90% (w/w), preferably about 5 to about 50% (w/w), more preferably about 5 to about 35% (w/w) based on the core (excluding the protective material coating).

As the solvent for the coating agent solution, water and an organic solvent can be used alone or as a mixture thereof. When using a mixture, the ratio of water and the organic solvent (water/organic solvent: weight ratio) may vary from 1 to 100%, and is preferably 1 to about 30%. While the organic solvent is not specifically limited in so far as it can dissolve the water-insoluble material, examples thereof include a lower alcohol such as methyl alcohol, ethyl alcohol, isopropyl alcohol, n-butyl alcohol and the like, a lower alkanone such as acetone, acetonitrile, chloroform, methylene chloride and the like. Among them, a lower alcohol is preferred, with ethyl alcohol and isopropyl alcohol being especially preferred. Water and a mixture of water and an organic solvent are used preferably as solvents for the coating agent solution. In this case, if necessary, an acid such as tartaric acid, citric acid, succinic acid, fumaric acid, maleic acid and the like may be added to the coating agent solution for the purpose of stabilizing the coating agent solution.

As the operation for carrying out the coating by spray coating, a conventional coating method can be used. Specifically, the core is sprayed with a coating agent solution by a fluidized bed coating method, a pan coating method or the like. At this time, a lubricant such as talc, titanium oxide, magnesium stearate, calcium stearate light silicic anhydride, etc., and a plasticizer such as glycerin fatty ester, hardened castor oil, triethyl citrate, cetyl alcohol, stearyl alcohol, etc. may also be added.

After coating with a coating agent, an antistatic agent such as talc may also be admixed, if necessary.

The quick-release preparation may be a liquid (solution, suspension, emulsion and the like) or a solid (particles, pill, tablet and the like). While an oral preparation and a parenteral preparation such as an injectable preparation may be used, an oral preparation is preferred.

The quick-release preparation may usually contain, a carrier, additives and an excipient (hereinafter sometimes abbreviated as excipients) which are conventionally used in the pharmaceutical field, in addition to a drug which is an active ingredient. The pharmaceutical excipients are not specifically limited in so far as it is conventional excipients used in the pharmaceutical field. Examples of the excipient for an oral solid preparation include lactose, starch, corn starch, crystalline cellulose (Avicel PH101 manufactured by Asahi Kasei Corporation, and the like), powdered sugar, granulated sugar, mannitol, light silicic anhydride, magnesium carbonate, calcium carbonate, L-cysteine and the like, with corn starch and mannitol being preferred. Any of these excipients may be employed alone or in combination with each other. The amounts of the excipients are, for example, about 4.5 to about 99.4 w/w%, preferably about 20 to about 98.5 w/w%, more preferably about 30 to about 97 w/w%, based on the total weight of the quick-release preparation.

The drug content in the quick-release preparation can be appropriately selected within the range from about 0.5 to about 95%, preferably about 1 to about 60%, based on the total weight of the quick-release preparation.

When the quick-release preparation is an oral solid preparation, it contains a disintegrant in addition to the components described above. Examples of the disintegrant include calcium carboxymethylcellulose (ECG505 manufactured by GOTOKU CHEMICAL COMPANY LTD.), sodium croscarmellose (for example, Ac-Di-Sol manufactured by Asahi Kasei Corporation), crospovidone (for example, COLIDON CL manufactured by BASF), low-substituted hydroxypropyl cellulose (Shin-Etsu chemical Co., Ltd.), carboxymethyl starch (MATSUTANI CHEMICAL INDUSTRY CO., LTD.), sodium carboxymethyl starch (EXORITAB manufactured by KIMURA SANGYO), partial $\alpha$ starch (PCS manufactured by Asahi Kasei Corporation) and the like. For example, one which disintegrates granules by water absorption or swelling on contact with water, or forming a channel between an active component which composes the core and an excipient can be used. Any of these disintegrants can be used alone or in combination with each other. While the amount of the disintegrant to be used can be appropriately selected according to the type and the amount of the drug to be used or a particular preparation design for the intended release performance, for example, it is about 0.05 to about 30 w/w%, preferably about 0.5 to about 15 w/w% based on the total weight of the quick-release preparation.

When the quick-release preparation is an oral solid quick-release preparation, it optionally contains additives conventionally used in a solid preparation in addition to the components described above. Examples of the additives include binders (for example, sucrose, gelatin, powdery gum arabic, methyl cellulose, hydroxypropyl cellulose, hydroxypropylmethyl cellulose, carboxymethylcellulose, polyvinyl pyrrolidone, pullran, dextrin and the like), lubricants (polyethylene glycol, magnesium stearate, talc, light silicic anhydride (for example, aerosil (NIPPON AEROSIL)), surfactants (for example, anionic surfactants such as sodium alkylsulfate, nonionic surfactants such as polyoxyethylene fatty ester, polyoxyethylene sorbitan fatty ester, polyoxyethylene castor oil derivatives and the like), colorants (for example, tar colorants, caramel, colcothar, titanium oxide, ribofravins), if necessary, corrigents (for example, sweeteners, flavors and the like), adsorbents, preservatives, wetting agents, antistatic agents and the like. Further, as a stabilizer, an organic acid such as tartaric acid, citric acid, succinic acid, fumaric acid or the like can also be added.

As the above binder, hydroxypropyl cellulose, polyethylene glycol and polyvinyl pyrrolidone and the like are preferably used.

The quick-release preparation can be prepared by mixing the components described above and kneading the resultant if necessary and then molding according to a conventional production method. The above mixing can be carried out by a generally employed method, such as mixing and kneading. Specifically, when the quick-release preparation is in the form of particles, it can be prepared by mixing components with a vertical granulator, a multipurpose kneader (HATA IRON WORKS CO., LTD), a fluidized bed granulator FD-5S (POWREX CORPORATION) or thee like, and then granulating the resultant by wet extrusion granulation or fluidized bed granulation according to a method similar to that for preparing the core of the sustained-release preparation described above.

The quick-release preparation and the sustained-release preparation thus obtained as they are or together with appropriate pharmaceutical excipients can be compounded in pharmaceutical preparations separately by a conventional method to prepare preparations for administering in combination with each other simultaneously or at suitable intervals. Alternatively, both may be compounded in a single oral dosage form (e.g., granules, fine particles, tablet, capsule) as they are or together with appropriate pharmaceutical excipients. Both preparations in the form of granules or fine particles may also be filled in a single capsule for oral administration.

[3] Sublingual, buccal or oral quick disintegration preparation and its production

A sublingual, buccal or oral quick disintegration preparation may be a solid preparation such as a tablet, or may be a oral mucosa plaster (film).

The sublingual, buccal or oral quick disintegration preparation is preferably that containing a medicine to be used together and an excipient. It may also contain auxiliary agents such as a lubricant, an isotonicity, a hydrophilic carrier, a water-dispersible polymer, a stabilizer and the like. Further, for the purpose of promoting the absorption and enhancing the bioavailability, it may also contain $\beta$-cyclodextrin or $\beta$-cyclodextrin derivatives (e.g., hydroxypropyl-$\beta$-cyclodextrin and the like).

Examples of the above excipient include lactose, sugar, D-mannitol, starch, crystalline cellulose, light silicic anhydride and the like. Examples of the lubricant include magnesium stearate, calcium stearate, talc, colloidal silica and the like, with magnesium stearate and colloidal silica being preferred. Examples of the isotonicity include sodium chloride, glucose, fructose, mannitol, sorbitol, lactose, saccharose, glycerin and urea, with mannitol being preferred especially. As the hydrophilic carrier, there are, for example, a swelling hydrophilic carrier such as crystalline cellulose, ethyl cellulose, crosslinked polyvinyl pyrrolidone, light silicic anhydride, silicic acid, dicalcium phosphate, calcium carbonate and the like, with crystalline cellulose (e.g., microcrystalline cellulose and the like) being preferred. As the water-dispersible polymer, there are, for example, a gum (e.g., tragacanth gum, acacia gum, guar gum), alginate (e.g., sodium alginate), cellulose derivatives (e.g., methyl cellulose, carboxymethylcellulose, hydroxymethyl cellulose, hydroxypropyl

cellulose, hydroxypropylmethyl cellulose), gelatin, water-soluble starch, polyacrylic acid (e.g., carbomer), polymethacrylic acid, polyvinyl alcohol, polyethylene glycol, polyvinyl pyrrolidone, polycarbophil, ascorbate palmitate salt and the like, with hydroxypropylmethyl cellulose, polyacrylic acid, alginate, gelatin, carboxymethylcellulose, polyvinyl pyrrolidone and polyethylene glycol being preferred. Hydroxypropylmethyl cellulose is especially preferred. As the stabilizer, there are, for example, cysteine, thiosorbitol, tartatic acid, citric acid, sodium carbonate, ascrobic acid, glycine, sodium sulfite and the like, with citric acid and ascorbic acid being preferred especially.

The sublingual, buccal or oral quick disintegration preparation can be produced by mixing the compound of the present invention or a medicine to be used together and an excipient by a method known per se. Further, if desired, the auxiliary agents described above, such as the lubricant, isotonicity, hydrophilic carrier, water-dispersible polymer, stabilizer, colorant, sweetener, preservative and the like may also be admixed. After mixing the components described above simultaneously or at certain time intervals, the mixture is compressed into tablets to obtain the sublingual, buccal or oral quick disintegration tablet. For the purpose of obtaining a suitable hardness, a solvent such as water and an alcohol can be used to moisturize or wet the components before or after tabletting, followed by drying.

When the oral mucosa plaster (film) is prepared, the compound of the present invention or a medicine to be used together and the above water-dispersible polymer (preferably, hydroxypropyl cellulose, hydroxypropylmethyl cellulose), excipient and the like are dissolved in a solvent such as water, and then the resultant solution is cast into a film. Further, additives such as a plasticizer, a stabilizer, an antioxidant, a preservative, a colorant, a buffering agent, a sweeteners and the like may be added. A glycol such as polyethylene glycol or propylene glycol may be added for the purpose of imparting a film with an appropriate elasticity, and a bioadhesive polymer (e.g., polycarbophile, carbopol) may be added for the purpose of enhancing the adhesion of the film to the oral mucosal lining. The casting can be carried out by pouring a solution onto a non-adhesive surface, spreading the solution using a coater such as a doctor blade in a uniform thickness (preferably about 10 to 1000 microns), and then drying the solution to form a film. The film thus formed is dried at room temperature or with warming, and then cut into pieces each having a desired surface area.

As the preferred oral quick disintegration preparation, there is, for example, a quick diffusion preparation in the form of a solid network comprising the compound of the present invention or a medicine to be used together and a water-soluble or water-diffusible carrier which is inert to the compound of the present invention or the medicine to be used together. The network is obtained by sublimating a solvent from a solid composition comprising a solution of the compound of the present invention or a medicine to be used together in a suitable solvent.

In addition to the compound of the present invention or a medicine to be used together, preferably, the composition of the oral quick disintegration preparation further contains a matrix-forming agent and a secondary component.

Examples of the matrix-forming agent include gelatins, dextrins and animal or vegetable proteins such as soybean, wheat and psyllium seed proteins, and the like; gummy materials such as gum arabic, guar gum, agar, xanthane gum and the like; polysaccharides; alginates; carboxymethylcelluloses; carrageenans; dextrans; pectins; synthetic polymers such as polyvinyl pyrrolidones; materials derived from gelatin-gum arabic complexes and the like. Further, also included are saccharides such as mannitol, dextrose, lactose, galactose, trehalose and the like; cyclic saccharides such as cyclodextrins and the like; inorganic salts such as sodium phosphate, sodium chloride, aluminum silicate and the like; amino acids having 2 to 12 carbon atoms such as glycine, L-alanine, L-aspartic acid, L-glutamic acid, L-hydroxyproline, L-isoleucine, L-leucine, L-phenylalanine and the like.

One or more matrix-forming agents can be introduced into a solution or suspension before solidification. The matrix-forming agents may be present in addition to a surfactant, or may be present in the absence of a surfactant. The matrix-forming agents serve not only to form a matrix itself, but also to aid in maintaining diffusion of the compound of the present invention or a medicine to be used together in the solution or suspension.

The composition may contain a secondary component such as a preservative, an antioxidant, a surfactant, a thickening agent, a colorant, pH adjuster, a flavor, a sweetener, a taste masking agent and the like. As the suitable colorant, there are, for example, iron oxide red, black and yellow, FD&C dyes available from ERIS AND EVERALD such as FD&C Blue No.2 and FD&C Red No.40 and the like. Examples of the suitable flavor include mint, raspberry, licorice, orange, lemon, grape fruit, caramel, vanilla, cherry, grape flavor and a combination thereof. Examples of the suitable pH adjuster include citric acid, tartaric acid, phosphoric acid, hydrochloric acid and maleic acid. Examples of the suitable sweetener include aspartame, acesulfame K and thaumatine. Examples of the suitable taste masking agent include sodium bicarbonate, ion exchange resins, cyclodextrin inclusion compounds, adsorbents and microencapsulated compounds.

Usually, the preparation contains the compound of the present invention or a medicine to be used together in an amount of about 0.1 to about 50% by weight, preferably about 0.1 to about 30% by weight and, preferably, the preparation (the above sublingual tablet or buccal ant the like) allows 90% or more of the compound of the present invention or a medicine to be used together to be dissolved (in water) within a time period of about 1 to about 60 minutes, preferably about 1 minute to about 15 minutes, more preferably about 2 minutes to about 5 minutes, or is a oral quick disintegration preparation which disintegrates within about 1 to about 60 seconds, preferably about 1 to

about 30 seconds, more preferably about 1 to about 10 seconds after being placed in the oral cavity.

The amount of the above excipient is about 10 to about 99% by weight, preferably about 30 to about 90% by weight based on the entire preparation. The amount of β-cyclodextrin or β-cyclodextrin derivative is about 0 to about 30% by weight based on the entire preparation. The amount of the lubricant is about 0.01 to about 10% by weight, preferably about 1 to about 5% by weight based on the entire preparation. The amount of the isotonicity is about 0.1 to about 90% by weight, preferably about 10 to about 70% by weight based on the entire preparation. The amount of the hydrophilic carrier is about 0.1 to about 50% by weight, preferably about 10 to about 30% by weight based on the entire preparation. The amount of the water-dispersible polymer is about 0.1 to about 30% by weight, preferably about 10 to about 25% by weight based on the entire preparation. The amount of the stabilizer is about 0.1 to about 10% by weight, preferably about 1 to about 5% by weight based on the entire preparation. If necessary, the preparation described above may further contain additives such as a colorant, a sweetener, a preservative and the like.

A daily dose of the combination preparations of the present invention is not limited specifically and varies depending on the severity of disease, subject's age, sex, body weight and susceptibility, the period and intervals of administration, the characteristics, formulation, type and active components of the pharmaceutical preparation, and the like. The dose in terms of the compound of the present invention is not limited specifically in so far as it causes no problematic side effects, but the daily oral dose in terms of the compound of the present invention per kg body weight in a mammal is about 0.005 to 100 mg, preferably about 0.05 to 50 mg, more preferably about 0.2 to about 30 mg and, usually, this is administered by dividing 1 to 3 times per day.

The compound of the present invention or a medicine to be used together may be employed in any amount within the range causing no problematic side effects. The daily dose of the compound of the present invention or a medicine to be used together is not limited specifically and varies depending on the severity of disease, subject's age, sex, body weight and susceptibility, the period and intervals of administration, the characteristics, formulation, type and active components of the pharmaceutical preparation and the like. However, usually, the daily oral dose per kg body weight in a mammal is about 0.001 to 2000 mg, preferably about 0.01 to 500 mg, more preferably about 0.1 to about 100 mg in terms of the amount of the medicaments. Usually, this is administered by dividing in 1 to 4 times per day.

When the combined preparations of the present invention are administered, the compound of the present invention and a medicine to be used together may be administered at the same time. Alternatively, a medicine to be used together is first administered and then the compound of the present invention is administered, or the compound of the present invention is first administered and then a medicine to be used together is administered. When they are administered at certain time intervals, the intervals vary depending on the active component to be administered, dosage form and administration method, and when a medicine to be used together is first administered, the compound of the present invention may be administered within 1 minute to 3 days, preferably 10 minutes to 1 day, more preferably 15 minutes to 1 hour after the administration of the medicine to be used together. When the compound of the present invention is first administered, a medicine to be used together may be administered within 1 minute to 1 day, preferably 10 minutes to 6 hours, more preferably 15 minutes to 1 hour after the administration of the compound of the present invention.

As a preferred administration method, for example, about 0.001 to 200 mg/kg of a medicine to be used together in the form of an oral dosage preparation is administered orally and, after about 15 minutes, about 0.005 to 100 mg/kg of the compound of the present invention in the form of an oral dosage preparation is administered orally as a daily dose.

In the combination preparations of the present invention, the amount of the compound of the present invention based on the entire preparation varies depending on the dosage form of the preparation, but is usually 0.01 to 100% by weight, preferably 0.1 to 50% by weight, more preferably 0.5 to 20% by weight based on the entire preparation.

## Examples

The present invention will be further illustrated by the following Reference Examples, Examples, Preparation Examples and Experiments, but they are not intended to restrict the present invention, and any modification without departing from the scope of the present invention can be contemplated.

Elution in the column chromatography in the Reference Examples and Examples was carried out with observation by TLC (thin layer chromatography) unless otherwise specified. In the TLC observation, 60F254 manufactured by Merck was used as a TLC plate and the same development solvent system as that used as the eluent in the column chromatography was used. For detection, an UV detector was used. As the silica gel for the column chromatography, Silica Gel 60 (70-230 mesh) manufactured by Merck was used. Usually, the term "room temperature" means a temperature of about 10°C to 35°C. An extract was dried using sodium sulfate or magnesium sulfate.

The abbreviations used in the Examples and Reference Examples mean as follows.

NMR: nuclear magnetic resonance

EI-MS: electron impact mass spectrometry
SI-MS: secondary electron ion mass spectrometry
DMF: dimethylformamide, THF: tetrahydrofuran, DMSO: dimethyl sulfoxide, Hz: hertz, J: coupling constant, m: multiplet, q: quartet, t: triplet, d: doublet, s: singlet, b: broad, like: approximate

Example 1

3-Benzhydryl-1-methyl-4-piperidinone

To a solution of 11.3 g of 1-methyl-4-piperidinone (0.1 mole) in 50 ml of dichloromethane was added dropwise 20 ml of trimethylsilyl trifluoromethane sulfonate (hereinafter referred to as TMSOTf) under ice-cooling. After stirring at room temperature for 25 minutes, 25 g of benzhydryl bromide and then 5 g of zinc bromide were added thereto. After stirring at room temperature for 16 hours, 100 ml of water and 20 g of sodium acetate were added thereto and the mixture was stirred vigorously. The dichloromethane layer was separated and washed with water. Then, 100 ml of ethanol and 8.4 ml of conc. hydrochloric acid were added thereto, and the solvent was distilled off under reduced pressure. The residue (crystal) was triturated with ethanol and filtered to obtain 20.6 g of the hydrochloride of the title compound (yield: 53%).
mp 252-253°C.
IR (KBr) 3025, 2950, 2900, 2475, 1730, 1490, 1450, 1090, 745, 700, 695, 540 $cm^{-1}$.

To a mixture of an ice cooled aqueous solution of sodium hydroxide (4 g of sodium hydroxide and 200 ml of water) and 200 ml of dichloromethane was added 29.3 g of the hydrochloride of the title compound, and the mixture was stirred vigorously. The dichloromethane layer was separated and dried over anhydrous magnesium sulfate. Then, the solvent was distilled off under reduced pressure. The residue (crystal) was triturated with hexane and filtered. After drying, 24 g of the title compound was obtained.
mp 127-128°C.
IR (KBr) 3025, 2970, 2950, 2940, 2800, 1710, 1595, 1490, 1450, 1375, 1145, 1060, 745, 705, 695, 545 $cm^{-1}$.

Example 2

4-Benzhydryl-1-ethyl-3-piperidinone

To 100 ml of dichloromethane were added 8 g of 1-ethyl-3-piperidinone hydrochloride and 20 ml of water, and 5 g of sodium carbonate was added thereto with stirring vigorously under ice-cooling. The dichloromethane layer was separated and dried over anhydrous magnesium sulfate. Then, the solvent was distilled off under reduced pressure to obtain 5 g of the free form of 1-ethyl-3-piperidinone as a residue. To a solution of 1.4 g of the free form obtained in 5 ml of dichloromethane were added 4 ml of TMSOTf and 1.8 g of benzhydrol with stirring under ice-cooling. After stirring at room temperature for 16 hours, ice-water was added thereto and the mixture was neutralized with sodium bicarbonate. After the dichloromethane layer was dried over anhydrous magnesium sulfate, the solvent was distilled off under reduced pressure. The residue was purified by subjecting it to silica gel column chromatography (100 g, ethyl acetate). The solvent was distilled off, and the residue (crystal) was triturated with a small amount of ethyl ether and filtered to obtain 0.68 g of the title compound (yield: 70%).
mp 73-74°C.
IR (KBr) 3025, 2960, 2925, 2800, 1725, 1490, 1450, 1090, 750, 710, 695, 560 $cm^{-1}$.

Example 3

4-Benzhydryl-1-benzyl-3-piperidinone

To 150 ml of dichloromethane were added 10 g of 1-benzyl-3-piperidinone hydrochloride and 50 ml of water, and 6 g of sodium carbonate was added to the mixture with stirring vigorously under ice-cooling. After the dichloromethane layer was dried over anhydrous magnesium sulfate, the solvent was distilled off under reduced pressure. The residue was dissolved in 50 ml of dichloromethane, 18 ml of TMSOTf and 8.2 g of benzhydrol were added thereto with stirring under ice-cooling. After stirring at room temperature for 16 hours, 100 ml of ice-water and 20 g of sodium acetate were added thereto and the mixture was stirred vigorously. After the dichloromethane layer was washed with water, 50 ml of ethanol and 5 ml of conc. hydrochloric acid were added thereto, and the solvent was distilled under reduced pressure. The residue (crystal) was triturated with ethanol and filtered to obtain 12.4 g of the hydrochloride of the title compound (yield: 72%).
mp 170-171°C.

To a calculated amount of. 1 N aqueous solution of sodium hydroxide and the same volume of dichloromethane was added the above hydrochloride and the mixture was stirred vigorously. The dichloromethane layer was separated and dried over magnesium sulfate, and then the solvent was distilled off under reduced pressure to obtain the title compound.
mp 106-107°C.
IR (KBr) 3025, 2950, 1710, 1595, 1490, 1450, 1360, 740, 700, 550 $cm^{-1}$.

Example 4

3-Benzhydryl-1-benzyl-4-piperidinone

To a solution of 95 g of 1-benzyl-4-piperidinone in 250 ml of dichloromethane was added dropwise 100 ml of TMSOTf with stirring under ice-cooling. After stirring at room temperature for 25 minutes, 125 g of benzhydryl bromide and then 5 g of zinc bromide were added thereto. After stirring at room temperature for 16 hours, 100 ml of water and 100 g of sodium acetate were added and the mixture was stirred vigorously. The dichloromethane layer was separated and washed with water. Then, 200 ml of ethanol and 100 ml of conc. hydrochloric acid were added thereto and the solvent was distilled off under reduced pressure. The residue (crystal) was triturated with ethanol to obtain 144 g of the hydrochloride of the title compound (74%).
mp 202-207°C.
IR (KBr) 3250, 2900, 2450, 2360, 1730, 1490, 1450, 760, 745, 705, 595, 540, 520 $cm^{-1}$.
The above hydrochloride was treated with an aqueous solution of sodium bicarbonate to obtain the title compound.
$^1$H-NMR ($CDCl_3$) δ: 2.20-2.40, 2.50-2.70, 2.75-2.90 (each m, 6H), 3.30-3.40 (m, 1H), 3.44 and 3.54 (ABq, 2H, J=13.2 Hz), 4.64 (d, 1H, J=11.2 Hz), 7.17, 7.27 (each m, 15H).

Example 5

1-Acetyl-3-benzhydryl-4-piperidinone

To a solution of 77 g of 1-acetyl-4-piperidinone in 300 ml of dichloromethane was added 200 ml of TMSOTf with stirring under ice-cooling. Then, 92 g of benzhydrol was added thereto. After allowing to stand the mixture at room temperature overnight, 500 ml of water and 50 g of sodium acetate were added thereto and the mixture was stirred vigorously. The dichloromethane layer was separated, washed with an aqueous solution of sodium bicarbonate, and then dried over magnesium sulfate. The solvent was distilled off under reduced pressure. To the residue was added 100 ml of ethyl ether, and a precipitate was collected by filtration to obtain 132.6 g of the title compound (yield: 86%).
mp 133-136°C.
IR (KBr) 3060, 3025, 2900, 286.0, 1715, 1640, 1490, 1450, 1420, 1250, 980, 745, 705, 695 $cm^{-1}$.

Example 6

1-Acetyl-3-[bis(4-chlorophenyl)methyl]-4-piperidinone

According to the same manner as that described in Example 5, the title compound was obtained (yield: 94%).
mp 169-171°C.
IR (KBr) 2860, 1720, 1640, 1490, 1455, 1440, 1425, 1300, 1090, 1015, 895, 810, 795, 540, 500 $cm^{-1}$.

Example 7

1-Acetyl-3-[bis(4-fluorophenyl)methyl]-4-piperidinone

According to the same manner as that described in Example 5, the title compound was obtained (yield: 86%).
mp 146-147°C.
IR (KBr) 2850, 1715, 1640, 1600, 1505, 1440, 1420, 1300, 1220, 1160, 1010, 820, 765, 605, 575, 545, 525 $cm^{-1}$.

Example 8

1-Acetyl-3-(10,11-dihydro-5H-dibenzo[a,d]cyclohepten-5-yl)-4-piperidinone

According to the same manner as that described in Example 5, the title compound was obtained (yield: 15%).
mp 107-109°C.
IR (KBr) 2900, 1710, 1665, 1630, 1430, 1310, 1230, 1020, 770 $cm^{-1}$.

Example 9

1-Acetyl-3-(9H-xanthen-9-yl)-4-piperidinone

To a solution of 8 g of 1-acetyl-4-piperidinone in 50 ml of dichloromethane was added dropwise 20 ml of TMSOTf with stirring under ice-cooling. After cooling to -78°C, 10 g of xanthohydrol was added thereto, and then the mixture was stirred for 2 hours (bat temperature was raised from -78°C to -50°C). After raising to 0°C, the mixture was stirred for additional 2 hours. After the reaction mixture was washed with water, the solvent was distilled off under reduced pressure. The residue (crystal) was triturated with ethyl ether and filtered. After drying, 9.5 g of the title compound (yield: 52%) was obtained.
mp 175-176°C.
IR (KBr) 1715, 1620, 1680, 1445, 1420, 1250, 760 $cm^{-1}$.

Example 10

3-Benzhydryl-1-ethyl-4-piperidinone

To a solution of 25.4 g of 1-ethyl-4-piperidinone (0.2 mole) in 100 ml of dichloromethane was added dropwise 40 ml of TMSOTf over 20 minutes with stirring under ice-cooling. After stirring at room temperature for 25 minutes, 25 g of benzhydryl bromide and then 5 g of zinc bromide were added thereto. After stirring at room temperature for 16 hours, 100 ml of water and 20 g of sodium acetate were added and the mixture was stirred vigorously. The dichloromethane layer was separated and washed with water, and then the solvent was distilled off. The residue was purified by subjecting it to silica gel column chromatography (300 g, ethyl acetate : hexane = 1 : 1). The solvent was distilled off, and the residue (crystal) was triturated with hexane and filtered to obtain 33 g of the title compound (yield: 56%).
mp 54-58°C.
IR (KBr) 2970, 2790, 1715, 1495, 1445, 1230, 1145, 740, 700 $cm^{-1}$.

Example 11

3-Benzhydryl-1-propyl-4-piperidinone.

According to the same manner as that described in Example 10, the title compound was obtained (yield: 42%).
mp 72-76°C.
IR (KBr) 2960, 2800, 1715, 1495, 1450, 1370, 1230, 1120, 740, 700 $cm^{-1}$.

Example 12

3-Benzhydryl-1-(2-phenylethyl)-4-piperidinone

According to the same manner as that described in Example 10, the title compound was obtained (yield: 38%).
mp 73-76°C.
IR (KBr) 2960, 2780, 1715, 1450, 1225, 1130, 740, 700 $cm^{-1}$.

Reference Example 1

1-(2-Methoxybenzyl)-4-piperidinone

To a solution of 14.3 g of 4-piperidinone, 13.8 g of 2-methoxybenzyl alcohol and 35 ml of diisopropylethylamine in 50 ml of dichloromethane was added 24 ml of ethyl o-phenylene phosphate (EPPA) with stirring under ice-cooling.. After allowing to stand at room temperature for 4 days, the reaction mixture was washed with water, and the solvent

was distilled off under reduced pressure. To the residue were added 300 ml of water, 100 ml of acetone and 25 ml of conc. hydrochloric acid and the mixture was stirred in an oil bath at 70°C for 16 hours. Acetone was distilled off under reduced pressure, and the mixture was neutralized with sodium bicarbonate. The mixture was extracted with dichloromethane, the extract was dried over magnesium sulfate, and the solvent was distilled off under reduced pressure. To the residue were added 100 ml of acetone and 10 ml of conc. hydrochloric acid, and the solvent was distilled off under reduced pressure. The residue was dissolved in acetone and cooled in ice. Precipitated crystals were collected by filtration to obtain 17.6 g of the hydrochloride of the title compound.
mp 173-175°C.
IR (KBr) 3330, 3280, 2940, 2700, 2650, 2560, 1600, 1495, 1470, 1440, 1290, 1255, 1150, 1100, 1040, 985, 960, 760, 600 $cm^{-1}$.

A solution of 17.6 g of the hydrochloride of the title compound, 10 g of sodium bicarbonate, 30 ml of water and 100 ml of dichloromethane was stirred vigorously. The dichloromethane layer was separated and dried over magnesium sulfate, and then the solvent was distilled off under reduced pressure to obtain 13.7 g of the title compound (yield: 62.8%).
$^1H$-NMR ($CDCl_3$) δ: 2.50 (t, 4H, J=6 Hz), 2.82 (t, 4H, J=6 Hz), 3.70 (s, 2H), 3.83 (s, 3H), 6.85-7.41 (m, 4H).

Example 13

1-(2-Methoxybenzyl)-3-(9H-xanthen-9-yl)-4-piperidinone

To a solution of 2.2 g of 1-(2-methoxybenzyl)-4-piperidinone in 20 ml of dichloromethane were added 4 ml of TMSOTf and then 1.9 g of xanthohydrol with stirring under ice-cooling. After stirring for 1 hour under ice-cooling, 30 ml of water was added and the mixture was neutralized with sodium bicarbonate. After the reaction mixture was washed with water, and the solvent was distilled off under reduced pressure. To the residue was added ethyl acetate and the mixture was filtered. The filtrate was concentrated, and purified by subjecting it to silica gel column chromatography (100 g, ethyl acetate : hexane = 1 : 4). The desired fraction was concentrated, and precipitated crystals were collected by filtration to obtain 0.89 g of the title compound (yield: 22%).
mp 96-97°C.
IR (KBr) 2800, 1720, 1600, 1480, 1460, 1240, 1100, 1025, 765 $cm^{-1}$.

Example 14

3-(5H-Dibenzo[a,d]cyclohepten-5-yl)-1-(2-methoxybenzyl)-4-piperidinone

To a solution of 2.2 g of 1-(2-methoxybenzyl)-4-piperidinone in 20 ml of dichloromethane was added 4 ml of TMSOTf with stirring under ice-cooling. After 5 minutes, 2 g of dibenzosuberenol was added with stirring at -50°C. The mixture was stirred for 60 minutes as it was (bath temperature was raised from -50°C to -10°C during this time). To the mixture was added 20 ml of water and the resulting mixture was neutralized with sodium bicarbonate. The dichloromethane layer was separated, washed with an aqueous solution of sodium bicarbonate, and then dried over magnesium sulfate. The solvent was distilled off under reduced pressure. To the residue was added a small amount of ethyl ether and precipitated crystals were collected by filtration to obtain 1.78 g of the title compound (yield: 44%).
mp 113-114°C.
IR (KBr) 2775, 1710, 1600, 1590, 1495, 1440, 1255, 1195, 1120, 1035, 810, 780, 760, 560, 460 $cm^{-1}$.

Example 15

3-[Bis(4-methoxyphenyl)methyl]-1-methyl-4-piperidinone

To a solution of 1.13 g of 1-methyl-4-piperidinone in 10 ml of dichloromethane was added 4 ml of TMSOTf. Then, 2.44 g of 4,4'-dimethoxybenzhydrol was added thereto slowly. After allowing to stand at -50°C for 45 minutes, ice-water was added thereto and the mixture was stirred vigorously. After addition of 20 ml of ethyl acetate, the mixture was basified with sodium bicarbonate. The ethyl acetate layer was dried over magnesium sulfate and purified by subjecting it to silica gel column chromatography (40 g, ethyl acetate). The solvent was distilled off under reduced pressure, hexane was added to the residue, and the resulting crystals were collected by filtration to obtain 1.12 g of the title compound (yield: 33%).
mp 104.5-106°C.
IR (KBr) 2950, 2840, 2810, 1715, 1610, 1515, 1470, 1305, 1260, 1250, 1180, 1030, 850, 830, 810, 560, 550 $cm^{-1}$.

Example 16

3-[Bis(4-chlorophenyl)methyl]-1-methyl-4-piperidinone

To a solution of 1.13 g of 1-methyl-4-piperidinone in 15 ml of dichloromethane were added 4 ml of TMSOTf and then 2.5 g of 4,4'-dichlorobenzhydrol under ice-cooling. After stirring at room temperature overnight, 20 ml of water was added thereto and the mixture was neutralized with sodium bicarbonate. The dichloromethane layer was concentrated under reduced pressure, and then purified by subjecting it to silica gel column chromatography (40 g, ethyl acetate). The solvent was distilled off under reduced pressure, hexane was added to the residue, and the resulting crystals were collected by filtration to obtain 2.26 g of the title compound (yield: 65%).
mp 146-147°C.
IR (KBr) 2975, 2780, 1715, 1490, 1415, 1140, 1090, 1015, 820, 795, 550, 500 $cm^{-1}$.

Example 17

1-Methyl-3-(9H-xanthen-9-yl)-4-piperidinone

To a solution of 1.13 g of 1-methyl-4-piperidinone in 15 ml of dichloromethane were added 4 ml of TMSOTf and then 1.98 g of xanthohydrol under ice-cooling. The mixture was stirred under ice-cooling for 60 minutes and 20 ml of water was added thereto. The mixture was neutralized with sodium bicarbonate. The dichloromethane layer was separated, and the solvent was distilled off under reduced pressure. The residue was purified by subjecting it to silica gel column chromatography (40 g, chloroform : methanol = 13 : 5). The solvent was distilled off, and crystals were triturated with ethyl ether and filtered. After drying, 0.89 g of the title compound (yield: 30%) was obtained.
mp 160-162°C.
IR (KBr) 2950, 2800, 1710, 1480, 1460, 1250, 770, 760 $cm^{-1}$.

Example 18

1-Benzyl-3-(9H-xanthen-9-yl)-4-piperidinone

To a solution of 1.13 g of 1-benzyl-4-piperidinone in 15 ml of dichloromethane were added 4 ml of TMSOTf and then 1.98 g of xanthohydrol under ice-cooling. The mixture was stirred under ice-cooling for 60 minutes and 20 ml of water added thereto. The mixture was neutralized with sodium bicarbonate. The dichloromethane layer was separated and the solvent was distilled off under reduced pressure. The residue was purified by subjecting it to silica gel column chromatography (100 g, ethyl acetate : hexane = 1 : 1). The solvent was distilled off, and the resulting crystals were triturated with hexane and filtered. After drying, 0.76 g of the title compound (yield: 21%) was obtained.
mp 124-125°C.
IR (KBr) 2960, 2760, 1710, 1475, 1460, 1255, 1190, 1095, 900, 765, 755, 695, 510, 480 $cm^{-1}$.

Example 19

1-Methyl-3-(9H-thioxanthen-9-yl)-4-piperidinone

To a solution of 1.13 g of 1-methyl-4-piperidinone in 15 ml of dichloromethane was added 4 ml of TMSOTf under ice-cooling. The mixture was cooled to -78°C and 1.98 g of thioxanthohydrol was added thereto. The mixture was stirred at the same temperature for 60 minutes. To the reaction mixture was added 20 ml of water and the mixture was neutralized with sodium bicarbonate. The dichloromethane layer was separated, and the solvent was distilled off under reduced pressure. The residue was purified by subjecting it to silica gel column chromatography (40 g, chloroform : methanol = 13 : 5). The solvent was distilled off, and the resulting crystals were triturated with ethyl ether and filtered. After drying, 0.56 g of the title compound (yield: 18%) was obtained.
mp 155-156°C.
IR (KBr) 2940, 2800, 1710, 1470, 1440, 1375, 1240, 1135, 1060, 780, 750, 575, 490 $cm^{-1}$.

Example 20

3-[Bis(4-fluorophenyl)methyl]-1-methyl-4-piperidinone

To a solution of 2.2 g of 1-methyl-4-piperidinone in 40 ml of dichloromethane was added dropwise 8 ml of

TMSOTf under ice-cooling. After stirring at room temperature for 25 minutes, 3.7 g of 4,4'-difluorobenzhydrol was added thereto. After stirring at room temperature for 16 hours, 50 ml of water was added and the mixture was neutralized with sodium bicarbonate. The dichloromethane layer was separated, washed with water, and then dried over anhydrous magnesium sulfate. The solvent was distilled off under reduced pressure, and the residue (crystal) was triturated with hexane and filtered to obtain 4.7 g of the title compound (yield: 88%).
mp 127-129°C.
IR (KBr) 2970, 2940, 2800, 2780, 1715, 1600, 1510, 1505, 1230, 825, 560, 540 $cm^{-1}$.

Example 21

1-Benzyl-3-[bis(4-fluorophenyl)methyl]-4-piperidinone.

To a solution of 10 g of 1-benzyl-4-piperidinone in 50 ml of dichloromethane was added dropwise 20 ml of TMSOTf with stirring under ice-cooling. After stirring at room temperature for 15 minutes, 11 g of 4,4'-difluorobenzhydrol was added thereto. After stirring at room temperature for 16 hours, 50 ml of water was added thereto and the mixture was neutralized with sodium acetate. The dichloromethane layer was separated and washed with water, and then the solvent was distilled off under reduced pressure to obtain the title compound as an oil.
$^1$H-NMR ($CDCl_3$) δ: 2.30-2.75, 2.80-2.94 (each m, 6H), 3.20-3.30 (m, 1H), 3.42 and 3.57 (ABq, 2H, J=13 Hz), 4.61 (d, 1H, J=11 Hz), 6.81-7.30 (m, 13H).
To the above oil were added 50 ml of ethanol and 10 ml of conc. hydrochloric acid, and the solvent was distilled off under reduced pressure. The residue (crystal) was triturated with hexane and filtered to obtain 16.9 g of the hydrochloride of the title compound (yield: 79%).
mp 180-182°C.
IR (KBr) 2900, 2450, 2390, 1735, 1600, 1510, 1455, 1405, 1415, 1240, 1230, 1220, 1160, 825, 770, 700, 580, 550, 530, 520 $cm^{-1}$.

Example 22

1-Benzyl-3-[bis(4-chlorophenyl)methyl]-4-piperidinone

To a solution of 1.9 g of 1-benzyl-4-piperidinone in 20 ml of dichloromethan were added 4 ml of TMSOTf and 2.5 g of 4,4'-dichlorobenzhydrol with stirring under ice-cooling. After stirring at room temperature for 18 hours, 50 ml of water was added thereto and the mixture was neutralized with sodium bicarbonate. The dichloromethane layer was washed with water, and the solvent was distilled off under reduced pressure. The residue was purified by subjecting it to silica gel column chromatography (100 g, ethyl acetate : hexane = 1 : 1) to obtain the title compound as an oil.
$^1$H-NMR ($CDCl_3$) δ: 2.30-2.90 (each m, 6H), 3.20-3.34(m, 1H), 3.42 and 3.57 (ABq, 2H, J=12.9 Hz), 4.56 (d, 1H, J=11 Hz), 7.00-7.32 (m, 13H).
To the above oil were added 30 ml of ethanol and 0.84 ml of conc. hydrochloric acid and the solvent was distilled off under reduced pressure. The residue (crystal) was triturated with ethanol and filtered to obtain 3.3 g of the hydrochloride of the title compound (yield: 72%).
mp 192-195°C.
IR (KBr) 2900, 2450, 2380, 1730, 1490, 1090, 1010, 795, 700, 525 $cm^{-1}$.

Example 23

3-[(4-Chlorophenyl)(phenyl)methyl]-1-methyl-4-piperidinone

To a solution of 2.3 g of 1-methyl-4-piperidinone in 40 ml of dichloromethane were added 8 ml of TMSOTf and 4.2 g of 4-chlorobenzhydrol with stirring under ice-cooling. After stirring at room temperature for 16 hours, 50 ml of water was added thereto and the mixture was neutralized with sodium bicarbonate. The dichloromethane layer was washed with water, and then dried over magnesium sulfate. The solvent was distilled off under reduced pressure to obtain the title compound as an oil.
$^1$H-NMR ($CDCl_3$) δ: 2.27, 2.28 (each s, 3H), 2.27-2.40, 2.40-2.85 (each m, 6H), 3.41 (m, 1H), 4.50 (d, 1/2H, J=11.6 Hz), 4.55 (d, 1/2H, J=11.2 Hz), 7.10-7.35 (m, 9H).
To the above oil were added 20 ml of ethanol and 1.6 ml of conc. hydrochloric acid and the solvent was distilled off under reduced pressure. The residue (crystal) was triturated with ethanol and filtered to obtain 3.3 g of the hydrochloride of the title compound (yield: 47%).
mp 192-193°C.

IR (KBr) 2950, 2460, 1730, 1490, 1090, 1015, 755, 700, 545 $cm^{-1}$.

Example 24

1-Benzyl-3-[(4-chlorophenyl)(phenyl)methyl]-4-piperidinone

To a solution of 1.9 g of. 1-benzyl-4-piperidinone in 20 ml of dichloromethane were added 4 ml of TMSOTf and 2.1 g of 4-chlorobenzhydrol with stirring under ice-cooling. After stirring at room temperature for 16 hours, 50 ml of water was added thereto and the mixture was neutralized with sodium bicarbonate. The dichloromethane layer was washed with water, and then dried over magnesium sulfate. The solvent was distilled off under reduced pressure to obtain the title compound as an oil.
$^1$H-NMR ($CDCl_3$) δ: 2.30-2.90 (m, 6H), 3.25-3.40 (m, 1H), 3.39-3.62 (m, 2H), 4.59 (d, 1/2H, J=11.4 Hz), 4.62 (d, 1/2H, J=11.2 Hz), 7.05-7.40 (m, 14H).

To the above oil were added 20 ml of ethanol and 2 ml of conc. hydrochloric acid and the solvent was distilled off under reduced pressure. The residue (crystal) was triturated with ethanol and filtered to obtain 2.9 g of the hydrochloride of the title compound (yield: 74%).
mp 175-178°C.
IR (KBr) 2900, 2450, 2380, 1730, 1490, 1450, 1090, 1015, 760, 700, 545, 525 $cm^{-1}$.

Example 25

3-(10, 11-Dihydro-5H-dibenzo[a, d]cyclohepten-5-yl)-1-methyl-4-piperidinone

To a solution of 1.13 g of 1-methyl-4-piperidinone in 20 ml of dichloromethane was added 4 ml of TMSOTf with stirring under ice-cooling. After addition of 2.1 g of dibenzosuberol cooled to -50°C, the mixture was stirred at the same temperature for 2 hours and 20 ml of water was added thereto. The mixture was neutralized with sodium bicarbonate. The dichloromethane layer was separated, washed with water, and dried over anhydrous magnesium sulfate. The solvent was distilled off under reduced pressure, and the residue (crystal) was triturated with hexane and filtered to obtain 1.4 g of the title compound (yield: 46%).
mp 146-147°C.
IR (KBr) 2940, 2790, 1715, 1230, 1135, 780, 755, 740, 560 $cm^{-1}$.

Example 26

3-(5H-Dibenzo[a,d]cyclohepten-5-yl)-1-methyl-4-piperidinone

To a solution of 1.13 g of 1-methyl-4-piperidinone in 15 ml of dichloromethane was added 4 ml of TMSOTf with stirring under ice-cooling. After addition of 2.1 g of dibenzosuberenol cooled to -70°C, the mixture was stirred at the same temperature for 1 hour and 20 ml of water was added thereto. The mixture was neutralized with sodium bicarbonate. The dichloromethane layer was separated, washed with water, and dried over anhydrous magnesium sulfate. The solvent was distilled off under reduced pressure, and the residue was subjected to silica gel column chromatography eluting with ethyl acetate. After distilling off the solvent, the residue (crystal) was triturated with hexane and filtered to obtain 1 g of the title compound (yield: 33%).
mp 144-158°C.
IR (KBr) 2960, 2775, 1710, 1480, 1360, 1270, 1250, 1220, 1155, 1135, 1050, 795, 780, 760, 725, 550, 465 $cm^{-1}$.

Example 27

1-Benzyl-3-(5H-dibenzo[a,d]cyclohepten-5-yl)-4-piperidinone

To a solution of 10 g of 1-benzyl-4-piperidinone in 100 ml of dichloromethane was added 4 ml of TMSOTf with stirring under ice-cooling. After addition of 10 g of dibenzosuberenol cooled to -50°C, the mixture was stirred for 1 hour (bath temperature was raised from -50°C to 0°C). Then, 20 ml of water was added and the mixture was neutralized with sodium bicarbonate. The dichloromethane layer was separated, washed with water, and then dried over anhydrous magnesium sulfate. The solvent was distilled off under reduced pressure, and the residue (crystal) was triturated with ethyl acetate and filtered to obtain 12.7 g of the title compound (yield: 67%).
mp 167-169°C.
IR (KBr) 2825, 1705, 1495, 1340, 1240, 1185, 1115, 810, 780, 730, 700, 465 $cm^{-1}$.

Example 28

3-Benzhydryl-4-piperadinone

To 10.2 g of 1-acetyl-3-benzhydryl-4-piperidinone were added 200 ml of water and 200 ml of conc. hydrochloric acid and the mixture was heated for 5 hours. Further, after stirring at 80°C overnight, the mixture was filtered while hot, and the filtrate was concentrated under reduced pressure. The residue (crystal) was triturated with ethanol and filtered to obtain 8 g of the hydrochloride of the title compound (yield: 80%).
mp 208-210°C.
IR (KBr) 2980, 2800, 2710, 1735, 1590, 1450, 1385, 1170, 755, 710, 700, 540 $cm^{-1}$.

Example 29

3-[Bis(4-chlorophenyl)methyl]-4-piperidinone

According to the same manner as that described in Example 28, the hydrochloride of the title compound was obtained (yield: 77%).
mp 145-147°C.
IR (KBr) 3400, 2900, 2700, 1730, 1590, 1490, 1410, 1090, 1010, 810, 790, 540, 500 $cm^{-1}$.

Example 30

3-[Bis(4-fluorophenyl)methyl]-4-piperidinone

According to the same manner as that described in Example 28, the hydrochloride of the title compound was obtained (yield: 97%).
mp 122-123°C.
IR (KBr) 3420, 2920, 2700, 2490, 1730, 1600, 1505, 1220, 1155, 825, 570, 550, 525 $cm^{-1}$.

Example 31

4-Benzhydryl-3-piperidinone

To a solution of 12 g of 4-benzhydryl-1-benzyl-3-piperidinone hydrochloride in 250 ml of methanol was added 6 g of 10% Pd-C hydrated powder (Nippon Engelhard Ltd.), and the mixture was stirred in a stream of hydrogen. After a calculated amount of the hydrogen was absorbed, the mixture was filtered and the filtrate was concentrated under reduced pressure. The residue (crystal) was triturated with ethanol and filtered to obtain 7 g of the hydrochloride of the title compound (yield: 76%).
mp 203-207°C.
IR (KBr) 3425, 2925, 2700, 2450, 1730, 1600, 1490, 1450, 1420, 1375, 1305, 1235, 1155, 1070, 1030, 770, 745, 705, 690, 535 $cm^{-1}$.

Example 32

3-Benzhydryl-1-(2-methoxybenzyl)-4-piperidinone

To a solution of 1 g of 3-benzhydryl-4-piperidinone hydrochloride, 0.5 g of 2-methoxybenzyl alcohol and 1.7 g of diisopropylethylamine in 10 ml of dichloromethane was added 1 ml of EPPA. After allowing to stand at room temperature for 16 hours, the solvent was distilled off under reduced pressure. The residue was purified by subjected it to silica gel column chromatography (100 g, ethyl acetate). Recrystallization from 3 ml of ethyl ether gave 0.87 g of the title compound (yield: 68%).
mp 114-116°C.
IR (KBr) 3010, 2900, 2780, 1705, 1590, 1575, 1490, 1455, 1430, 1315, 1280, 1235, 1170, 1110, 1080, 1015, 750, 695, 540 $cm^{-1}$.

Example 33

4-Benzhydryl-1-(2-methoxybenzyl)-3-piperidinone

To a solution of 1 g of 4-benzhydryl-3-piperidinone hydrochloride, 0.5 g of 2-methoxybenzyl alcohol and 1.7 g of diisopropylethylamine in 10 ml of dichloromethane was added 1 ml of EPPA. After allowing to stand at room temperature for 16 hours, the solvent was distilled off under reduced pressure. The residue was purified by subjecting it to silica gel column chromatography (100 g, ethyl acetate : hexane = 1 : 1). The oily title compound was dissolved in 5 ml of ethanol and 0.3 ml of conc. hydrochloric acid. Crystals precipitated were collected by filtration to obtain 0.5 g of the hydrochloride of the title compound (yield: 36%).
mp 195-196°C.
IR (KBr) 2925, 2460, 2360, 1730, 1605, 1495, 1425, 1255, 1030, 970, 750, 710, 540 $cm^{-1}$.

Example 34

3-Benzhydryl-1-[1-(2-methoxyphenyl)ethyl]-4-piperidinone

To a solution of 3 g of 3-benzhydryl-4-piperidinone hydrochloride, 1.5 g of (±)-1-(2-methoxyphenyl)ethanol and 5.1 g of diisopropylethylamine is 30 ml of dichloromethane was added 3 ml of EPPA. After allowing to stand at room temperature for 16 hours, the solvent was distilled off under reduced pressure. The residue was purified by subjecting it to silica gel column chromatography (100 g, ethyl acetate : hexane = 1 : 1) to obtain the title compound as an oil.
$^1$H-NMR ($CDCl_3$) δ: 1.51 (d, 3H, J=6.5 Hz), 2.20-2.90 (m, 7H), 3.40 (m, 1H), 3.70, 3.76 (each s, 3H), 4.54, 4.63 (each d, 1H, J=11 Hz), 6.79-7.45 (m, 14H).
The above oil was dissolved in 5 ml of ethanol and 0.8 ml of conc. hydrochloric acid, and the solvent was distilled off under reduced pressure. After washing with ethyl ether, the residue was decanted and dried to obtain 1.18 g of the hydrochloride of the title compound (yield: 27%).
mp 125-127°C.
IR (KBr) 3425, 2975, 2350, 1730, 1600, 1495, 1450, 1250, 1020, 745, 705, 540 $cm^{-1}$.

Example 35

3-[Bis(4-chlorophenyl)methyl]-1-(2-methoxybenzyl)-4-piperidinone

According to the same manner as that described in Example 33, the hydrochloride of the title compound was synthesized (yield: 70%).
mp 186-187°C.
IR (KBr) 2940, 2500, 2400, 1720, 1605, 1495, 1470, 1410, 1260, 1090, 1015, 815, 755 $cm^{-1}$.

Example 36

3-[Bis(4-fluorophenyl)methyl]-1-(2-methoxybenzyl)-4-piperidinone

According to the same manner as that described in Example 33, the hydrochloride of the title compound was synthesized (yield: 51%).
mp 164-166°C.
IR (KBr) 2950, 2475, 2400, 1725, 1605, 1510, 1470, 1255, 1230, 1220, 1160, 1030, 840, 825, 755, 555, 530 $cm^{-1}$.

Example 37

3-Benzhydryl-1-[bis(4-methoxyphenyl)methyl]-4-piperidinone

To a solution of 1 g of 3-benzhydryl-4-piperidinone hydrochloride, 0.8 g of 4,4'-dimethoxybenzhydrol and 1.7 ml of diisopropylethylamine in 10 ml of dichloromethane was added 1 ml of EPPA. After allowing to stand at room temperature for 3 hours, the reaction mixture was washed with water, and the solvent was distilled off under reduced pressure. Recrystallization from ethyl ether gave 1.3 g of the title compound (yield: 79%).
mp 137-139°C.
IR (KBr) 2960, 2800, 1720, 1605, 1510, 1465, 1300, 1250, 1170, 1085, 1035, 815, 700, 560, 540 $cm^{-1}$.

Example 38

3-Benzhydryl-1-(2-hydroxybenzyl)-4-piperidinone

To a solution of 1 g of 3-benzhydryl-4-piperidinone hydrochloride, 0.5 g of 2-hydroxybenzyl alcohol and 1.7 ml of diisopropylethylamine in 15 ml of dichloromethane was added 1 ml of EPPA. After stirring at room temperature for 1 hour, the reaction mixture was washed with water, and the solvent was distilled off under reduced pressure. The residue was purified by subjecting it to silica gel column chromatography (100 g, ethyl acetate). Recrystallization from ethyl ether gave 0.78 g of the title compound (yield: 63%).
mp 109-110°C.
IR (KBr) 2840, 1730, 1590, 1495, 1470, 1450, 1415, 1365, 1260, 1235, 1090, 760, 700, 545 $cm^{-1}$.

Example 39

3-Benzhydryl-1-(3-hydroxybenzyl)-4-piperidinone

According to the same manner as that described in Example 38, the title compound was obtained (yield: 75%). mp 134-137°C.
IR (KBr) 3250, 3010, 2800, 1685, 1670, 1585, 1490, 1475, 1440, 1350, 1330, 1270, 1250, 750, 700, 685, 535 $cm^{-1}$.

Example 40

3-Benzhydryl-1-(4-hydroxybenzyl)-4-piperidinone

According to the same manner as that described in Example 38, the title compound was obtained (yield: 66%). mp 133-134°C.
IR (KBr) 2800, 1720, 1615, 1595, 1515, 1495, 1450, 1240, 750, 700 $cm^{-1}$.

To an ethanol solution of the title compound was added a calculated amount of conc. hydrochloric acid and the solvent was distilled off under reduced pressure. Ethyl ether was added to the residue, and the resulting crystalline powder was collected by filtration to obtain the hydrochloride of the title compound.
mp 149-150°C.
IR (KBr) 2930, 1735, 1610, 1585, 1510, 1465, 1450, 1300, 1210, 1030, 705 $cm^{-1}$.

Example 41

3-Benzhydryl-1-(4-hydroxy-3-methoxybenzyl)-4-piperidinone

According to the same manner as that described in Example 38, the title compound was obtained (yield: quantitative).
mp 131-132°C.
IR (KBr) 3010, 2950, 2800, 1715, 1600, 1515, 1485, 1460, 1445, 1340, 1270, 1230, 1150, 1025, 740, 700 $cm^{-1}$.

Example 42

3-Benzhydryl-1-(1, 3-benzodioxol-5-ylmethyl)-4-piperidinone

According to the same manner as that described in Example 32, the title compound was obtained (yield: 38%). mp 113-114°C.
IR (KBr) 3010, 2860, 2780, 1705, 1590, 1495, 1480, 1430, 1240, 1175, 1025, 920, 730 $cm^{-1}$.

Example 43

3-Benzhydryl-1-(2,3-dimethoxybenzyl)-4-piperidinone

According to the same manner as that described in Example 32, the title compound was obtained as an oil (yield: 54%).
$^1H$-NMR ($CDCl_3$) δ: 2.25-2.90(m, 6H), 3.35 (m, 1H), 3.50 and 3.59 (ABq, 2H, J=13 Hz), 3.81, 3.87 (each s, 6H), 4.64 (d, 1H, J=11 Hz), 6.80-7.32 (m, 13H).

To the above oil were added ethanol and a calculated amount of conc. hydrochloric acid, and the solvent was distilled off under reduced pressure. Ethyl ether was added to the residue, and the resulting crystalline powder was collected by filtration to obtain the hydrochloride of the title compound.
mp 157-160°C.
IR (KBr) 2900, 2450, 2360, 1730, 1580, 1480, 1445, 1425, 1270, 1075, 1000, 740, 695, 535 $cm^{-1}$.

Example 44

3-Benzhydryl-1-(2, 4-dimethoxybenzyl)-4-piperidinone

According to the same manner as that described in Example 32, the title compound was obtained as an oil. $^1$H-NMR ($CDCl_3$) δ: 2.35-3.00 (m, 6H), 3.40 (m, 1H), 3.54 (s, 2H), 3.78, 3.86 (each s, 6H), 4.64 (d, 1H, J=11 Hz), 6.47, 7.10-7.40 (m, 13H).

To the above oil were added ethanol and a calculated amount of conc. hydrochloric acid, and the solvent was distilled off under reduced pressure. Ethyl ether was added to the residue, and the resulting crystalline powder was collected by filtration to obtain 1.6 g of the hydrochloride of the title compound (yield: 71%).
mp 173-174°C.
IR (KBr) 2930, 1735, 1610, 1585, 1510, 1465, 1450, 1300, 1210, 1030, 705 $cm^{-1}$.

Example 45

3-Benzhydryl-1-(3, 4-dimethoxybenzyl)-4-piperidinone

According to the same manner as that described in Example 32, the title compound was obtained as an oil. $^1$H-NMR ($CDCl_3$) δ: 2.30-2.90 (m, 6H), 3.30-3.45 (m, 1H), 3.35 and 3.49 (ABq, 2H, J=13 Hz), 3.88, 3.91 (each s, 6H), 4.60 (d, 1H, J=11.4 Hz), 6.75-7.32 (m, 13H).

To the above oil, ethanol and a calculated amount of conc. hydrochloric acid were added, and the solvent was distilled off under reduced pressure. Ethyl ether was added to the residue, and the resulting crystalline powder was collected by filtration to obtain the hydrochloride of the title compound (yield: 73%).
mp 174-180°C.
IR (KBr) 2950, 2450, 2360, 1720, 1590, 1515, 1445, 1265, 1230, 1145, 1015, 700 $cm^{-1}$.

Example 46

3-Benzhydryl-1-(3,4,5-trimethoxybenzyl)-4-piperidinone

According to the same manner as that described in Example 32, the title compound was obtained as an oil (yield: 81%).
$^1$H-NMR ($CDCl_3$) δ: 2.40-2.90 (m, 6H), 3.30-3.45 (m, 1H), 3.43 and 3.56 (ABq, 2H, J=13 Hz), 3.85, 3.87 (each s, 9H), 4.56 (d, 1H, J=11 Hz), 7.05-7.35 (m, 12H).

To the above oil were added ethanol and a calculated amount of conc. hydrochloric acid, and the solvent was distilled off under reduced pressure. Ethyl ether was added to the residue, and the resulting crystalline powder was collected by filtration to obtain the title compound.
mp 182-185°C.
IR (KBr) 2925, 2500, 1720, 1585, 1500, 1465, 1445, 1420, 1330, 1240, 1180, 1160, 1120, 995, 850, 740, 700 $cm^{-1}$.

Example 47

3-Benzhydryl-1-(10,11-dihydro-5H-dibenzo[a,d]cyclohepten-5-yl)-4-piperidinone

According to the same manner as that described in Example 32, the title compound was obtained (yield: 48%).
mp 180-181°C.
IR (KBr) 3000, 2910, 2760, 1700, 1485, 1440, 1420, 1195, 1075, 760, 750, 700 $cm^{-1}$.

Example 48

3-Benzhydryl-1-(5H-dibenzo[a,d]cyclohepten-5-yl)-4-piperidinone

According to the same manner as that described in Example 32, the title compound was obtained (yield: 88%).
mp 175-178°C.
IR (KBr) 3030, 2780, 1715, 1495, 1445, 1310, 1250, 1090, 810, 800, 775, 750, 710, 550, 480 $cm^{-1}$.

Example 49

3-Benzhydryl-1-(4-methoxybenzyl)-4-piperidinone

According to the same manner as that described in Example 32, 0.85 g of the title compound was obtained (yield: 66%).
mp 103-104°C.
IR (KBr) 3025, 2950, 2780, 171.0, 1610, 1510, 1240, 1030, 740, 700, 540 $cm^{-1}$.

Example 50

3-Benzhydryl-1-(9H-xanthen-9-yl)-4-piperidinone

According to the same manner as that described in Example 32, the title compound was synthesized (yield: 39%).
mp 180-181°C.
IR (KBr) 3020, 2900, 2780, 1705, 1595, 1565, 1485, 1470, 1450, 1330, 1320, 1250, 1200, 1190, 1180, 895, 760, 750, 695, 535 $cm^{-1}$.

Example 51

3-Benzhydryl-1-(9H-thioxanthen-9-yl)-4-piperidinone

According to the same manner as that described in Example 32, the title compound was synthesized (yield: 39%).
mp 186-188°C.
IR (KBr) 2940, 2800, 1715, 1590, 1500, 1475, 1440, 1335, 750, 710 $cm^{-1}$.

Example 52

3-Benzhydryl-1-cinnamyl-4-piperidinone

According to the same manner as that described in Example 32, the title compound was synthesized (yield: 61%).
mp 114-115°C.
IR (KBr) 3010, 2780, 1710, 1590, 1485, 1440, 1330, 1170, 1125, 1030, 965, 735, 700, 685, 540 $cm^{-1}$.

Example 53

3-Benzhydryl-1-(pyridin-3-ylmethyl)-4-piperidinone

According to the same manner as that described in Example 32, the title compound was synthesized (yield: 40%).
mp 121-122°C.
IR (KBr) 3025, 2950, 2910, 2800, 1710, 1590, 1570, 1490, 1445, 1420, 1345, 1185, 1085, 1025, 780, 700, 550 $cm^{-1}$.

Example 54

3-Benzhydryl-1-(pyridine-2-ylmethyl)-4-piperidinone

According to the same manner as that described in Example 32, the title compound was synthesized (yield: 36%).
mp 100°C.
IR (KBr) 3025, 2900, 2800, 1710, 1585, 1565, 1490, 1470, 1450, 1430, 1340, 1185, 1080, 990, 780, 760, 740, 700, 690, 545, 490 $cm^{-1}$.

Example 55

3-Benzhydryl-1-[(3-hydroxypyridin-2-yl)methyl]-4-piperidinone

According to the same manner as that described in Example 32, the title compound was synthesized (yield: 61%).
mp 121-122°C.
IR (KBr) 3025, 2950, 2820, 1710, 1595, 1575, 1490, 1445, 1415, 1355, 1335, 1260, 1235, 1220, 1165, 1115, 1090, 980, 800, 775, 740, 700, 690, 540 $cm^{-1}$.

Example 56

3-Benzhydryl-1-[(3-hydroxy-6-methylpyridin-2-yl)methyl]-4-piperidinone

According to the same manner as that described in Example 32, the title compound was synthesized (yield: 78%).
mp 164-165°C.
IR (KBr) 3020, 2920, 2800, 2500, 1715, 1575, 1490, 1470, 1445, 1420, 1340, 1300, 1275, 1190, 1165, 1125, 840, 740, 700, 635, 600, 540 $cm^{-1}$.

Example 57

3-Benzhydryl-1-(2-propargyl)-4-piperidinone

According to the same manner as that described in Example 32, the title compound was synthesized (yield: 20%).
mp 85-87°C.
IR (KBr) 3250, 3025, 2800, 2795, 1710, 1595, 1495, 1470, 1450, 1340, 1315, 1195, 800, 780, 750, 700, 540 $cm^{-1}$.

Example 58

3-Benzhydryl-1-(1H-indol-3-ylmethyl)-4-piperidinone

According to the same manner as that described in Example 32, the title compound was synthesized (yield: 36%).
mp 170-171°C.
IR (KBr) 3270, 3050, 2800, 1700, 1490, 1450, 1435, 1350, 1240, 1195, 1095, 1090, 1005, 740, 700, 545 $cm^{-1}$.

Example 59

3-Benzhydryl-1-(2-thienylmethyl)-4-piperidinone

According to the same manner as that described in Example 32, the title compound was synthesized as an oil (yield: 48%).
$^1H$-NMR ($CDCl_3$) δ: 2.27-2.95 (m, 6H), 3.33-3.45 (m, 1H), 3.67 and 3.76 (ABq, 2H, J=14 Hz), 4.66 (d, 1H, J=11.2 Hz), 6.84-7.36 (m, 13H).
The above oil was dissolved in ethanol, conc. hydrochloric acid was added thereto, and the solvent was distilled off under reduced pressure. Ethyl ether was added to the residue, and the resulting crystalline powder was

collected by filtration to obtain the hydrochloride of the title compound.
mp 177-179°C.
IR (KBr) 3425, 3025, 2440, 2375, 1730, 1490, 1450, 1370, 855, 745, 700 $cm^{-1}$.

Example 60

3-Benzhydryl-1-(2-furylmethyl)-4-piperidinone

According to the same manner as that described in Example 32, the title compound was synthesized as an oil (yield: 52%).
$^1$H-NMR ($CDCl_3$) δ: 2.30-2.90 (m, 6H), 3.35-3.45 (m, 1H), 3.46 and 3.63 (ABq, 2H, J=14 Hz), 4.57 (d, 1H, J=10 Hz), 6.11-7.40 (m, 13H).

The above oil was dissolved in ethanol, conc. hydrochloric acid was added thereto, and the solvent was distilled off under reduced pressure. Ethyl ether was added to the residue, and the resulting crystalline powder was collected by filtration to obtain the hydrochloride of the title compound.
mp 188-190°C.
IR (KBr) 3425, 2900, 2425, 2390, 1730, 1600, 1495, 1450, 1150, 1020, 750, 700, 600, 545 $cm^{-1}$.

Example 61

3-Benzhydryl-1-[(4-methyl-1H-imidazol-5-yl)methyl]-4-piperidinone

According to the same manner as that described in Example 32, the synthesis was carried out. Purification by silica gel column chromatography (dichloromethane : methanol = 4 : 1) gave the title compound (yield: 51%). $^1$H-NMR ($CDCl_3$) δ: 2.11 (s, 3H), 2.50, 2.80 (each m, 6H), 3.30-3.55 (m, 1H), 3.38 and 3.51 (ABq, 2H, J=13.6 Hz), 4.55 (d, 1H, J=11.4 Hz), 7.00-7.46 (m, 11H).

The above oil was dissolved in ethanol, conc. hydrochloric acid was added thereto, and the solvent was distilled off under reduced pressure. Ethyl ether was added to the residue, and the resulting crystalline powder was collected by filtration to obtain the hydrochloride of the title compound.
mp 172-174°C.
IR (KBr) 3450, 3000, 2650, 1720, 1640, 1490, 1450, 1370, 1190, 745, 700, 690, 630, 635 $cm^{-1}$.

Example 62

3-Benzhydryl-1-[(E)-3-(2-methoxyphenyl)-2-propenyl]-4-piperidinone

To a solution of 1.62 g of 3-(2-methoxyphenyl)acrylaldehyde in 20 ml of ethanol was added 0.5 g of sodium borohydride, and the mixture was stirred at room temperature for several hours. Then, 30 ml of ethyl acetate was added and the mixture was washed three times with brine. After drying over magnesium sulfate, the solvent was distilled off under reduced pressure. The residue was dissolved in 20 ml of dichloromethane, and 3 g of 3-benzhydryl-4-piperidinone hydrochloride, 5.1 g of diisopropyl ethylamine and 2.4 ml of EPPA were added thereto with stirring under ice-cooling. After allowing to stand at room temperature for 16 hours, the solvent was distilled off under reduced pressure. The residue was subjected to silica gel column chromatography (100 g, ethyl acetate : hexane = 1 : 1) eluting with the same solvent. The desired fraction was concentrated and precipitated crystals were triturated with ethyl ether and filtered to obtain 1.39 g of the title compound (yield: 34%).
mp 117-118°C.
IR (KBr) 2800, 1715, 1600, 1490, 1470, 1450, 1240, 1030, 755, 705, 545 $cm^{-1}$.

Example 63

3-Bnzhydryl-1-[2-(2-methoxyphenyl)ethaneimidoyl]-4-piperidinone

A solution of 3 g of 3-benzhydryl-4-piperidinone hydrochloride, 3.5 g of ethyl(2-methoxyphenyl)acetoimidate hydrochloride and 3.5 ml of triethylamine in 20 ml of dichloromethane was stirred at 40°C for 16 hours. After washing with water and drying over magnesium sulfate, the solvent was distilled off under reduced pressure. The residue was purified by subjecting it to silica gel column chromatography (100 g, dichloromethane : methanol : trimethylamine = 8 : 2 : 1). The solvent was distilled off, and the residue (crystal) was triturated with ethyl ether and filtered to obtain 0.78 g of the title compound (yield: 19%).
mp 57-58°C.

IR (KBr) 3400, 3300, 3000, 1715, 1580, 1490, 1465, 1450, 1250, 1110, 1020, 760, 705 $cm^{-1}$.

Example 64

3-[Bis(4-chlorophenyl)methyl]-1-[2-(2-methoxyphenyl)ethaneimidoyl]-4-piperidinone

A solution of 1 g of 3-[bis(4-chlorophenyl)methyl-4-piperidinone hydrochloride, 0.8 g of ethyl2-methoxyphenylacetoimidate hydrochloride and 1 ml of triethylamine in 10 ml of dichloromethane was refluxed for 48 hours. After washing with water and drying over magnesium sulfate, the solvent was distilled off under reduced pressure. The residue was purified by subjecting it to silica gel column chromatography (100 g, dichloromethane : methanol : triethylamine = 8 : 2 : 1). The solvent was distilled off to obtain 0.38 g of the title compound as a powder (amorphous, yield: 29%).
IR (KBr) 2920, 1715, 1600, 1495, 1460, 1250, 1110, 1090, 1015, 795, 750, 540 $cm^{-1}$.

Example 65

3-[Bis(4-fluorophenyl)methyl]-1-[2-(2-methoxyphenyl)ethaneimidoyl]-4-piperidinone

A solution of 1.5 g of 3-[bis(4-fluorophenyl)methyl]-4-piperidinone hydrochloride, 0.8 g of ethyl 2-methoxyphenylacetoimidate hydrochloride and 1 ml of triethylamine in 10 ml of dichloromethane was refluxed for 20 hours. After washing with water and drying over magnesium sulfate, the solvent was distilled off under reduced pressure. The residue was purified by subjecting it to silica gel column chromatography (100 g, dichloromethane : methanol : triethylamine = 8 : 2 : 1). The solvent was distilled off to obtain 0.6 g of the title compound as a powder (yield: 30%).
IR (KBr) 2925, 2840, 1715, 1600, 1510, 1460, 1440, 1250, 1220, 1160, 1105, 1050, 1030, 825, 755, 550, 530 $cm^{-1}$.

Example 66

3-Benzhydryl-1-(3-methyl-1, 2, 4-thiadiazol-5-yl)-4-piperidinone

A solution of 1.5 g of 3-benzhydryl-4-piperidinone hydrochloride, 1.3 g of 5-chloro-3-methyl-1, 2, 4-thiazole and 1.4 ml of triethylamine in 10 ml of dichloromethane was allowed to stand at room temperature for 48 hours. The reaction mixture was washed with water and then dried over magnesium sulfate. The solvent was distilled off under reduced pressure and the residue (crystal) was triturated with ethyl ether and filtered to obtain 1.3 g of the title compound (yield: 72%).
mp 164-166°C.
IR (KBr) 2900, 2860, 1715, 1560, 1490, 1450, 1385, 1315, 795, 745, 705, 540 $cm^{-1}$.

Example 67

N-[(3-Benzhydryl-4-oxo-1-piperidinyl)carbothioyl]benzamide

A solution of 3 g of 3-benzhydryl-4-piperidinone hydrochloride, 1.6 g of benzoylisothiocyanate and 1.4 ml of triethylamine in 30 ml of dichloromethane was stirred at room temperature for 5 hours. The solvent was distilled off under reduced pressure. Water and ethyl ether were added to the residue to precipitate crystals. Filtration gave 4 g of the title compound (yield: 93%).
mp 168-169°C.
IR (KBr) 3200, 1725, 1700, 1600, 1530, 1455, 1430, 1240, 1170, 1150, 855, 755, 720, 700, 655 $cm^{-1}$.

Example 68

3-Benzhydryl-4-oxo-1-piperidinecarboxamide

A solution of 1 g of 3-benzhydryl-4-piperidinone hydrochloride and 0.35 g of potassium isocyanate in 20 ml of ethanol and 10 ml of water was stirred at room temperature for 16 hours. Crystals precipitated were collected by filtration to obtain 1 g of the title compound (yield: 97%).
mp 88-89°C.
IR (KBr) 3540, 3380, 3220, 1705, 1675, 1615, 1585, 1495, 1480, 1450, 1310, 1090, 710, 545 $cm^{-1}$.

Example 69

3-Benzhydryl-4-oxo-1-piperidinecarbothioamide

A solution of 3 g of 3-benzhydryl-4-piperidinone hydrochloride and 1.1 g of potassium isothiocyanate in 100 ml of ethanol was refluxed for 16 hours. After the solvent was distilled off under reduced pressure, the residue was extracted with ethyl acetate and then the extract was washed with water. After drying over magnesium sulfate, the solvent was distilled off under reduced pressure. The residue was dissolved in ethanol and allowed to stand. Crystals were collected by filtration to obtain 1.3 g of the title compound (yield: 40%).
mp 107°C.
IR (KBr) 3500, 3425, 3300, 318.0, 1705, 1650, 1625, 1600, 1500, 1450, 1395, 1370, 1310, 1090, 980, 705, 550 $cm^{-1}$.

Example 70

3-Benzhydryl-N-(2-methoxyphenyl)-4-oxo-1-piperidinecarboxamide

A solution of 3 g of 3-benzhydryl-4-piperidinone hydrochloride, 1.5 g of orthomethoxyphenylisocyanate and 1.4 ml of triethylamine in 30 ml of dichloromethane was stirred at room temperature for 2 hours. The solvent was distilled off under reduced pressure, and water and hexane were added to the residue. Crystals precipitated were collected by filtration to obtain 3.98 g of the title compound (yield: 96%).
mp 150-151°C.
IR (KBr) 3450, 3030, 1720, 1655, 1600, 1530, 1490, 1460, 1390, 1335, 1295, 1250, 1210, 1115, 1020, 755, 710, 540 $cm^{-1}$.

Example 71

3-Benzhydryl-N-(2-methoxyphenyl)-4-oxo-1-piperidinecarbothioamide

A solution of 3 g of 3-benzhydryl-4-piperidinone hydrochloride, 1.65 g of orthomethoxyphenyl isothiocyanate and 1.4 ml of triethylamine in 30 ml of dichloromethane was stirred at room temperature for 2 hours. The reaction mixture was washed with water and dried over magnesium sulfate, and then the solvent was distilled off under reduced pressure to obtain 3 g of the title compound as an amorphous powder (yield: 70%).
IR (KBr) 3390, 2925, 1720, 1600, 1500, 1450, 1330, 1240, 1110, 1020, 745, 700, 540 $cm^{-1}$.

Example 72

3-(3-Benzhydryl-4-oxo-1-piperidinyl)propanenitrile

A solution of 1 g of 3-benzhydryl-4-piperidinone hydrochloride, 0.1 g of acrylonitrile and 1.52 g of DBU in 10 ml of dichloromethane was stirred for 3 hours. After the solvent was distilled off under reduced pressure, the residue was subjected to silica gel column chromatography (100 g, ethyl acetate) eluting with ethyl acetate. The solvent was distilled off under reduced pressure to obtain the title compound as an oil.
$^1$H-NMR ($CDCl_3$) $\delta$: 2.30-2.45, 2.55-2.84. 2.69-3.10, 3.35-3.50 (each m, 11H), 4.65 (d, 1H, J=11.4 Hz), 7.10-7.40 (m, 10H).
The above oil was dissolved in ethanol, 0.3 ml of conc. hydrochloric acid was added thereto, and the solvent was distilled off under reduced pressure. Crystals were triturated with ethyl ether and filtered to obtain 0.6 g of the hydrochloride of the title compound (yield: 51%).
mp 163-166°C.
IR (KBr) 3000, 2300, 1730, 1595, 1495, 1450, 1410, 1380, 750, 705, 540 $cm^{-1}$.

Example 73

3-Benzhydryl-4-oxopiperidine-1-sulfonate sodium

A solution of 1 g of 3-benzhydryl-4-piperidinone hydrochloride, 1.5 g of pyridine sulfuric acid complex and 0.6 ml of diisopropyl ethylamine in 5 ml of dimethylformamide was stirred for 1 hour. Then, 30 ml of 1 N aqueous solution of sodium hydroxide was added thereto under ice-cooling, and the mixture was applied to Amberite XAD-2 column and eluted with water and then methanol. The yellow solution of the desired fraction was concentrated under reduced

pressure. After the residue was crystallized, it was lyophilized to obtain 1.1 g of the title compound (yield: 90%).
mp 136-140°C.
IR (KBr) 3450, 3025, 2830, 1710, 1625, 1595, 1560, 1230, 1190, 1040, 980, 920, 740, 700, 630, 610, 540 $cm^{-1}$.

Example 74

N-[(E)-1-[(3-Benzhydryl-4-oxo-1-piperidinyl)carbonyl]-2-(3-piperidinyl)ethenyl]acetamide

A solution of 3 g of 3-benzhydryl-4-piperidinone hydrochloride, 1.9 g of 2-methyl-4-(3-pyridylmethylidene)-5-oxazolone and 1.4 ml of triethylamine in 10 ml of dimethylformamide was stirred at room temperature for 5 hours. After addition of 30 ml of water and stirring at room temperature for 16 hours, crystals precipitated were collected by filtration and washed with a small amount of ethanol and then ethyl ether to obtain 2 g of the title compound (yield: 44%).
mp 224-225°C.
IR (KBr) 3550, 3280, 3020, 1710, 1680, 1660, 1640, 1515, 1430, 1370, 1300, 1280, 1265, 860, 750, 705, 540 $cm^{-1}$.

Example 75

3-[Bis(4-fluorophenyl)niethyl]-1-(2-hydroxybenzyl)-4-piperidinone

According to the same manner as that described in Example 38, the title compound was obtained (yield: 81%).
mp 151-152°C.
IR (KBr) 2820, 1710, 1600, 1585, 1500, 1485, 1460, 1350, 1315, 1230, 1220, 1190, 1155, 825, 760, 545, 475 $cm^{-1}$.

Example 76

3-[Bis(4-fluorophenyl)methyl]-1-(5-bromo-2-methoxybenzyl)-4-piperidinone

According to the same manner as that described in Example 32, the title compound was synthesized (yield: 66%).
mp 129-130°C.
IR (KBr) 2775, 1705, 1600, 1515, 1485, 1440, 1250, 1220, 1155, 1115, 1030, 825, 795, 620, 560, 550, 525 $cm^{-1}$.

Example 77

3-[Bis(4-fluorophenyl)methyl]-1-(3-methoxybenzyl)-4-piperidinone .
According to the same manner as that described in Example 32, the title compound was obtained as an oil.
$^1$H-NMR ($CDCl_3$) δ: 2.25-2.95 (m, 6H), 3.26 (m, 1H), 3.40 and 3.54 (ABq, 2H, J=13 Hz), 3.83 (s, 3H, OMe), 4.59 (d, 1H, J=11 Hz), 6.75-7.85 (m, 12H).
The above oil was dissolved in ethanol, a calculated amount of conc. hydrochloric acid was added thereto, and the solvent was distilled off under reduced pressure. To the residue was added ethyl ether, the mixture was stirred for 16 hours, and then the resulting powder was collected by filtration and dried to obtain the hydrochloride of the title compound (yield: 67%).
mp 161-163°C.
IR (KBr) 2925, 2600, 2550, 1730, 1600, 1585, 1505, 1490, 1445, 1440, 1270, 1220, 1180, 1160, 1050, 830, 785, 695, 54 5 $cm^{-1}$.

Example 78

3-[Bis(4-fluorophenyl)methyl]-1-(2,3,4-trimethoxybenzyl)-4-piperidinone

According to the same manner as that described in Example 32, the title compound was obtained as an oil.
$^1$H-NMR ($CDCl_3$) δ: 2.25-2.95 (m, 6H), 3.20 (m, 1H), 3.31 and 3.53 (ABq, 2H, J=13 Hz), 2.85 (s, 3H), 3.88 (s, 6H), 4.55 (d, 1H, J=11 Hz), 6.58-7.26 (m, 10H).
The above oil was dissolved in ethanol, a calculated amount of conc. hydrochloric acid was added thereto, and the solvent was distilled off under reduced pressure. To the residue was added ethyl ether, the mixture was stirred for 16 hours, and then the resulting powder was collected by filtration and dried to obtain the hydrochloride of the title compound (yield: 64%).
mp 174-176°C.

IR (KBr) 2925, 2460, 1600, 1505, 1470, 1420, 1280, 1225, 1160, 1100, 1010, 825, 800, 550 $cm^{-1}$.

Example 79

3-[(4-Fluorophenyl)(phenyl)methyl]-1-(2-methoxybenzyl)-4-piperidinone

To a solution of 1 g of 1-(2-methoxybenzyl)-4-piperidinone in 20 ml of dichloromethane was added 2 ml of TMSOTf under ice-cooling. Then, 1 g of 4-fluorobenzhydrol was added and the mixture was stirred at room temperature for 16 hours. Then, 20 ml of water was added thereto, and the mixture was neutralized with sodium bicarbonate. The dichloromethane layer was dried over magnesium sulfate, and the solvent was distilled off under reduced pressure. The residue (crystal) was triturated with hexane and filtered to obtain 0.45 g of the title compound (yield: 22%).
mp 115-116°C.
IR (KBr) 2800, 1710, 1600, 1505, 1495, 1245, 1220, 1030, 810 $cm^{-1}$.

Example 80

3-[Bis(4-fluorophenyl)methyl]-1-[3,5-bis(trifluoromethyl)benzyl]-4-piperidinone

(Step 1) To 10 ml of dimethylformamide were added 2.33 g of 4-piperidinone ethyleneketal, 5 g of 3, 5-bis (trifluoromethyl)benzyl bromide and 2.25 g of potassium carbonate with stirring. After completion of an exothermic reaction, the mixture was stirred in an oil bath at 65°C for 60 minutes. The mixture was extracted with 30 ml of ethyl acetate and the extract was washed with water, and then the solvent was distilled off under reduced pressure. To the residue were added 20 ml of water, 20 ml of acetone and 2.7 ml of conc. hydrochloric acid, and the mixture was stirred in an oil bath at 65°C for 16 hours. After cooling, 30 ml of dichloromethane was added and the mixture was neutralized with sodium bicarbonate. The dichloromethane layer was dried over magnesium sulfate, and the solvent was distilled off under reduced pressure to obtain 3.8 g of 1-[3,5-bis(trifluoromethyl)benzyl]-4-piperidinone (yield: 72%).
$^1$H-NMR ($CDCl_3$) δ: 2.50 (t, 4H, J=6 Hz), 2.79 (t, 4H, J=6 Hz), 3.74 (s, 2H), 7.70-7.90 (m, 3H).
(Step 2) To a solution of 1.4 g of 1-[3, 5-bis(trifluoromethyl)benzyl]-4-piperidinone obtained in Step 1 in 15 ml of dichloromethane was added 2 ml of TMSOTf under ice-cooling. Then, 1,1 g of 4,4'-difluorobenzhydrol was added thereto and the mixture was stirred at room temperature for 16 hours. To this was added 20 ml of water and the mixture was neutralized with sodium carbonate. The dichloromethane layer was dried over magnesium sulfate, and the solvent was distilled off under reduced pressure. The residue (crystal) was triturated with hexane and filtered to obtain 1.4 g of the title compound. Recrystallization from a small amount of hot ethyl acetate gave 0.97 g (yield: 37%).
mp 205-206°C.
IR (KBr) 2800, 1710, 1505, 1350, 1280, 1220, 1170, 1120, 885, 830, 700, 680, 555, 525 $cm^{-1}$.

Example 81

3-Benzhydryl-1-[3,5-bis(trifluoromethyl)benzyl]-4-piperidinone

According to the same manner as that described in Example 80, the title compound was obtained (yield: 44%).
mp 180-182°C.
IR (KBr) 2960, 2800, 1710, 1495, 1450, 1355, 1345, 1280, 1170, 1120, 880, 750, 700, 680, 545 $cm^{-1}$.

Example 82

3-[Bis(4-fluorophenyl)methyl]-1-(2-fluorobenzyl)-4-piperidinone

According to the same manner as that described in Example 80, the title compound was obtained as an oil (yield: 56%).
$^1$H-NMR ($CDCl_3$) δ: 2.25-3.00 (m, 6H), 3.20 (m, 1H), 3.51 and 3.60 (ABq, 2H, J=13 Hz), 4.59 (d, 1H, J=11 Hz), 6.75-7.35 (m, 12H).
The above oil was dissolved in ethyl ether, and a calculated amount of a solution of 4 N Cl/dioxane was added thereto. Crystals precipitated were collected by filtration to obtain the hydrochloride of the title compound.
mp 162-164°C.
IR (KBr) 2990, 2525, 1730, 1600, 1505, 1455, 1415, 1220, 1185, 1155, 1115, 880, 760, 550 $cm^{-1}$.

Example 83

3-Benzhydryl-1-(2-fluorobenzyl)-4-piperidinone

According to the same manner as that described in Example 80, the hydrochloride of the title compound was obtained (yield: 50%).
mp 165-166°C.
IR (KBr) 1730, 1620, 1585, 1495, 1450, 1240, 1190, 1110, 1030, 760, 740, 700 $cm^{-1}$.

Example 84

3-[Bis(4-fluorophenyl)methyl]-1-[2-(2-methoxyphenyl)-2-oxoethyl]-4-piperidinone

To 10 ml of dimethylformamide were added 1.1 g of 3-[bis(4-fluorophenyl)methyl]-4-piperidinone hydrochloride, 0.8 g of 2-bromo-2'-methoxyacetophenone and 1.4 g of potassium carbonate and the mixture was stirred at room temperature for 16 hours. After dissolving in 30 ml of ethyl acetate, the mixture was washed with water, and then dried over magnesium sulfate, and the solvent was distilled off under reduced pressure. The residue was recrystallized from ethyl acetate to obtain 0.88 g of the title compound (yield: 59%).
mp 148-149°C.
IR (KBr) 2975, 2910, 2850, 1710, 1660, 1595, 1510, 1290, 1250, 1220, 1165, 830, 765, 560, 530 $cm^{-1}$.

Example 85

3-[Bis(4-fluorophenyl)methyl]-1-(2-fluoromethoxybenzyl)-4-piperidinone

To 5 ml of dimethylformamide were added 1 g of 3-[bis(4-fluorophenyl)methyl]-1-(2-hydroxybenzyl)-4-piperidinone, 1 ml of 2.75 M solution of bromofluoromethane dichloromethane, 0.5 g of potassium carbonate and 0.48 ml of diisopropylethylamine, and the mixture was stirred at room temperature for 26 hours. After dissolving in 30 ml of ethyl acetate, the mixture was washed with water, and then dried over magnesium sulfate, and the solvent was distilled off under reduced pressure. The residue was subjected to silica gel column chromatography (40 g, ethyl acetate : hexane = 1 : 1) to obtain 1.2 g of the title compound as an oil.
$^1$H-NMR ($CDCl_3$) δ: 2.30-3.00 (m, 6H), 3.25 (m, 1H) , 3.52 and 3.62 (ABq, J=13.3 Hz), 4.58 (d, 1H, J=11 Hz), 5.70 (d, 2H, J=54.6 Hz), 6.80-7.35 (m, 12H).

The above oil was dissolved in 10 ml of ethyl ether, and 0.65 ml of a solution of 4 N HCl/dioxane was added thereto. The solvent was distilled off under reduced pressure. To the residue was added ethyl ether to obtain 1.2 g (quantitative) of the hydrochloride of the title compound as a powder (amorphous).
IR (KBr) 1730, 1605, 1510, 1460, 1420, 1235, 1160, 1130, 1080, 980, 830, 760 $cm^{-1}$.

Example 86

3-[Bis(4-fluorophenyl)methyl]-1-(2-isopropoxybenzyl)-4-piperidinone

To 5 ml of dimethylformamide were added 1 g of 3-[bis(4-fluorophenyl)methyl]-1-(2-hydroxybenzyl)-4-piperidinone, 1.3 ml of isopropyl iodide and 2.8 g of potassium carbonate and the mixture was stirred in an oil bath at 100°C for 16 hours. The solvent was replaced with 30 ml of ethyl acetate, the solution was washed with water, and then dried over magnesium sulfate, and the solvent was distilled off under reduced pressure. The residue was purified by subjecting it to a silica gel column chromatography (40 g, ethyl acetate : hexane = 1 : 1). The residue (crystal) was triturated with hexane and filtered to obtain 0.35 g of the title compound (yield: 31%).
mp 155-156°C.
IR (KBr) 2975, 2810, 1710, 1605, 1510, 1490, 1245, 1220, 1115, 960, 835, 760, 560, 530 $cm^{-1}$.

Example 87

3-[Bis(4-fluorophenyl)methyl]-1-(pyridin-3-ylmethyl)-4-piperidinone

To 7 ml of dimethylformamide were added 1.1 g of 3-[bis(4-fluorophenyl)methyl]-4-piperidinone hydrochloride, 0.66 g of 3-chloromethylpyridine hydrochloride, 1.4 g of potassium carbonate and the mixture was stirred at room temperature for 7 hours. The mixture was extracted with 30ml of ethyl acetate, the extract was washed with water, and

then dried over magnesium sulfate, and the solvent was distilled off under reduced pressure. The residue was purified by subjecting it to silica gel column chromatography (40 g, ethyl acetate : hexane = 1 : 1) to obtain 1 g of the title compound as an oil.
$^1$H-NMR ($CDCl_3$) δ: 2.30-2.95 (m, 6H), 3.27 (m, 1H), 3.42 and 3.58 (ABq, 2H, J=13.3 Hz), 4.57 (d, 1H, J=11.3 Hz), 6.80-7.30, 7.58, 8.55 (each m, 12H).

The above oil was dissolved in ethyl ether and 1.3 ml of a solution of 4 N HCl/dioxane was added thereto to obtain 1 g of the hydrochloride of the title compound as a powder (amorphous, yield: 68%).
IR (KBr) 3425, 1730, 1605, 1510, 1225, 1130, 835 $cm^{-1}$.

Example 88

3-[Bis(4-fluorophenyl)methyl]-1-[[2-(2-diethylamino)ethoxy]benzyl]-4-piperidinone

To 10 ml of dimethylformamide were added 1 g of 3-[bis(4-fluorophenyl)methyl]-1-(2-hydroxybenzyl)-4-piperidinone, 1.7 g of diethylaminoethyl chloride hydrochloride and 2.8 g of potassium carbonate and the mixture was stirred in an oil bath at 90°C for 7 hours. The mixture was extracted with 30 ml of ethyl acetate, the extract was washed with water, and dried over magnesium sulfate, and the solvent was distilled off under reduced pressure. The residue was purified by subjecting it to silica gel column chromatography (40 g, ethyl acetate : methanol = 4 : 1) to obtain 0.7 g of the title compound as an oil.
$^1$H-NMR ($CDCl_3$) δ: 1.07 (t, 6H, J=7.2 Hz), 2.35-3.00 (m, 6H), 2.64 (q, 4H, J=7.2 Hz), 2.83 (t, 2H, J=6.2 Hz), 3.28 (m, 1H), 3.48 and 3.64 (ABq, 2H, J=13.2 Hz), 4.02 (t, 2H, J=6.2 Hz), 4.57 (d, 1H, J=11.4 Hz), 6.80-7.29 (m, 12H).

The above oil was dissolved in ethyl ether and 1.3 ml of a solution of 4 N HCl/dioxane thereto to obtain the hydrochloride of the title compound as a powder (amorphous, yield: 48%).
IR (KBr) 3450, 1730, 1605, 1510, 1460, 1225, 1160, 1120, 840, 760 $cm^{-1}$.

Example 89

3-[Bis(4-fluorophenyl)methyl]-1-[(3, 5-dimethylisooxazol-4-yl)methyl]-4-piperidinone

To 10 ml of dimethylformamide were added 1.1 g of 3-[bis(4-fluorophenyl)methyl]-4-piperidinone hydrochloride, 0.5 g of 4-chloromethyl-3, 5-dimethylisooxazole and 1.4 g of potassium carbonate and the mixture was stirred at room temperature for 16 hours. The mixture was extracted with 30 ml of ethyl acetate, the extract was washed with water, and dried over magnesium sulfate, and the solvent was distilled off under reduced pressure to obtain the title compound as an oil.
$^1$H-NMR ($CDCl_3$) δ: 2.22, 2.32 (each s, 6H), 2.30-2.70 (m, 6H), 2.80 (m, 1H), 3.19 (s, 2H), 4.50 (d, 1H, J=11 Hz), 6.89-7.26 (m, 8H).

The above oil was dissolved in ethyl ether and 1 ml of a solution of 4 N HCl/dioxane was added thereto to obtain 1.2 g of the hydrochloride of the title compound as a crystalline powder (yield: 81%).
mp 114-116°C.
IR (KBr) 3450, 1730, 1630, 1605, 1510, 1420, 1230, 1160, 830, 555 $cm^{-1}$.

Example 90

3-[Bis(4-fluorophenyl)methyl]-1-(quinolin-2-ylmethyl)-4-piperidinone

To 10ml of dimethylformamide were added 1.1 g of 3-[bis(4-fluorophenyl)methyl]-4-piperidinone hydrochloride, 0.8 g of 2-chloromethylquinoline hydrochloride and 2.8 g of potassium carbonate and the mixture was stirred at room temperature for 16 hours. The mixture was extracted with 30 ml of ethyl acetate, the extract was washed with water, and dried over magnesium sulfate, and the solvent was concentrated under reduced pressure. Crystals precipitated were collected by filtration to obtain 1.1 g of the title compound (yield: 73%).
mp 188-189°C.
IR (KBr) 2820, 1705, 1600, 1510, 1345, 1310, 1205, 1165, 1110, 855, 835, 760, 555 $cm^{-1}$.

Example 91

3-[Bis(4-fluorophenyl)methyl]-1-(4-trifluoromethylbenzyl)-4-piperidinone

According to the same manner as that described in Example 80, the title compound was obtained (yield: 33%).

mp 126-127°C.
IR (KBr) 2950, 2825, 1715, 1600, 1505, 1415, 1325, 1220, 1165, 1120, 1065, 825, 545 $cm^{-1}$.

Example 92

3-Benzhydryl-1-(4-trifluoromethylbenzyl)-4-piperidinone

According to the same manner as that described in Example 80, the title compound was obtained as an oil.
$^1H$-NMR ($CDCl_3$) δ: 2.30-2.95 (m, 6H), 3.37 (m, 1H), 3.45 and 3.60 (ABq, 2H, J=13 Hz), 4.60 (d, 1H, J=11,2 Hz), 7.08-7.57 (m, 14H).

The above oil was dissolved in ethyl ether, a calculated amount of a solution of 4 N HCl/dioxane was added thereto, and the mixture was stirred. Crystals were collected by filtration to obtain the hydrochloride of the title compound (yield: 52%).
mp 168-169°C.
IR (KBr) 2500, 1735, 1495, 1455, 1420, 1330, 1165, 1130, 1070, 700 $cm^{-1}$.

Example 93

3-[Bis(4-fluorophenyl)methyl]-1-(3-trifluoromethylbenzyl)-4-piperidinone

According to the same manner as that described in Example 80, the title compound was obtained (yield: 43%).
mp 150-151°C.
IR (KBr) 2950, 2800, 1705, 1600, 1510, 1450, 1330, 1225, 1170, 1160, 1120, 825, 785, 700, 660, 560, 525 $cm^{-1}$.

Example 94

3-Benzhydryl-1-(3-trifluoromethylbenzyl)-4-piperidinone

According to the same manner as that described in Example 80, the title compound was obtained (yield: 25%).
mp 128-129°C.
IR (KBr) 1700, 1490, 1450, 1325, 1160, 1115, 1070, 785, 740, 700, 545 $cm^{-1}$.

Example 95

3-[Bis(4-fluorophenyl)methyl]-1-(2-trifluoromethylbenzyl)-4-piperidinone

According to the same manner as that described in Example 80, the title compound was obtained (yield: 75%).
mp 102-103°C.
IR (KBr) 2900, 1700, 1605, 1510, 1450, 1380, 1340, 1315, 1255, 1220, 1155, 1115, 1035, 1025, 825, 765, 725, 560, 530.

Example 96

3-Benzhydryl-1-(2-trifluoromethylbenzyl)-4-piperidinone

According to the same manner as that described in Example 80, the title compound was obtained (yield: 60%).
$^1H$-NMR ($CDCl_3$) δ: 2.30-2.95 (m, 6H), 3.38 (m, 1H), 3.60 and 3.72 (ABq, 2H, J=14 Hz), 4.64 (d, 1H, J=11.2 Hz), 7.09-1.74 (m, 14H).

The above oil was dissolved in ethyl ether and a calculated amount of a solution of 4 N HCl/dioxane was added thereto. The solvent was distilled off under reduced pressure, and ethyl ether was added to the crystalline residue and crystals were collected by filtration to obtain the hydrochloride of the title compound.
mp 138-140°C.
IR (KBr) 2480, 1730, 1495, 1455, 1315, 1180, 1120, 1040, 775, 705, 545 $cm^{-1}$.

Example 97

3-[Bis(4-fluorophenyl)methyl]-1-[2-(2-methylpropoxy)benzyl]-4-piperidionone

To 10 ml of dimethylformamide were added 1 g of 3-[Bis(4-fluorophenyl)methyl]-1-(2-hydroxybenzyl)-4-piperidinone, 0.9 g of isobutyl iodide and 0.7 g of potassium carbonate and the mixture was stirred in an oil bath at 70°C for 16 hours. The mixture was extracted with 30 ml of ethyl acetate, the extract was washed with water, and then dried over magnesium sulfate, and the solvent was distilled off under reduced pressure. The residue was dissolved in hexane, and crystals precipitated were collected by filtration to obtain 0.9 g of the title compound (yield: 78%).
mp 143-144°C.
IR (KBr) 2960, 1710, 1600, 1505, 1240, 1220, 1160, 1105, 1030, 830, 760, 525 $cm^{-1}$.

Example 98

3-[Bis(4-fluorophenyl)methyl]-1-(2-ethoxybenzyl)-4-piperidinone

According to the same manner as that described in Example 32, the title compound was obtained (yield: 59%).
mp 137-138°C.
IR (KBr) 2975, 2800, 1710, 1600, 1505, 1240, 1220, 1120, 1045, 830, 760, 560, 525 $cm^{-1}$.

Example 99

3-Benzhydryl-1-(2-ethoxybenzyl)-4-piperidinone

According to the same manner as that described in Example 32, the title compound was obtained as an oil.
$^1$H-NMR ($CDCl_3$) δ: 1.33 (t, J=7 Hz), 2.30-2.95 (m, 6H), 3.40 (m, 1H), 3.54 and 3.64 (ABq, 2H), 4.62 (d, 1H, J=11.2 Hz), 6.80-7.35 (m, 14H).

The above oil was dissolved in ethyl ether and a calculated amount of a solution of 4 N HCl/dioxane was added thereto. Crystals precipitated were collected by filtration to obtain the hydrochloride of the title compound (yield: 72%).
mp 172-174°C.
IR (KBr) 2980, 2925, 2490, 1730, 1605, 1500, 1455, 1260, 1130, 1045, 765, 750, 710, 545 $cm^{-1}$.

Example 100

3-[Bis(4-fluorophenyl)methyl]-1-(2-hydroxy-5-nitrobenzyl)-4-piperidinone

To 10 ml of dimethylformamide were added 1.1 g of 3-[bis(4-fluorophenyl)methyl]-4-piperidinone hydrochloride, 0.7 g of 2-chloromethyl-4-nitrophenol and 1.4 g of potassium carbonate and tne mixture was stirred at 70°C for 3 hours. The mixture was extracted with 30 ml of ethyl acetate, the extract was washed with water, and the ethyl acetate was distilled off under reduced pressure. The residue was dissolved in a small amount of ethyl ether and allowed to stand. Crystals precipitated were collected by filtration to obtain 0.64 g of the title compound (yield: 42%).
mp 169-170°C.
IR (KBr) 2840, 1715, 1615, 1600, 1580, 1500, 1475, 1450, 1335, 1280, 1215, 1160, 1085, 820, 745, 525 $cm^{-1}$.

Example 101

2-[[3-[Bis(4-fluorophenyl)methyl]-4-oxopiperidin-1-yl]methyl]benzonitrile

To 10 ml of dimethylformamide were added 1.1 g of 3-[bis(4-fluorophenyl)methyl]-4-piperidinone hydrochloride, 0.7 g of 2-cyanobenzylbromide and 1.4 g of potassium carbonate and the mixture was stirred at room temperature for 16 hours. The mixture was extracted with 30 ml of ethyl acetate, the extract was washed with water, and then dried over magnesium sulfate, and the solvent was distilled off under reduced pressure. The residue was purified by silica gel column chromatography (40 g, ethyl acetate : hexane = 1 : 1). Crystals were triturated with hexane and filtered to obtain 0.92 g of the title compound (yield: 66%).
mp 83-84°C.
IR (KBr) 2900, 2210, 1710, 1700, 1600, 1505, 1440, 1340, 1275, 1220, 1200, 1155, 1030, 1000, 620, 555, 520 $cm^{-1}$.

Example 102

3-[[3-[Bis(4-fluorophenyl)methyl]-4-oxopiperidin-1-yl]methyl]benzonitrile

To 10 ml of dimethylformamide were added 1.1 g of 3-[bis(4-fluorophenyl)methyl]-4-piperidinone hydrochloride, 0.7 g of 3-cyanobenzylbromide and 1.4 g of potassium carbonate and the mixture was stirred at room temperature for 16 hours. The mixture was extracted with 30 ml of ethyl acetate, the extract was washed with water, and then dried over magnesium sulfate, and the solvent was distilled off under reduced pressure. The residue was dissolved in 5 ml of hot ethanol, and then ice-cooled. Crystals precipitated were collected by filtration to obtain 1.18 g of the title compound (yield: 85%).
mp 159-160°C.
IR (KBr) 2950, 2790, 2740, 2225, 1705, 1600, 1505, 1355, 1295, 1220, 1155, 825, 780, 685, 570, 550, 525 $cm^{-1}$.

Example 103

4-[[3-[Bis(4-fluorophenyl)methyl]-4-oxopiperidin-1-yl]methyl]benzonitrile

To 10 ml of dimethylformamide were added 1.1 g of 3-[bis(4-fluorophenyl)methyl]-4-piperidinone hydrochloride, 0.7 g of 4-cyanobenzyl bromide and 1.4 g of potassium carbonate and the mixture was stirred at room temperature for 16 hours. The mixture was extracted with 30 ml of ethyl acetate, the extract was washed with water, and then dried over magnesium sulfate, and the solvent was distilled off under reduced pressure. The residue was purified by silica gel column chromatography (40 g, ethyl acetate : hexane = 1 : 1). Crystals were triturated with hexane and filtered to obtain 1 g of the title compound (yield: 72%).
mp 118-120°C.
IR (KBr) 2800, 2225, 1710, 1600, 1505, 1465, 1410, 1340, 1220, 1155, 1100, 1015, 990, 830, 790, 550 $cm^{-1}$.

Example 104

1-(1,1'-Biphenyl-9-ylmethyl)-3-[bis(4-fluorophenyl)methyl]-4-piperidinone

According to the same manner as that described in Example 102, the title compound was obtained using 4-phenylbenzyl chloride (yield: 73%).
mp 148-149°C.
IR (KBr) 2800, 1705, 1600, 1500, 1340, 1220, 1155, 1100, 825, 770, 735, 695, 545, 520 $cm^{-1}$.

Example 105

3-[Bis(4-fluorophenyl)methyl]-1-(2, 6-dichlorobenzyl)-4-piperidinone

According to the same manner as that described in Example 102, the title compound was obtained using 2,6-dichlorobenzyl bromide (yield: 75%).
mp 137-138°C.
IR (KBr) 2800, 1715, 1600, 1500, 1435, 1215, 825, 765 $cm^{-1}$.

Example 106

3-[Bis(4-fluorophenyl)methyl]-1-(4-hydroxybenzyl)-4-piperidinone

To 10 ml of dichloromethane were added 1.1 g of 3-[bis(4-fluorophenyl)methyl]-4-piperidinone hydrochloride, 1.7 ml of diisopropylethylamine and 0.5 g of p-hydroxybenzyl alcohol were added, and then 1 ml EPPA was added there to at room temperature. After stirring for 16 hours, the solvent was distilled off under reduced pressure. The residue was purified by silica gel column chromatography (40 g ethyl acetate : hexane = 1 : 1) to obtain the title compound as an oil.
$^1$H-NMR ($CDCl_3$) δ: 2.30-2.90 (m, 6H), 3.25 (m, 1H), 3.34 and 3.50 (ABq, 2H, J=13 Hz), 4.58 (d, 1H, J=11 Hz), 6.75-7.27 (m, 12H).
The above oil was dissolved in 50 ml of ethyl ether, and 1 ml of a solution of 4 N HCl/dioxane was added thereto. The crystalline powder was collected by filtration to obtain 1.2 g of the hydrochloride of the title compound (yield: 81%).
mp 145-146°C.
IR (KBr) 3200, 2950, 2550, 1730, 1615, 1510, 1450, 1415, 1270, 1220, 1155, 825, 550, 525 $cm^{-1}$.

Example 107

3-[Bis(4-chlorophenyl)methyl]-1-(4-hydroxybenzyl)-4-piperidinone

According to the same manner as that described in Example 106, the title compound was obtained as an oil using 3-[bis(4-chlorophenyl)methyl]-4-piperidinone hydrochloride.
$^1$H-NMR ($CDCl_3$) δ: 2.30-2.90 (m, 6H), 3.25 (m, 1H), 3.33 and 3.50 (ABq, 2H, J=12.8 Hz), 4.53 (d, 1H, J=11 Hz), 6.75-7.27 (m, 12H).

The above oil was dissolved in ethyl ether, and a calculated amount of a solution of 4 N HCl/dioxane was added thereto. The crystalline powder was collected by filtration to obtain the hydrochloride of the title compound (yield: 69%).
mp 142-143°C.
IR (KBr) 3200, 2550, 1730, 1610, 1590, 1515, 1490, 1450, 1410, 1270, 1230, 1175, 1090, 1010, 840, 820, 790, 540, 500 $cm^{-1}$.

Example 108

3-[Bis(4-fluorophenyl)methyl]-1-(3-phenoxybenzyl)-4-piperidinone

To 10 ml of dichloromethane were added 1.1 g of 3-[bis(4-fluorophenyl)methyl]-4-piperidinone hydrochloride, 1.7 ml of diisopropylethylamine and 0.7 g of 3-phenoxybenzyl alcohol, and then 1 ml of EPPA was added thereto at room temperature. After allowing to stand at room temperature for 4 days, the solvent was distilled off under reduced pressure. The residue was purified by silica gel column chromatography (40 g, ethyl acetate : hexane = 1 : 1 ) to obtain the title compound as an oil.
$^1$H-NMR ($CDCl_3$) δ: 2.25-2.95 (m, 6H), 3.25 (m, 1H), 3.40 and 3.53 (ABq, 2H, J=13.2 Hz), 4.58 (d, 1H, J=11 Hz), 6.83-7.41 (m, 17H).

The above oil was dissolved in 50 ml of ethyl ether, and 2 ml of a solution of 4 N HCl/dioxane was added thereto. The crystalline powder was collected by filtration to obtain 1.1 g of the hydrochloride of the title compound (yield: 63%).
mp 164-165°C.
IR (KBr) 3425, 2950, 2500, 1730, 1600, 1580, 1510, 1485, 1440, 1260, 1220, 1160, 830, 780, 690, 540 $cm^{-1}$.

Example 109

3-Benzhydryl-1-(3-phenoxybenzyl)-4-piperidinone

According to the same manner as that described in Example 108, the title compound was obtained as an oil using 3-benzhydryl-4-piperidinone hydrochloride.
$^1$H-NMR ($CDCl_3$) δ: 2.25-2.95 (m, 6H), 3.35 (m, 1H), 3.41 and 3.50 (ABq, 2H, J=13 Hz), 4.61 (d, 1H, J=11.4 Hz), 6.89-7.41 (m, 19H).

The above oil was dissolved in ethyl ether and a calculated amount of a solution of 4 N HCl/dioxane was added thereto. The crystalline powder was collected by filtration to obtain the hydrochloride of the title compound (yield: 62%).
mp 166-167°C.
IR (KBr) 2500, 1730, 1580, 1490, 1450, 1400, 1255, 1215, 745, 700, 540 $cm^{-1}$.

Example 110

3-[Bis(4-fluorophenyl)methyl]-1-[2, 4-bis(trifluoromethyl)benzyl]-4-piperidinone

To 10 ml of dimethylformamide were added 1.1 g of 3-[bis(4-fluorophenyl)methyl]-4-piperidinone hydrochloride, 1 g of 2,4-bis(trifluoromethyl)benzyl bromide and 1.4 g of potassium carbonate were added and the mixture was stirred at room temperature for 10 hours. The mixture was extracted with 30 ml of ethyl acetate, the extract was washed with water, and then dried over magnesium sulfate, and the solvent was distilled off under reduced pressure. The residue (crystal) was triturated with hexane and filtered to obtain 1 g of the title compound (yield: 57%).
mp 104-105°C.
IR (KBr) 2950, 2875, 1700, 1625, 1600, 1510, 1345, 1275, 1255, 1220, 1170, 1155, 1025, 1085, 1050, 1030, 1000, 905, 840, 820, 780, 765, 560, 525 $cm^{-1}$.

Example 111

3-Benzhydryl-1-(1, 1'-biphenyl-4-ylmethyl)-4-piperidinone

According to the same manner as that described in Example 102, the title compound was obtained from 3-benzhydryl-4-piperidinone hydrochloride and 4-phenylbenzyl chloride (yield: 70%).
mp 157-158°C.
IR (KBr) 3025, 2800, 1710, 1590, 1450, 1340, 1180, 765, 740, 705, 695, 540 $cm^{-1}$.

Example 112

3-[Bis(4-fluorophenyl)methyl]-1-(1, 1'-biphenyl-2-ylmethyl)-4-piperidinone

To 10 ml of dimethylformamide were added 1.1 g of 3-[bis(4-fluorophenyl)methyl]-4-piperidinone hydrochloride, 0.85 g of 2-phenylbenzyl bromide and 1.4 g of potassium carbonate and the mixture was stirred at room temperature for 7 hours. The mixture was extracted with 30 ml of ethyl acetate, the extract was washed with water, and then dried over magnesium sulfate, and the solvent was distilled off under reduced pressure. The residue was purified by silica gel column chromatography (40 g, ethyl acetate : hexane = 1 : 1) to obtain the title compound as an oil.
$^1$H-NMR ($CDCl_3$) δ: 2.20-2.70 (m, 6H), 3.12 (m, 1H), 3.36 and 3.53 (ABq, 2H, J=13.2 Hz), 4.48 (d, 1H, J=11 Hz), 6.78-7.45 (m, 17H).
The above oil was dissolved in 50 ml of ethyl ether and 1 ml of a solution of 4 N Cl/dioxane was added thereto. The crystalline powder was collected by filtration to obtain 1.2 g of the hydrochloride of the title compound (yield: 71%).
mp 144-146°C.
IR (KBr) 2900, 2425, 2325, 1730, 1600, 1500, 1475, 1450, 1415, 1220, 1155, 830, 775, 705, 575, 555, 515 $cm^{-1}$.

Example 113

3-Benzhydryl-1-(1,1'-biphenyl-2-ylmethyl)-4-piperidinone

According to the same manner as that described in Example 112, the title compound was obtained from 3-benzhydryl-4-piperidinone hydrochloride and 2-phenylbenzyl chloride.
$^1$H-NMR ($CDCl_3$) δ: 2.20-2.70 (m, 6H), 3.25 (m, 1H), 3.36 and 3.51 (ABq, 2H, J=13.5 Hz), 4.52 (d, 1H, J=11 Hz), 7.03-7.51 (m, 19H).
The above oil was dissolved in 50 ml of ethyl ether and 1 ml of a solution of 4 N HCl/dioxane was added thereto. The crystalline powder was collected by filtration to obtain 1.2 g of the hydrochloride of the title compound (yield: 77%).
mp 146-148°C.
IR (KBr) 3050, 2275, 1730, 1595, 1490, 1480, 1450, 1410, 1385, 780, 760, 745, 700, 540 $cm^{-1}$.

Example 114

3-[Bis(4-fluorophenyl)methyl]-1-(2-nitrobenzyl)-4-piperidinone

To 10 ml of dimethylformamide were added 1.1 g of 3-[bis(4-fluorophenyl)methyl]-4-piperidinone hydrochloride, 0.8 g of 2-nitrobenzyl bromide and 1.4 g of potassium carbonate and the mixture was stirred at room temperature for 7 hours. The mixture was extracted with 30 ml of ethyl acetate and the extract was washed with water, and the ethyl acetate was distilled off under reduced pressure. The residue was dissolved in a small amount of ethanol and allowed to stand. Crystals precipitated were collected by filtration to obtain 1.2 g of the title compound (yield: 82%).
mp 151-152°C.
IR (KBr) 2800, 1705, 1600, 1530, 1500, 1465, 1360, 1215, 1185, 1155, 820, 770, 720, 540 $cm^{-1}$.

Example 115

3-[Bis(4-fluorophenyl)methyl]-1-(3-nitrobenzyl)-4-piperidinone

According to the same manner as that described in Example 114, the title compound was obtained from 3-[bis (4-fluorophenyl)methyl]-4-piperidinone hydrochloride and 3-nitrobenzyl bromide (yield: 89%).
mp 131-133°C.

IR (KBr) 2800, 1705, 1595, 1525, 1500, 1340, 1220, 1155, 820, 730, 690, 540 $cm^{-1}$.

Example 116

3-[Bis(4-fluorophenyl)methyl]-1-(4-nitrobenzyl)-4-piperidinone

To 10 mi of dimethylformamide were added 1.1 g of 3-[bis(4-fluorophenyl)methyl]-4-piperidinone hydrochloride, 0.8 g of 4-nitrobenzyl bromide and 1.4 g of potassium carbonate, and the mixture was stirred at room temperature for 1 hour. The mixture was extracted with 30 ml of ethyl acetate, the extract was washed with water, and then dried over magnesium sulfate, and the solvent was distilled off under reduced pressure. The residue was purified by subjecting it to silica gel column chromatography (40 g, ethyl acetate). After addition of ethanol and allowing to stand, crystals precipitated were collected by filtration to obtain 1 g of the title compound (yield: 69%).
mp 118-119°C.
IR (KBr) 2800, 1715, 1600, 1520, 1500, 1340, 1215, 1155, 1100, 840, 730, 550, 545 $cm^{-1}$.

Example 117

3-[Bis(4-methylphenyl)methyl)-1-(2-methoxybenzyl)-4-piperidinone

To a solution of 2.18 g of 1-(2-methoxybenzyl)-4-piperidinone in 20 ml of dichloromethane were added 4 ml of TMSOTf and then 2.12 g of 4,4'-dimethylbenzhydrol under ice-cooling. After stirring at room temperature for 4 hours, 20 ml of water was added thereto and the mixture was neutralized with sodium bicarbonate. The dichloromethane layer was separated, and the solvent was distilled off under reduced pressure. The residue was purified by silica gel column chromatography (40 g, ethyl acetate : hexane = 1 : 4) to obtain 1.4 g of the title compound as an oil.
$^1$H-NMR ($CDCl_3$) δ: 2.24 (s, 6H), 2.25-2.95 (m, 6H), 3.32 (m, 1H), 3.56 (s, 2H), 3.77 (s, 3H), 4.56 (d, 1H, J=11.4 Hz), 6.83-7.33 (m, 11H).

The above oil was dissolved in 50 ml of ethyl ether, and 1.25 ml of a solution of 4 N HCl/dioxane was added thereto. The crystalline powder was collected by filtration to obtain 1.35 g of the hydrochloride of the title compound (yield: 30%).
mp 161-164°C.
IR (KBr) 3400, 2900, 2500, 172.5, 1600, 1510, 1495, 1460, 1250, 750, 550 $cm^{-1}$.

Example 118

3-[Bis(4-fluorophenyl)methyl]-1-(2, 6-dimethoxybenzyl)-4-piperidinone

To 15 ml of dichloromethane were added 1.1 g of 3-[bis(4-fluorophenyl)methyl]-4-piperidinone hydrochloride, 1.7 ml of isopropyl ethylamine and 0.6 g of 2, 6-dimethoxybenzyl alcohol and the mixture was stirred at room temperature. Then, 0.8 ml of EPPA was added thereto and the mixture was allowed to stand at room temperature for 4 days. The solvent was distilled off under reduced pressure, and the residue was purified by subjecting it to silica gel column chromatography (40 g, ethyl acetate : hexane = 1 : 1) to obtain 0.88 g of the title compound (yield: 60%).
mp 125-126°C.
IR (KBr). 1705, 1595, 1505, 1470, 1250, 1215, 1110, 830, 555, 525 $cm^{-1}$.

Example 119

3-[Bis(4-fluorophenyl)methyl]-1-(2-difluoromethoxybenzyl)-4-piperidinone

To 15 ml of dichloromethane were added 1.1 g of 3-[bis(4-fluorophenyl)methyl]-4-piperidinone hydrochloride, 1.7 ml of isopropyl ethylamine and 0.6 g of 2-difluoromethoxybenzyl alcohol and the mixture was stirred at room temperature. Then, 0.8 ml of EPPA was added thereto and the mixture was allowed to stand at room temperature for 5 days. After the solvent was distilled off under reduced pressure, the residue was purified by subjecting it to silica gel column chromatography (40 g, ethyl acetate : hexane = 1 : 1) to obtain 0.84 g of the title compound (yield: 55%).
mp 83-84°C.
IR (KBr) 2950, 2800, 1710, 1600, 1500, 1380, 1220, 1120, 1055, 830, 760, 525 $cm^{-1}$.

Example 120

3-[Bis(4-fluorophenyl)methyl]-1-(2-trifluoromethoxybenzyl)-4-piperidinone

To 15 ml of dichloromethane were added 1.1 g of 3-[bis(4-fluorophenyl)methyl]-4-piperidinone hydrochloride, 1.7 ml of isopropyl ethylamine and 0.7 g of 2-trofluoromethoxybenzyl alcohol and the mixture was stirred at room temperature. Then, 0.81 ml of EPPA was added thereto and the mixture was allowed to stand at room temperature for 5 days. After the solvent was distilled off under reduced pressure, the residue was purified by silica gel column chromatography (40 g, ethyl acetate : hexane = 1 : 1) to obtain 0.73 g of the title compound (yield: 46%).
mp 88-89°C.
IR (KBr) 1710, 1600, 1505, 125.0, 1215, 1170, 830, 760, 560, 525 $cm^{-1}$.

Example 121

3-Benzhydryl-1-(4-methylthiobenzyl)-4-piperidinone

To 15 ml of dichloromethane were added 1.1 g of 3-benzhydryl-4-piperidinone hydrochloride, 1.7 ml of isopropyl ethylamine and 0.7 g of 4-methylthiobenzyl alcohol and the mixture was stirred at room temperature. Then, 0.8 ml of EPPA was added and the mixture was allowed to stand at room temperature for 5 days. The solvent was distilled off under reduced pressure, and the residue was purified by subjecting it to silica gel column chromatography (40 g, ethyl acetate : hexane = 1 : 1) to obtain 0.94 g of the title compound (yield: 70%).
mp 126-128°C.
IR (KBr) 2800, 1710, 1595, 1490, 1340, 1180, 780, 740, 700, 690, 540 $cm^{-1}$.

Example 122

Methyl 4-[(3-benzhydryl-4-oxopiperidin-1-yl)methyl]benzoate

To 50 ml of dimethylformamide were added 5.25 g of 3-benzhydryl-4-piperidinone hydrochloride, 4.2 g of methyl 4-(bromomethyl)benzoate and 2.45 g of potassium carbonate and the mixture was stirred at room temperature for 7 hours. The mixture was extracted with 30 ml of ethyl acetate and the extract was washed with water, and the ethyl acetate was distilled under reduced pressure. The residue was triturated with hexane and filtered to obtain 6.13 g of the title compound (yield: 85%).
mp 107-108°C.
IR (KBr) 2800, 1710, 1610, 1490, 1430, 1280, 1105, 760, 740, 700, 540 $cm^{-1}$.

Example 123

4-[(3-Benzhydryl-4-oxopiperidin-1-yl)methyl]benzoic acid

To a solution of 4.13 g of 4-[(3-benzhydryl-4-oxopiperidin-1-yl)methyl]benzoic acid methyl ester in 30 ml of methanol was added 20 ml of 1 N aqueous solution of sodium hydroxide, and the mixture was stirred in an oil at 60°C bath for 3 hours. After cooling, 120 ml of 1 N hydrochloric acid was added thereto and a precipitate was collected by filtration. After dissolving in $CH_2Cl_2$/MeOH, the solution was purified by subjecting it to silica gel column chromatography (150 g, ethyl acetate : hexane = 1 : 1) to obtain 1.8 g of the title compound as foam (yield: 44%).
$^1$H-NMR ($CDCl_3$) $\delta$: 2.30-2.95 (m, 6H), 3.42 (m, 1H), 3.50 and 3.64 (ABq, 2H, J=13.5 Hz), 3.93 (s, 3H), 4.62 (d, 1H, J=11.2 Hz), 7.05-8.08 (m, 14H).

To a solution of 0.81 g of the foam residue in 30 ml of ethyl ether was added 1 ml of a solution of 4 N HCl/dioxane. The precipitate was collected by filtration to obtain 0.81 g of the hydrochloride of the title compound. mp 179-181°C.
IR (KBr) 3025, 2550, 1720, 1490, 1450, 1415, 1370, 1205, 1180, 1105, 750, 700, 540 $cm^{-1}$.

Example 124

1-Acetyl-3-[bis(4-methylphenyl)methyl]-4-piperidinone

According to the same manner as that described in Example 7, the title compound was obtained (yield: 22%).
mp 142-144°C.

IR (KBr) 2925, 1720, 1650, 1510, 1425, 1300, 1240, 1050, 985, 800, 760, 540 $cm^{-1}$.

Example 125

3-[Bis(4-methylphenyl)methyl]-4-piperidinone

To 1-acetyl-3-[bis(4-methylphenyl)methyl]-4-piperidinone were added 30 ml of water and 30 ml of conc. hydrochloric acid and the mixture was stirred in an oil bath at 130°C for 8 hours, and further at 90°C for additional 16 hours. The solvent was distilled off under reduced pressure, and the residue was triturated with ethanol and filtered. Washing with ethyl ether gave 3.6 g of the hydrochloride of the title compound (yield: 79%).
mp 209-210°C.
IR (KBr) 3050, 2700, 1725, 1510, 1425, 1130, 810, 755, 555 $cm^{-1}$.

Example 126

2-Oxo-2-phenylethyl [4-[(3-Benzhydryl-4-oxopiperidin-1-yl)methyl]phenyl]acetate

To 40 ml of dimethylformamide were added 4.3 g of 3-benzhydryl-4-piperidinone hydrochloride, 5 g of 2-oxo-2-phenylethyl [4-(bromomethyl)phenyl]acetate and 2 g of potassium carbonate and the mixture was stirred at room temperature for 2 hours. The mixture was extracted with 30 ml of ethyl acetate and the extract was washed with water, and the ethyl acetate was distilled off under reduced pressure. The residue was purified by subjecting it to silica gel column chromatography (150 g, ethyl acetate : hexane = 1 : 1) to obtain 7.1 g of the title compound as an oil (yield: 88%).
$^1$H-NMR ($CDCl_3$) δ: 2.30-2.90 (m, 6H), 3.36 (m, 1H), 3.47 and 3.59 (ABq, 2H, J=13.6 Hz), 3.93 (s, 3H), 4.62 (d, 1H, J=11 Hz), 7.05-8.00 (m, 14H).

To a solution of 1 g of the oily title compound in 50 ml of ethyl ether was added 1 ml of a solution of 4 N HCl/dioxane. The precipitate was collected by filtration to obtain 0.81 g of the hydrochloride of the title compound. mp 99-110°C.
IR (KBr) 3400, 1730, 1700, 1595, 1450, 1220, 1150, 970, 745, 700, 540 $cm^{-1}$.

Example 127

[4-[(3-Benzhydryl-4-oxopiperidin-1-yl)methyl]phenyl]acetic acid

To a solution of 5.9 g of 2-oxo-2-phenylethyl [4-[(3-benzhydryl-4-oxopiperidin-1-yl)methyl]phenyl]acetate in 20 ml of tetrahydrofuran was added 15 ml of 1 N aqueous solution of sodium hydroxide under ice-cooling in ice. After stirring for 60 minutes, 15 ml of 1 N aqueous solution of sodium hydroxide was further added thereto. After allowing to stand at room temperature for 16 hours, 30 ml of 1 N hydrochloric acid was added with stirring, and tetrahydrofuran was distilled off under reduced pressure. The gummy water-insoluble residue was dissolved in $CH_2Cl_2$/MeOH, and then the solution was subjected to silica gel column chromatography (150 g, ethyl acetate : hexane = 1 : 1). The desired fraction was concentrated and ethyl ether was added thereto. The mixture was filtered and 2 ml of a solution of 4 N HCl/dioxane was added to the filtrate. The precipitate was collected by filtration to obtain 2.67 g of a hydrochloride of the title compound (yield: 57%).
mp 176-178°C.
IR (KBr) 2950, 2560, 1760, 1730, 1495, 1450, 1350, 1170, 780, 745, 700, 540 $cm^{-1}$.

Example 128

4-[(3-Benzhydryl-4-oxopiperidin-1-yl)methyl]benzamide

To 10 ml of dimethylformamide were added 1 g of 3-benzhydryl-4-piperidinone hydrochloride, 0.6 g of 4-(bromomethyl)benzamide and 1.4 g of potassium carbonate and the mixture was stirred at room temperature for 3 hours, and then at 100°C for 20 minutes. The mixture was extracted with 30 ml of ethyl acetate and the extract was washed with water and ethyl acetate was distilled off under reduced pressure. The residue was purified by subjecting it to silica gel column chromatography (40 g, ethyl acetate) to obtain 1 g of the title compound as an oil.
$^1$H-NMR ($CDCl_3$) δ: 2.30-2.95 (m, 6H), 3.30-3.70 (m, 3H), 4.61 (m, 1H), 7.00-7.78 (m, 14H).

To a solution of 1 g of the oily title compound in 10 ml of ethanol was added 1 ml of a solution of 4 N HCl/dioxane, and the solvent was distilled off under reduced pressure. To the residue was added ethyl ether and the mixture was stirred. The precipitate was collected by filtration to obtain 1 g of the hydrochloride of the title compound (yield:

69%).
mp 133-134°C (decomposition)
IR (KBr) 1730, 1660, 1615, 1565, 1490, 1450, 1420, 1380, 740, 700, 540 $cm^{-1}$.

Example 129

3-[Bis(4-methylphenyl)methyl]-1-(4-hydroxybenzyl)-4-piperidinone

To 15 ml of dichloromethane were added 1.1 g of 3-[bis(4-methylphenyl)methyl]-4-piperidinone hydrochloride, 1.7 ml of isopropylethylamine and 0.7 g of 4-hydroxybenzyl alcohol, the mixture was stirred at room temperature and 1 ml of EPPA was added thereto. After stirring at room temperature for 15minute, the solvent was distilled off under reduced pressure. The residue was purified by subjecting it to silica gel column chromatography (40 g, ethyl acetate : hexane = 1 : 1) and recrystallized from ethyl ether to obtain 0.73 g of the title compound (yield: 46%).
mp 156-157°C.
IR (KBr) 3300, 2900, 1690, 1610, 1590, 1510, 1340, 1255, 1230, 1180, 1100, 800, 775, 550, 540 $cm^{-1}$.

Example 130

4-Benzhydryl-1-(4-hydroxybenzyl)-3-piperidinone

To 15 ml of dichloromethane were added 1,1 g of 4-benzhydryl-3-piperidinone hydrochloride, 1.7 ml of isopropylethylamine and 0.5 g of 4-hydroxybenzyl alcohol. After addtion of 1 ml of EPPA, the mixture was stirred at room temperature for 15 minutes, the solvent was distilled off under reduced pressure and the residue was purified by subjecting it to silica gel column chromatography (40 g, ethyl acetate : hexane = 1 : 1) to obtain the title compound as an oil.
$^1$H-NMR ($CDCl_3$) δ: 1.60-1.90 (m, 2H), 2.50, 2.85 (m, 2H), 2.89 and 3.13 (ABq, 2H, J=13 Hz), 3.30 (m, 1H), 3.51 (s, 2H), 4.39 (d, 1H, J=9.8 Hz), 6.73-7.28 (m, 14H).

The above oil was dissolved in ethyl acetate and 1 ml of a solution of 4N HCl/dioxane was added thereto. The precipitate was collected by filtration to obtain 1.2 g of the hydrochloride of the title compound (yield: 88%).
mp 187-188°C.
IR (KBr) 3150, 2550, 1730, 1610, 1590, 1515, 1490, 1450, 1370, 1270, 1230, 1210, 1170, 740, 700 $cm^{-1}$.

Example 131

3-(5H-Dibenzo[a,d]cyclohepten-5-yl)-4-piperidinone

To a suspension of 6.3 g of 3-(5H-dibenzo[a, d]cyclohepten-5-yl)-1-benzyl-4-piperidinone in 100 ml of methanol was added 1.5 ml of cone. hydrochloric acid and the mixture was stirred. Then, after addition of 3 g of 10% Pd-C, the mixture was stirred for 6 hours under a hydrogen atmosphere. After filtration, the filtrate was distilled under reduced pressure, and then the residue was dissolved in a small amount of ethanol. Crystals precipitated were collected by filtration to obtain 4.5 g of the hydrochloride of the title compound as a crystal (yield: 83%).
mp 182-183°C.
IR (KBr) 2900, 2430, 2375, 1720, 1490, 1450, 1445, 1400, 1185, 805, 770, 755, 695 $cm^{-1}$.

Example 132

3-(5H-Dibenzo[a,d]cyclohepten-5-yl)-1-(4-hydroxybenzyl)-4-piperidinone

To 15 ml of dichloromethane were added 1.1 g of 3-(5H-dibenzo[a, d]cyclohepten-5-yl)-4-piperidinone hydrochloride, 1.7 ml of isopropylethylamine and 0.5 g of 4-hydroxybenzyl alcohol and the mixture was stirred at room temperature. Then, 1 ml of EPPA was added thereto and, after stirring at room temperature for 30 minutes, the solvent was distilled off under reduced pressure. The residue was purified by subjecting it to silica gel column chromatography (40 g, ethyl acetate : hexane = 1 : 1) and recrystallized from ethyl ether to obtain 0.56 g of the title compound (yield: 42%).
mp 156-157°C.
IR (KBr) 3020, 2820, 2790, 1700, 1490, 1450, 1335, 1235, 1180, 1110, 805, 775, 760, 730, 695, 550, 460 $cm^{-1}$.

Example 133

3-(9H-Xanten-9-yl)-4-piperidinone

To a suspension of 6 g of 1-benzyl-3-(9H-xanten-9-yl)-4-piperidinone in 100 ml of methanol was added 1.5 ml of conc. hydrochloride and the mixture was stirred. After addition of 3 g of 10% palladium carbon, the mixture was stirred under a hydrogen atmosphere for 6 hours. The mixture was filtered, the filtrate was distilled off under reduced pressure, and then the residue was dissolved in a small amount of ethanol. Crystals precipitated were collected by filtration to obtain 3.2 g of the hydrochloride of the title compound as crystals (yield: 63%).
mp 202-203°C.
IR (KBr) 2900, 2800, 2700, 1705, 1595, 1570, 1475, 1450, 1250, 900, 755, 470, 455 $cm^{-1}$.

Example 134

1-(4-Hydroxybenzyl)-3-(9H-xanten-9-yl)-4-piperidinone

To 15 ml of dichloromethane were added 1.1 g of 3-(9H-xanten-9-yl)-4-piperidinone hydrochloride, 1.7 ml of isopropylethylamine and 0.5 g of 4-hydroxybenzyl alcohol and the mixture was stirred at room temperature. Then, 1 ml of EPPA was added thereto, the mixture was stirring at room temperature for 30 minutes, and the solvent was distilled off under reduced pressure. The residue was purified by subjecting it to silica gel column chromatography (40 g, ethyl acetate : hexane = 1 : 1) and recrystallized from ethyl ether to obtain 1 g of the title compound (yield: 75%).
mp 171-172°C.
IR (KBr) 3350, 2810, 2760, 1700, 1615, 1590, 1515, 1450, 1340, 1240, 1210, 1185, 1100, 1090, 760 $cm^{-1}$.

Example 135

3-[Bis(4-fluorophenyl)methyl]-1-(2-methoxy-5-nitrobenzyl)-4-piperidinone

To a solution of 3 g of 3-[bis(4-fluorophenyl)methyl]-4-piperidinone hydrochloride and 2.46 g of 2-methoxy-5-nitrobenzyl bromide in 20 ml of dimethylformamide was added 4.2 mg of potassium carbonate with stirring at room temperature. After stirring for 3 hours, 50 ml of ethyl acetate and 50 ml of brine were added thereto. The ethyl acetate layer was separated and washed with water, and the solvent was distilled off under reduced pressure. The residue was purified by subjecting it to silica gel column chromatography (11 g, ethyl acetate : hexane = 1 : 1) to obtain the title compound as an oil.
$^1$H-NMR ($CDCl_3$) δ: 2.35-2.90 (m, 6H), 3.57 (s, 2H), 3.88 (s, 3H), 4.55 (d, 1H, J=11 Hz), 6.80-7.28, 8.15-8.35 (m, 11H).
The above oil was dissolved in 30 ml of ethyl ether and 3 ml of a solution of 4 N HCl/dioxane was added thereto with stirring. The precipitate was collected by filtration to obtain 4.5 g of the hydrochloride of the title compound (yield: quantitative).
mp 166-167°C.
IR (KBr) 3425, 2950, 1730, 1590, 1505, 1340, 1275, 1220, 1155, 1095, 1015, 830, 750, 635, 550 $cm^{-1}$.

Example 136

1-(5-Amino-2-methoxybenzyl)-3-[bis(4-fluorophenyl)methyl]-4-piperidinone

To a mixture of 80 ml of methanol and 10 ml of water were added 2.8 g of 3-[bis(4-fluorophenyl)methyl]-1-(2-methoxy-5-nitrobenzyl)-4-piperidinone and 1.8 g of 5% Pd-C. The mixture was stirred at room temperature for 2 hours under a hydrogen atmosphere. After confirming the absorption of a calculated amount of hydrogen, the reaction mixture was filtered, 1 ml of a solution of 4 N HCl/dioxane was added to the filtrate, and the solvent was distilled off under reduced pressure. To the residue was added 30 ml of ethanol, and the ethanol was distilled off. The residue was dissolved in ethyl acetate to precipitate crystals. The crystals were collected by filtration to obtain 1.4 g of the dihydrochloride of the title compound (yield: 50%).
mp 200-203°C.
IR (KBr) 2800, 2550, 1730, 1600, 1505, 1450, 1270, 1220, 1160, 840, 555 $cm^{-1}$.
The above hydrochloride was added to a mixture of chloroform and water, and the mixture was neutralized with sodium bicarbonate. The solvent was distilled off under reduced pressure to obtain the title compound.
$^1$H-NMR ($CDCl_3$) δ: 2.35-2.95 (m, 6H), 3.29 (m, 1H), 3.49 (s, 2H), 3.70 (s, 3H), 4.58 (d, 1H, J=11 Hz), 6.55-7.27 (m, 11H).

Example 137

4-Benzhydryl-1-benzyl-3-pyrrolidinone

To a solution of 1.75 g of 1-benzyl-3-pyrrolidinone in 15 ml of dichloromethane were added 4 ml of TMSOTf and then 1.84 g of benzhydrol with stirring under ice-cooling. After stirring at room temperature for 16 hours, ice water was added to the mixture and the mixture was neutralized with sodium bicarbonate. The dichloromethane layer was separated, and the solvent was distilled off under reduced pressure. The residue was purified by subjecting it to silica gel column chromatography (40 g, ethyl acetate : hexane = 1 : 4) to obtain the title compound as an oil.
$^1$H-NMR ($CDCl_3$) δ: 2.47-2.68, 3.15-3.50 (each m, 5H), 3.49 and 3.70 (ABq, 2H, J=13 Hz), 4.50 (d, 1H, J=7 Hz), 7.00-7.36 (m, 15H).
The above oil was dissolved in ethyl ether, and 1 ml of a solution of 4 N HCl/dioxane was added thereto with stirring. The precipitate was collected by filtration to obtain 0.84 g of the hydrochloride of the title compound as a powder (yield: 22%).
IR (KBr) 3400, 3025, 2350, 1760, 1595, 1490, 1450, 1170, 1025, 745, 695 $cm^{-1}$.

Example 138

1-Benzyl-4-[bis(4-fluorophenyl)methyl]-3-pyrrolidinone

According to the same manner as that described in Example 137, the title compound was obtained as an oil.
$^1$H-NMR ($CDCl_3$) δ: 2.43-2.69, 3.15-3.32 (each m, 5H), 3.50 and 3.70 (ABq, 2H, J=13 Hz), 4.45 (d, 1H, J=6.9 Hz), 6.89-7.35 (m, 13H).
The title compound was dissolved in ethyl ether and a calculated amount of a solution of 4 N HCl/dioxane was added thereto, and then the solvent was distilled off under reduced pressure. Hexane was added to the residue, and the resultant powder was collected by filtration to obtain 1 g of the hydrochloride of the title compound (yield: 24%).
IR (KBr) 3400, 2925, 2360, 1760, 1600, 1505, 1455, 1415, 1225, 1155, 1010, 825, 750, 700, 565, 545, 520 $cm^{-1}$.

Example 139

4-[Bis(4-fluorophenyl)methyl]-3-pyrrolidinone

To 150 ml of methanol were added 7.5 g of 1-benzyl-4-[bis(4-fluorophenyl)methyl]-3-pyrrolidinone, 3 g of 10% Pd-C and 2 ml of conc. hydrochloric acid. After stirring for 5 hours under a hydrogen atmosphere, the reaction mixture was filtered and the filtrate was distilled off under reduced pressure. After dissolving in a small amount of ethanol, crystals precipitated were collected by filtration to obtain 4 g of the hydrochloride of the title compound (yield: 63%).
mp 180-181°C.
IR (KBr) 2875, 2730, 1760, 1600, 1510, 1415, 1230, 1015, 875, 835, 610, 575, 545 $cm^{-1}$.
To the hydrochloride of the title compound were added chloroform and water and the mixture was neutralized with sodium bicarbonate. After separation of the chloroform layer, the solvent was distilled off to obtain the title compound.
$^1$H-NMR ($CDCl_3$) δ: 2.95-3.60 (each m, 5H), 4.49 (d, 1H, J=5.6 Hz), 6.90-7.27 (m, 8H).

Example 140

4-[Bis(4-fluoropheny)methyl]-1-(4-hydroxybenzyl)-3-pyrrolidinone

To 15 ml of dichloromethane were added 1 g of 4-[bis(4-fluorophenyl)methyl]-3-pyrrolidinone hydrochloride, 1.7 ml of isopropylethylamine and 0.5 g of 4-hydroxybenzyl alcohol. Then, 1 ml of EPPA was added thereto, the mixture was stirred at room temperature for 15 minutes, the solvent was distilled off under reduced pressure. The residue was purified by subjecting it to silica gel column chromatography (40 g, ethyl acetate : hexane = 1 : 1) to obtain the title compound was obtained as an oil.
$^1$H-NMR ($CDCl_3$) δ: 2.35-2.70, 3.14-3.35 (each m, 5H), 3.44 and 3.62 (ABq, 2H, J=12.8 Hz), 4.44 (d, 1H, J=6.6 Hz), 6.75-7.30 (m, 12H).
The above oil was dissolved in 70 ml of ethyl ether and 2 ml of a solution of 4 N HCl/dioxane was added thereto. The precipitate was collected by filtration to obtain 1.2 g of the hydrochloride of the title compound (yield: 84%).
mp 126-127°C.
IR (KBr) 3200, 2950, 2550, 1770, 1610, 1505, 1445, 1415, 1270, 1230, 1175, 1160, 1010, 835, 560, 550, 520 $cm^{-1}$.

Example 141

4-[Bis(4-fluorophenyl)methyl]-1-(2-methoxybenzyl)-3-pyrrolidinone

To 15 ml of dichloromethane were added 0.8 g of 4-[bis(4-fluorophenyl)methyl]-3-pyrrolidinone hydrochloride, 1.7 ml of isopropylethylamine and 0.5 g of 2-methoxybenzyl alcohol. Then, 1 ml of EPPA was added thereto, the mixture was allowed to stand at room temperature for 48 hours, and the solvent was distilled off under reduced pressure. The residue was purified by subjecting it to silica gel column chromatography (40 g, ethyl acetate : hexane = 1 : 2) to obtain the title compound as an oil.
$^1$H-NMR ($CDCl_3$) δ: 2.40-2.74, 3.20-3.35 (each m, 5H), 3.60 and 3.71 (ABq, 2H, J=13 Hz), 3.79 (s, 3H), 4.42 (d, 1H, J=6.9 Hz), 6.84-7.29 (m, 12H).
The above oil was dissolved in 50 ml of ethyl ether, 1 ml of a solution of 4 N HCl/dioxane was added thereto, and the solvent was distilled off under reduced pressure. Hexane was added and the precipitate was collected by filtration to obtain 1.2 g of the hydrochloride of the title compound (yield: 84%).
IR (KBr) 3400, 2950, 2500, 1760, 1660, 1510, 1460, 1250, 1220, 1155, 1120, 1020, 830, 755, 565 $cm^{-1}$.

Example 142

3-(Diphenylmethylene)-1-methyl-4-piperidinone

To a solution of 1.1 g of 1-methyl-4-piperidinone in 10 ml of dichloromethane were added 2 ml of TMSOTf, 2.4 g of dichlorodiphenylmethane and then 2 g of zinc bromide with stirring under ice-cooling. After stirring at room temperature for 16 hours, ice water was added thereto and the mixture was neutralized with sodium bicarbonate.
The dichloromethane layer was dried over anhydrous magnesium sulfate, and then the solvent was distilled off under reduced pressure. The residue was purified by subjecting it to silica gel column chromatography (100 g, ethyl acetate). The solvent was distilled off, and the residue (crystal) was triturated with a small amount of ethyl ether and filtered to obtain 0.38 g of the title compound (yield: 14%).
mp 129-131°C.
IR (KBr) 3660, 2950, 2840, 2790, 2760, 1690, 1605, 1590, 1490, 1440, 1260, 1230, 1170, 1035, 930, 770, 700, 630, 545, 420 $cm^{-1}$.

Example 143

1-Benzyl-3-(diphenylmethylene)-4-piperidinone

To a solution of 1.9 g of 1-benzyl-4-piperidinone in 10 ml of dichloromethane were added 4 ml of TMSOTf, 2.4 g of dichlorodiphenylmethane and then 1 g of zinc bromide with stirring under ice-cooling. After stirring at room temperature for 16 hours, ice water was added thereto and the mixture was neutralized with sodium bicarbonate. The dichloromethane layer was dried over anhydrous magnesium sulfate, and then the solvent was distilled off under reduced pressure. The residue was purified by subjecting it to silica gel column chromatography (100 g, ethyl acetate : hexane = 1 : 1). The solvent was distilled off, and the residue (crystal) was triturated with a small amount of ethyl ether and filtered to obtain 0.35 g of the title compound (yield: 6.6%).
mp 135-137°C.
IR (KBr) 3030, 2810, 2750, 1740, 1695, 1600, 1495, 1450, 1445, 1315, 1250, 1200, 1125, 780, 705, 550 $cm^{-1}$.

Example 144

3-Benzhydryl-1-[3, 5-bis(trifluoromethyl)benzoyl]-4-piperidinone

To a solution of 0.30 g of 3-benzhydryl-4-piperidinone hydrochloride, 0.31 g of 3, 5-bis(trifluoromethyl)benzoic acid, 0.20 g of 1-hydroxy-1H-benzotriazole-monohydrate and 0.11 g of triethylamine in 5 ml of dimethylformamide was added 0.38 g of 1-ethyl-3-(3-dimethylaminopropyl)-carbodiimide hydrochloride, and the mixture was stirred at room temperature for 14 hours. After extracted with 50 ml of ethyl acetate, the extract was washed with an aqueous solution of sodium hydrogen carbonate and then water, and dried over magnesium sulfate. The solvent was distilled off under reduced pressure. The residue was purified by subjecting it to silica gel chromatography (6 g, ethyl acetate : hexane = 1 : 4). The residue (amorphous) was collected by filtration with hexane to obtain 0.47 g of the title compound (yield: 92%).
$^1$H-NMR ($CDCl_3$) δ: 2.40-2.70 (2H, m), 3.20-3.80 (4H, m), 4.10-4.60 (2H, m), 6.80-8.00 (13H, m).

Example 145

3-[Bis(4-chlorophenyl)methyl]-1-[3, 5-bis(trifluoromethyl)benzoyl]-4-piperidinone

According to the same manner as that described in Example 144, 0.37 g of the title compound was obtained (yield: 64%).
$^1$H-NMR ($CDCl_3$) δ: 2.40-2.80 (2H, m), 3.00-3.35 (2H, m), 3.35-3.60 (2H, m), 3.80-4.80 (2H, m), 6.60-7.40 (8H, m), 7.70-8.05 (3H, m).

Example 146

3-[Bis(4-fluorophenyl)methyl]-1-ethoxycarbonyl-4-piperidinone

A 200 ml-volume three-necked flask was charged with 5.0 g (29.2 mmol) of 1-ethoxycarbonyl-4-piperidinone and 20 ml of dichloromethane, and a solution of 11.6 ml (64.3 mmol) of trimethyl silyltriflate (TMSOTf) in 10 ml of dichloromethane was added dropwise thereto with stirring under ice-cooling at 5°C or below. Then, to the mixture was added 5.2 g (23.4 mmol) of 4, 4'-difluorobenzhydrol with stirring at the same temperature. The cooling bath was removed, the mixture was allowed to stand at room temperature overnight, and then 30 ml of water was added thereto with stirring under ice-cooling. The organic phase was separated. The aqueous layer was extracted with 30 ml of dichloromethane and combined with the above organic layer, followed by washing with 30 ml of water. The combined organic layer was concentrated under reduced pressure, 30 ml of ether was added to the residue, and the mixture was stirred under ice-cooling. The precipitated crystals were collected by filtration, washed with ether and dried under reduced pressure to obtain 6.4 g of the title compound as crystals (yield: 73%).
Elemental analysis: $C_{21}H_{21}F_2NO_3$:
Calculated: C, 67.55; H, 5.67; N, 3.75.
Found: C, 67.46; H, 5.61; N, 3.77.
$^1$H-NMR ($CDCl_3$) δ: 1.19-1.25 (m, 2H), 3.32 (m, 2H), 3.50-4.00 (m, 3H), 4.16-4.20 (m, 2H), 4.34 (d, 1H, J=10.4Hz), 4.35 (s, 1H), 6.93-7.01 (m, 4H), 7.18-7.25 (m, 4H).

Example 147

3-[(2-Fluorophenyl)(phenyl)methyl]-4-piperidinone

To 2.0 g (4.88 mmol) of 1-benzyl-3-[(2-fluorophenyl)(phenyl)methyl]-4-piperidinone hydrochloride were added 50 ml of methanol and 5 ml of water and the mixture was dissolved with warming. After purged with nitrogen, 0.5 g of 10%Pd-C (wet) was added thereto and the mixture was cooled to room temperature. Then, in a hydrogen stream, the mixture was stirred at atmospheric pressure for 7 hours. The mixture was filtered through Celite, washed with 5 ml of methanol, and the filtrate was concentrated. The mixture was allowed to stand in a refrigerator and then precipitated crystals were collected by filtration, dried to obtain 1.24 g of the hydrochloride of the title compound (yield: 79%).
mp 204-205°C.
$^1$H-NMR ($CDCl_3$) δ: 2.61-3.70 (m, 6H), 6.28 (m, 1H), 4.72 (d, 0.5H, J=11.0Hz), 4.78 (d, 0.5H, J=10.4Hz), 6.95-7.37 (m, 9H).
IR (KBr): 3441, 2900, 2778, 2699, 2637, 2511, 2465, 1720, 1589, 1490, 1454, 1382, 1226, 757, 742, 699, 519 $cm^{-1}$.
Elemental analysis: $C_{18}H_{18}FNO{\bullet}HCl$:
Calculated: C, 67.60; H, 5.99.
Found: C, 66.99; H, 5.86.

Example 148

3-[Bis(4-fluorophenyl)methyl]-1-[(2-methoxy-1-naphthyl)methyl]-4-piperidinone

A 25 ml round-bottomed flask was charged with 1.0 g (2.96 mmol) of 3-[bis(4-fluorophenyl)methyl]-4-piperidinone hydrochloride, 613 mg (3.26 mmol) of 2-methoxy-1-naphthalenemethanol, 2.18 g (16.87 mmol) of diisopropylethylamine, 10 ml of dichloromethane and 889 mg (4.44 mmol) of EPPA and the mixture was stirred at room temperature for 2 days. To the reaction mixture was added 10 ml of water and shaken. The dichloromethane layer was separated, and the aqueous layer was extracted once again with 10 ml of dichloromethane. The organic layers were combined and washed with water (10 ml) and then saturated brine (10 ml). The mixture was dried over an anhydrous magnesium sulfate and concentrated under reduced pressure, and the residue was purified by silica gel column chromatography

(50g, n-hexane-ethyl acetate 4 : 1, V/V) to obtain 644 mg of the title compound as crystals (yield: 46%).
mp 125-126°C.
Elemental analysis: $C_{30}H_{27}F_2NO_2$
Calculated: C, 76.41; H, 5.77; N, 2.97.
Found: C, 76.21; H, 5.82; N, 2.79.
IR (KBr): 2948, 2844, 2786, 1708, 1625, 1598, 1504, 1471, 1415, 1340, 1297, 1250, 1191, 1157, 1104, 1085, 1068, 1057, 1024, 953, 854, 824cm$^{-1}$.

Example 149

3-[Bis(4-fluorophenyl)methyl]-1-[(3-hydroxy-6-methyl-2-pyridinyl)methyl]-4-piperidinone

A 25 ml round-bottomed flask was charged with 1.0 g (2.96 mmol) of 3-[bis(4-fluorophenyl)methyl]-4-piperidinone hydrochloride, 453 mg (3.26 mmol) of 2, 6-lutidine-$\alpha^2$-3-diol, 2.18 g (16.87 mmol) of diisopropylethylamine, 10 ml of dichloromethane and 889 mg (4.44 mmol) of EPPA and the mixture was stirred at room temperature for 6 days. To the reaction mixture was added 10 ml of water and shaken. The dichloromethane layer was separated, and the aqueous layer was extracted once again with 10 ml of dichloromethane. The organic layers were combined and washed with water (10 ml) and saturated brine (10 ml). The mixture was dried over an anhydrous magnesium sulfate and concentrated under reduced pressure, and the residue was purified by silica gel column chromatography (50g, n-hexane-ethyl acetate 1 : 1, V/V) to obtain 537 mg of the title compound (yield: 43%).
Elemental analysis: $C_{25}H_{24}F_2N_2O_2$
Calculated: C, 71.07; H, 5.73; N, 6.63.
Found: C, 70.92; H, 5.74; N, 6.69.
IR (KBr): 3433, 1721, 1604, 1509, 1468, 1272, 1226, 1160, 829, 733, 556 cm$^{-1}$.

Example 150

3-[Bis(4-fluorophenyl)methyl]-1-[(2, 6-dimethoxy-3-pyridinyl)methyl]-4-piperidinone

(Step 1) A 200 ml round-bottomed flask was charged with 5.0 g (27.3 mmol) of 2, 6-dimethoxynicotinic acid, 100ml of ethanol and 5.4 g (54.6 mmol) of sulfuric acid, and the mixture was heated under reflux for 2 days. After concentration under reduced pressure, to the residue were added 100 ml of ethyl acetate and 50 ml of water, the mixture was shaken, and the organic layer was separated. The aqueous layer was neutralized and then extracted once again with ethyl acetate. The ethyl acetate layers were combined, and washed with 100 ml of water and 100 ml of saturated brine. The mixture was dried over magnesium sulfate and concentrated under reduced pressure to obtain 2, 6-dimethoxynicotinic acid ethyl ester. This was dissolved in 50 ml of toluene and 10.26 g (35.5 mmol) of $NaAlH_2(OCH_2CH_2OMe)_2$ was added thereto. Then, the mixture was stirred under ice-cooling for 1 hour and then at room temperature for 7 hours. To the reaction mixture were added 50 ml of 10% Rochelle salt and 50 ml of ethyl acetate, and the organic layer was separated. The aqueous layer was extracted once again with ethyl acetate and the ethyl acetate layers were combined and washed with 50 ml of water and 50 ml of saturated brine. The mixture was dried over magnesium sulfate and concentrated under reduced pressure to obtain 2, 6-dimethoxy-3-pyridylmethanol.
(Step 2) A 25 ml round-bottomed flask was charged with 1.0 g (2.96 mmol) of 3-[bis(4-fluorophenyl)methyl]-4-piperidinone hydrochloride, 551 mg (3.26 mmol) of 2, 6-dimethoxy-3-pyridylmethanol, 2.18 g (16.87 mmol) of diisopropylethylamine, 10 ml of dichloromethane, and 889 mg (4.44 mmol) of EPPA and the mixture was stirred at room temperature for about 3 days. To the reaction mixture was added 10 ml of water, the mixture was shaken and the dichloromethane layer was separated. The aqueous layer was extracted once again with 10 ml of dichloromethane, and the organic layers were combined and washed with water (10 ml), followed by a saturated brine (10 ml). The mixture was dried over an anhydrous magnesium sulfate, concentrated under reduced pressure, and the residue was purified by silica gel column chromatography (50g, n-hexane-ethyl acetate 4 : 1, V/V) to obtain 550 mg of the title compound (yield:41%).
IR (KBr):2949, 2803, 1718, 1605, 1589, 1506, 1482, 1421, 1389, 1318, 1225, 1159, 1105, 1074, 1022, 955, 826, 806, 735, 578, 546 cm$^{-1}$.

Example 151

3-Benzhydryl-1-[(2, 6-dimethoxy-3-pyridinyl)methyl]-4-piperidinone

A 25 ml round-bottomed flask was charged with 1.0 g (3.64 mmol) of 3-benzhydryl-4-piperidinone hydrochloride, 617 mg (3.64 mmol) of 2, 6-dimethoxy-3-pyridylmethanol, 2.44 g (18.89 mmol) of diisopropylethylamine, 10 ml of dichloromethane, and 995 mg (4.97 mmol) of EPPA and the mixture was stirred at room temperature for about 5 days. To the reaction mixture was added 10 ml of water, the mixture was shaken and the dichloromethane layer was separated. The aqueous layer was extracted once again with 10 ml of dichloromethane, and the organic layers were combined and washed with water (10 ml) followed by a saturated brine (10 ml). The mixture was dried over an anhydrous magnesium sulfate, concentrated under reduced pressure, and the residue was purified by silica gel column chromatography (50g, n-hexane-ethyl acetate 4 : 1, V/V) to obtain 806 mg of the title compound (yield:58%).
IR (KBr):3443, 2947, 1718, 1604, 1588, 1481, 1453, 1421, 1389, 1317, 1249, 1209, 1102, 1022, 807, 748, 704, 547 $cm^{-1}$.

Example 152

3-Benzhydryl-1-[[3, 5-(bistrifluoromethyl)phenyl]acetyl]-4-piperidinone

A solution of 3.0 g (10 mmol) of 3-benzhydryl-4-piperidinone hydrochloride, 1 g (10 mmol) of triethylamine, 2.99 g (11 mmol) of 3, 5-(bistrifluoromethyl)phenylacetic acid, 3.26 g (17 mmol) of WSC, 610 mg (5 mmol) of DMAP, and 13 ml of DMF was stirred at room temperature for 4.5 hours. To the reaction mixture was added 150 ml of water, 100 ml of ethyl acetate and 30 ml of hexane, and the mixture was shaken and then separated into layers. The organic layer was washed with 0.3 N hydrochloric acid (30 ml x 2) and an aqueous sodium bicarbonate, dried and concentrated under reduced pressure. The crystalline residue was washed with isopropyl ether and dried to obtain 4.59 g of the title compound as crystals (yield: 88%).
mp 124-124.5°C.
Elemental analysis: $C_{28}H_{23}F_6NO_2$
Calculated: C, 64.74; H, 4.46; N, 2.70.
Found: C, 64.75; H, 4.55; N, 2.60.
$^1$H-NMR ($CDCl_3$) δ: 2.42-4.37 (m, 10H), 7.17-7.83 (m, 13H).

Example 153

3-Benzhydryl-1-[(2-methoxy-4-methylphenyl)sulfonyl]-4-piperidinone

A suspension of 604 mg (2 mmol) of 3-benzhydryl-4-piperidinone hydrochloride in 4 ml of dichloromethane was dissolved by addition of 323 mg (2.5 mmol) of isopropylethylamine. Then, 122 mg (1 mmol) of DMAP and 663 mg (3 mmol) of 2-methoxy-4-methylbenzensulfonyl chloride were added thereto and the mixture was stirred at room temperature for 1.5 hours. To the reaction mixture were added 20 ml of ethyl acetate, 20 ml of diisopropyl ether and 20 ml of water, the mixture was shaken and separated into layers. The organic layer was washed with an aqueous sodium bicarbonate, dried and concentrated under reduced pressure. The crystalline residue was washed with diisopropyl ether and dried to obtain 820 mg of the title compound as crystals (yield: 91%).
mp 130-131°C.
Elemental analysis: $C_{26}H_{27}NO_4$•$0.1H_2O$(451.364)
Calculated: C, 69.19;H, 6.07;N, 3.10.
Found: C, 69.11;H, 6.10;N, 3.00.
$^1$H-NMR ($CDCl_3$)δ:2.39 (s, 3H), 2.41-2.58 (m, 2H), 3.16-3.21 (m, 1H), 3.43-3.48 (m, 1H), 3.57 (br, 1H), 3.79 (s, 3H), 4.49 (d, 1H), 6.78 (s, 1H), 7.13-7.30 (m, 10H), 7, 69 (d, 1H).

Example 154

3-Benzhydryl-1-[[2-(methylthio)-3-pyridinyl]carbonyl]-4-piperidinone

To 604 mg (2.0 mmol) of 3-benzhydryl-4-piperidinone hydrochloride was added with 6 ml of dichloromethane, and then 774 mg (6.0 mmol) of diisopropylethylamine was added to the mixture with stirring at room temperature. After dissolution, 122 mg (6.0 mmol) of DMAP and 774 mg (6 mmol) of 2-methylthionicotinic acid chloride were added thereto and the mixture was stirred for 2 hours. To the reaction mixture were added 20 ml of isopropyl ether, 20 ml of ethyl acetate and 20 ml of water, the mixture was shaken, and then the organic layer was separated. The organic layer was washed successively with aqueous sodium bicarbonate, dilute hydrochloric acid, aqueous sodium bicarbonate and water. After drying over magnesium sulfate, the layer was concentrated under reduced pressure to obtain 0.83 g of the title compound as a pale yellow solid (quantitative).
$^1$H-NMR ($CDCl_3$) δ: 2.53-4.75 (m, 11H), 6.87-7.45 (m, 12H), 8.44, 8.52 (m, 1H).

Elemental analysis: $C_{25}H_{24}N_2O_2$•0.2AcOEt
Calculated: C, 71.37; H, 5.94; N, 6.45.
Found: C, 71.28; H, 5.91; N, 6.57.

Example 155

3-Benzhydryl-1-[(3-methoxy-2-naphtyl)methyl]-4-piperidinone

(Step 1) A 50 ml round-bottomed flask was charged with 2.3 g (10.6 mmol) of 3-methoxy-2-naphthalenecarboxylic acid methyl ester and 18ml of toluene, and to the mixture was added 4.61 g (16 mmol) of $NaAlH_2(OCH_2CH_2OMe)_2$ with stirring under ice-cooling. The mixture was further stirred for about 2 hours under ice-cooling and then for 1 hour at room temperature. To the reaction mixture were added 20 ml of ethyl acetate and 20 ml of a 10% aqueous solution of Rochelle salt, the mixture was shaken and the organic layer was separated. The aqueous layer was extracted once again with 20 ml of ethyl acetate, and the organic layers were combined and washed twice with 20 ml of water and then twice with 20 ml of a brine. The organic layer was filtered through Celite, dried over $MgSO_4$ (anhydrous), concentrated under reduced pressure to obtain 1.47 g of 3-methoxy-2-naphthylmethanol (yield: 73%).
$^1$H-NMR ($CDCl_3$) δ: 3.96 (s, 3H), 4.82 (s, 2H), 7.12 (s, 1H), 7.19-7.51 (m, 2H), 7.72-7.80 (m, 3H).

(Step 2) A 25 ml round-bottomed flask was charged with 700 mg (3.72 mmol) of 3-methoxy-2-naphthylmethanol, 1.12 g (3.72 mmol) of 3-benzhydryl-4-piperidinone hydrochloride, 2.74 g (21.2 mmol) of diisopropylethylamine and 4 ml of dichloromethane, and an EPPA 1.12 g (5.58 mmol)/dichloromethane 2ml solution was added thereto with stirring and then the mixture was stirred at room temperature for 2 days. To the reaction mixture were added 20 ml of dichloromethane and 20 ml of an aqueous sodium bicarbonate, the mixture was shaken, and then dichloromethane layer was separated. The aqueous layer was extracted again with 20 ml of dichloromethane, and then the organic layers were combined, and then washed successively with 20 ml of a saturated aqueous sodium bicarbonate, 20 ml of water and 20 ml of saturated brine. After drying over anhydrous magnesium sulfate followed by concentration under reduced pressure, the residue was subjected to silica gel column chromatography (50g, n-hexane-ethyl acetate 5 : 1, V/V) to obtain 0.31 g of the title compound (yield:19%).
$^1$H-NMR ($CDCl_3$) δ: 2.40-2.42 (m, 1H), 2.47-2.56 (m, 2H), 2.69-2.72 (m, 1H), 2.80-2.82 (m, 1H), 3.40-3.43 (m, 1H), 3.69 (m, 2H), 3.84 (m, 3H), 4.59 (d, 1H, J=10.8Hz), 6.98-7.03 (m, 3H), 7.10-7.13 (m, 4H), 7.18-7.23 (m, 2H), 7.28-7.36 (m, 3H), 7.40-7.44 (m, 1H), 7.70-7.75 (m, 3H).
Elemental analysis: $C_{30}H_{29}NO_2$•0.2$H_2O$
Calculated: C, 82.05; H, 6.75; N, 3.19.
Found: C, 82.12; H, 6.81; N, 3.07.
IR (KBr): 3026, 2951, 2795, 1716, 1633, 1599, 1494, 1473, 1451, 1432, 1398, 1344, 1245, 1194, 1157, 1089, 1016, 898, 862, 830, 783, 745, 702, 622, 545, 477$cm^{-1}$.

Example 156

3-Benzhydryl-1-[(3-methoxy-1-benzothien-2-yl)methyl]-4-piperidinone

To 604 mg (2.0 mmol) of 3-benzhydryl-4-piperidinone hydrochloride was added 5 ml of dichloromethane, and to the mixture was added 1.3 g (10 mmol) of diisopropylethylamine with stirring at room temperature. After dissolution, 388 mg (2.0 mmol) of (3-methoxy-1-benzothien-2-yl)methanol and 800 mg (4 mmol) of EPPA were added thereto, and the mixture was stirred for 40 hours. To the reaction mixture were added 40 ml of isopropyl ether, 40 ml of ethyl acetate and 30 ml of water, the mixture was shaken, and the organic layer was separated. The organic layer was dried over anhydrous magnesium sulfate and concentrated under reduced pressure, and the residue was subjected to silica gel column chromatography (40g, n-hexane-ethyl acetate 4 : 1, V/V) to obtain 200 mg of the title compound (yield:23%).
$^1$H-NMR ($CDCl_3$) δ: 2.31-4.37 (m, 1H), 2.53-2.73 (m, 4H), 2.94-2.99 (m, 1H), 3.37-3.40 (m, 1H), 3.73 (d, 1H), 3.79 (d, 1H), 3.84 (s, 3H), 4.67 (d, 1H), 7.00-7.15 (m, 4H), 7.22-7.26 (m, 4H), 7.33-7.38 (m, 4H), 7.67 (d, 1H), 7.77 (d, 1H).
Elemental analysis: $C_{28}H_{27}NO_2S$
Calculated: C, 76.16; H, 6.16; N, 3.17.
Found: C, 76.05; H, 6.13; N, 3.17.

Example 157

1-Benzyl-3-[(2-fluorophenyl)(phenyl)methyl]-4-piperidinone

(Step 1) To a solution of 2.0 g (10 mmol) of 2-fluorobenzophenone in 20 ml of methanol was added 0.50 g of

sodium borohydride with stirring under ice-cooling, and the mixture was stirred for 2 hours at room temperature until effervescence ceased. Then, methanol was distilled off under reduced pressure and 15 ml of 1 N HCl was added thereto. The mixture was extracted twice with 25 ml of toluene. The organic layer was washed with water, and concentrated under reduced pressure. For dehydration, the residue was dissolved in 25 ml of toluene and the solution was reconcentrated to obtain 1.75 g of 2-fluorobenzhydrol (yield: 87%).

(Step 2) To a solution of 1.89 g (10.0 mmol) of 1-benzyl-4-piperidinone in 20 ml of dichloromethane was added dropwise 4.07 ml (22.5 mmol) of trimethylsilyl triflate (TMSOTf) with stirring under ice-cooling at a temperature of 10°C or below. After 10 minutes, a solution of 1.75 g (8.65 mmol) of 2-fluorobenzhydrol in 3 ml of dichloromethane was added thereto. After stirring for 1 hour at room temperature, an aqueous solution of sodium carbonate ($Na_2CO_3$ 2g/ $H_2O$ 20 ml) was added dropwise under ice-cooling. The organic layer was separated, and the aqueous layer was extracted once again with 20 ml of ethyl acetate, and the organic layers were combined and dried over anhydrous magnesium sulfate. The mixture was concentrated under reduced pressure and the residue was subjected to silica gel column chromatography (50 g, n-hexane-ethyl acetate 6 : 1 - 3 : 1, V/V) to obtain the title compound. Then, this was dissolved in a solution of 1 ml of conc. hydrochloric acid diluted with 10 ml of ethanol and allowed to stand in a refrigerator overnight. Then, the precipitated crystals were collected by filtration, washed with 20 ml of ethanol and dried under reduced pressure to obtain 2.53 g of the hydrochloride of the title compound (yield: 71%).
mp 214-215°C.
$^1$H-NMR ($CDCl_3$) δ: 2.41-3.04 (m, 3H), 3.42-3.66 (m, 3H), 3.92-4.06 (m, 1H), 4.24-4.31 (m, 1H), 4.56 (d, 0.5H, J=10.2Hz), 4.68 (d, 0.5H, J=11.4Hz), 4.85-5.02 (m, 1H), 6.91-7.53 (m, 14H).
IR (KBr): 3436, 2911, 2380, 1728, 1491 $cm^{-1}$.
Elemental analysis: $C_{25}H_{24}FNO$•HCl
Calculated: C, 73.25; H, 6.15.
Found: C, 73.10; H, 5.93.

Example 158

1-Benzyl-3-[(2-chlorophenyl)(4-chlorophenyl)methyl]-4-piperidinone

According to the same manner as that described in Step 2 of Example 157, the hydrochloride of the title compound was obtained from 2,4'-dichlorobenzhydrol and trimethylsilyl triflate.
mp 190-191°C.
$^1$H-NMR ($CDCl_3$) δ: 2.42-4.05 (m, 7H), 4.23-4.31 (m, 1H), 4.24-4.31 (m, 1H), 4.68 (d, J=10.6Hz), 4.93-5.06 (m, 1H), 7.07-7.55 (m, 13H).
IR (KBr): 3442, 3063, 2908, 2459, 2383, 1732, 1492, 1425, 1438, 1444, 1411, 1368, 1341, 1089, 1036, 1014, 755, 700, 549, 522 $cm^{-1}$.
Elemental analysis: $C_{25}H_{23}Cl_2NO$•HCl•0.5EtOH
Calculated: C, 64.54; H, 5.62.
Found: C, 64.54; H, 5.46.

Example 159

3-[Bis(4-fluorophenyl)methyl]-1-(3, 5-di-tert-butyl-2-methoxybenzyl)-4-piperidinone

(Step 1) A 100ml four-necked flask was charged with 5.0 g (20.1 mmol) of 3, 5-di-tert-butyl-2-methoxybenzyl aldehyde and 100 ml of ethanol. Then, 762 mg (20.1 mmol) of sodium borohydride was added thereto with stirring under ice-cooling and the mixture was stirred for 1 hour and allowed to stand at room temperature overnight. After concentrating under reduced pressure, to the residue were added 100 ml of ethyl acetate and 100 ml of water, the mixture was shaken, and then the organic layer was separated. The aqueous layer was extracted with 100 ml of ethyl acetate, and combined with the above organic layer. The mixture was washed with 100 ml of water followed by 100 ml of saturated brine. After drying over anhydrous magnesium sulfate, the mixture was concentrated under reduced pressure, and the residue was recrystallized from ether to obtain 2.0 g of 3,5-di-tert-butyl-2-methoxybenzyl alcohol (yield:40%).
$^1$H-NMR ($CDCl_3$) δ: 1.31 (s, 9H), 1.40 (s, 9H), 3.82 (s, 3H), 4.77 (d, 1H, J= 5.2Hz), 7.26 (d, 1H, J= 2.4Hz), 7.30 (d, 1H, J= 2.8Hz).

(Step 2) A 25 ml round-bottomed flask was charged with 1.0 g (2.96 mmol) of 3-[bis(4-fluorophenyl)methyl]-4-piperidinone hydrochloride, 0.82 g (3.26 mmol) of 3,5-di-tert-butyl-2-methoxybenzyl alcohol, 2.18 g (16.87 mmol) of diisopropylethylamine, 10 ml of dichloromethane, and 889 mg (4.44 mmol) of EPPA, and the mixture was stirred at room temperature for about 3 days. To the reaction mixture was added 10 ml of water, the mixture was shaken and

then the dichloromethane layer was separated. The aqueous layer was extracted with 10 ml of dichloromethane, and then the organic layers were combined and washed with water (10 ml) followed by a saturated brine (10 ml). After drying over anhydrous magnesium sulfate, the mixture was concentrated under reduced pressure, and the residue was purified by silica gel column chromatography (50g, n-hexane-ethyl acetate 4 : 1, V/V) to obtain 854 mg of the title compound (yield:54%).
Elemental analysis: $C_{34}H_{41}F_2NO_2$
Calculated: C, 76.52; H, 7.74; N, 2.62.
Found: C, 76.44; H, 7.79; N, 2.49.
IR (KBr): 3421, 2962, 2868, 2800, 1719, 1604, 1508, 1478, 1429, 1392, 1362, 1346, 1301, 1274, 1229, 1158, 1102, 1074, 1013, 904, 884, 825, 795, 768, 751, 653, 579, 550 $cm^{-1}$.

Example 160

3-[Bis(4-fluorophenyl)methyl]-1-(2, 5-dimethoxybenzyl)-4-piperidinone

(Step 1) A 100ml four-necked flask was charged with 5.0 g (30.1 mmol) of 2, 5-dimethoxybenzaldehyde and 100 ml of ethanol. Then, 1.14 g (30.1 mmol) of sodium borohydride was added thereto with stirring under ice-cooling and the mixture was stirred for 2 hours and then allowed to stand at room temperature overnight. After concentrating under reduced pressure, to the residue were added 100 ml of ethyl acetate and 100 ml of water, the mixture was shaken, and then the organic layer was separated. The aqueous layer was extracted once again with 100 ml of ethyl acetate, and the ethyl acetate layers were combined and then washed with 100 ml of water followed by 100 ml of saturated brine. After drying over anhydrous magnesium sulfate, the mixture was concentrated under reduced pressure to obtain 2.5 dimethoxybenzyl alcohol.
$^1$H-NMR ($CDCl_3$) δ: 3.77 (s, 3H), 3.82 (s, 3H), 4.65 (s, 2H), 6.79 (d, 1H, J= 2.8Hz), 6.80 (s, 1H), 6.89 (d, 1H, J= 2.8Hz).

(Step 2) A 25 ml round-bottomed flask was charged with 1.0 g (2.96 mmol) of 3-[bis(4-fluorophenyl)methyl]-4-piperidinone hydrochloride, 0.55 g (3.26 mmol) of 2, 5-dimethoxybenzyl alcohol, 2.18 g (16.87 mmol) of diisopropylethylamine, 10 ml of dichloromethane, and 889 mg (4.44 mmol) of EPPA and the mixture was stirred at room temperature for about 6 days. To the reaction mixture was added 10 ml of water, the mixture was shaken and the dichloromethane layer was separated. The aqueous layer was extracted once again with 10 ml of dichloromethane, and the organic layers were combined and washed with water (10 ml) followed by saturated brine (10 ml). After drying over anhydrous magnesium sulfate, the mixture was concentrated under reduced pressure, the residue was purified by silica gel column chromatography (50 g, n-hexane-ethyl acetate 3 : 1, V/V) to obtain 649 mg of the title compound (yield:48%).
Elemental analysis: $C_{27}H_{27}F_2NO_3$
Calculated: C, 71.82; H, 6.03; N, 3.10.
Found: C, 71.61; H, 6.23; N, 2.93.
IR (KBr): 2952, 2834, 1716, 1603, 1507, 1466, 1347, 1274, 1224, 1178, 1158, 1103, 1049, 1028, 825, 712, 579, 553 $cm^{-1}$.

Example 161

3-Benzhydryl-1-(3, 5-di-tert-butyl-2-methoxybenzyl)-4-piperidinone

A 25 ml round-bottomed flask was charged with 1.0 g (3.28 mmol) of 3-benzhydryl-4-piperidinone hydrochloride, 0.91 g (3.61 mmol) of 3, 5-di-tert-butyl-2-methoxybenzyl alcohol, 2.42 g (18.7 mmol) of diisopropylethylamine, 10 ml of dichloromethane and 985 mg (4.92 mmol) of EPPA, and the mixture was stirred at room temperature for about 5 days. To the reaction mixture was added with 10 ml of water, the mixture was shaken and the dichloromethane layer was separated. The aqueous layer was extracted once again with 10 ml of dichloromethane, and the organic layers were combined and washed with water (10 ml) followed by saturated brine (10 ml). After drying over anhydrous magnesium sulfate, the mixture was concentrated under reduced pressure, the residue was purified by silica gel column chromatography (50 g, n-hexane-ethyl acetate 6 : 1, V/V) to obtain 512 mg of the title compound (yield: 32%).
mp 136-138°C.
Elemental analysis: $C_{34}H_{43}NO_2$
Calculated: C, 82.05; H, 8.71; N, 2.81.
Found: C, 81.97; H, 9.00; N, 2.59.
IR (KBr): 3431, 2960, 1719, 1477, 1361, 1231, 1011, 744, 703, 549 $cm^{-1}$.
$^1$H-NMR ($CDCl_3$) δ: 1.33 (s, 9H), 1.42 (s, 9H), 2.33-2.38 (m, 1H), 2.46-2.59 (m, 2H), 2.64-2.73 (m, 2H), 2.79-2.84 (m, 1H), 3.36-3.41 (m, 1H), 3.50 (d, 1H , J= 13.2Hz), 3.61 (d, 1H , J=13.2Hz), 3.77 (s, 3H), 4.62 (d, 1H , J=11.2Hz), 7.08-7.16

(m, 6H), 7.21-7.30 (m, 4H), 7.35-7.36 (m, 1H).

Example 162

(+)-3-[Bis(4-fluorophenyl)methyl]-1-(5-bromo-2-methoxybenzyl)-4-piperidinone

The optically active title compound (412 mg) was obtained by subjecting 1 g of 3-[bis(4-fluorophenyl)methyl]-1-(5-bromo-2-methoxybenzyl)-4-piperidinone obtained in Example 76 to HPLC (CHIRALPAK AD 50 mm ID × 500 mm L, hexane/IPA).
Retention time: short
Optical purity: 99.6% ee.
mp 54-55°C.
$[\alpha]_D$= +93.0° (c = 1.049%, in $CHCl_3$).

Example 163

(-)-3-[Bis(4-fluorophenyl)methyl]-1-(5-bromo-2-methoxybenzyl)-4-piperidinone

The optically active title compound (427 mg) was obtained by subjecting 1 g of 3-[bis(4-fluorophenyl)methyl]-1-(5-bromo-2-methoxybenzyl)-4-piperidinone obtained in Example 76 to HPLC (CHIRALPAK AD 50 mm ID x 500 mm L, hexane/IPA).
Retention time: long
Optical purity: 99.9% ee.
mp 52-53°C.
$[\alpha]_D$= -94.3°(c = 1.049%, in $CHCl_3$).

Example 164

3-[Bis(4-fluorophenyl)methyl]-1-[2-(methylsulfanyl)benzyl]-4-piperidinone

(Step 1) A 100 ml round-bottomed flask was charged with 2.12 g (39.2 mmol) of sodium methylate, 50 ml of methanol, 5 g (35.7 mmol) of 2-mercaptobenzyl alcohol and 5.57 g (39.2 mmol) of methyl iodide and the mixture was allowed to stand at room temperature for about 3 days. After concentrating under reduced pressure, 50 ml of ethyl acetate and 50 ml of water were added to the residue, the mixture was shaken, and then the ethyl acetate layer was separated. The aqueous layer was extracted once again with 50 ml of ethyl acetate, and the organic layers were combined, washed with 50 ml of water followed by 50 ml of saturated brine. After drying over anhydrous magnesium sulfate, the mixture was concentrated under reduced pressure, the residue was purified by silica gel column chromatography (200g, n-hexane-ethyl acetate 4 : 1, V/V) to obtain 4.95 g of the title compound (yield:90%).

(Step 2) A 25 ml round-bottomed flask was charged with 1.0 g (2.96 mmol) of 3-[bis(4-fluorophenyl)methyl]-4-piperidinone hydrochloride, 502 mg (3.26 mmol) of 2-methylthiobenzyl alcohol, 2.18 g (16.87 mmol) of diisopropylethylamine, 10 ml of dichloromethane and 889 mg (4,44 mmol) of EPPA, the mixture was stirred at room temperature for about 5 days. To the reaction mixture was added 10 ml of water, the mixture was shaken and the dichloromethane layer was separated. The aqueous layer was extracted once again with 10 ml of dichloromethane, and then the organic layers were combined and washed with water (10 ml) followed by saturated brine (10 ml). The mixture was dried over an anhydrous magnesium sulfate, concentrated under reduced pressure, and the residue was purified by silica gel column chromatography (50g, n-hexane-ethyl acetate 4 : 1, V/V) to obtain 432 mg of the title compound (yield: 33%).
IR (KBr): 2923, 2803, 1715, 1603, 1508, 1469, 1440, 1347, 1224, 1189, 1159, 1103, 1070, 1015, 830, 750, 578, 547 $cm^{-1}$.
$^1$H-NMR ($CDCl_3$)δ:2.24-2.28 (m, 1H), 2.44-2.48 (m, 1H), 2.52-2.66 (m, 2H), 2.54 (s, 3H), 3.05-3.15 (m, 2H), 3.40 (d, 1H, J=13.2Hz), 3.68 (d, 1H, J=12.4Hz), 4.64 (d, 1H, J=11.2Hz), 6.72-6.77 (m, 2H), 6.89-6.99 (m, 4H), 7.03-7.13 (m, 2H), 7.24-7.26 (m, 2H), 7.30-7.35 (m, 2H).

Example 165

3-Benzhydryl-1-[2-(methylsulfanyl)benzyl]-4-piperidinone

According to the same treatment as that described with respect to 3-[bis(4-fluorophenyl)methyl]-1-[2-(methylsulfanyl)benzyl]-4-piperidinone, a crude material was obtained by using 1.0 g (2.61 mmol) of 3-benzhydryl-4-piperidinone hydrochloride, 442 mg (2.87 mmol) of 2-methylthiobenzyl alcohol, 1.92 g (14.87 mmol) of diisopropylethylamine and 783 mg (3.91 mmol) of EPPA, and purified by silica gel column chromatography (50 g, n-hexane-ethyl acetate 6 : 1, V/V) to obtain 812 mg of the title compound (yield: 77%).
IR (KBr): 3060, 3028, 2921, 2801, 1715, 1590, 1494, 1468, 1451, 1345, 1323, 1240, 1190, 1135, 1089, 1068, 1034, 992, 911, 784, 747, 704, 549cm$^{-1}$.

Example 166

3-[Bis(4-fluorophenyl)methyl]-1-(5-fluoro-2-methoxybenzyl)-4-piperidinone

(Step 1) A 100ml four-necked flask was charged with 5.0 g (32.4 mmol) of 5-fluoro-2-methoxybenzaldehyde and 100ml of ethanol, and 1.23 g (32.4 mmol) of sodium borohydride was added thereto with stirring under ice-cooling. Then, the mixture was stirred for 1 hour and allowed to stand at room temperature overnight. After concentrating under reduced pressure, 50 ml of ethyl acetate and 50 ml of water were added to the residue, and the mixture was shaken and separated into layers. The aqueous layer was extracted once again with 50 ml of ethyl acetate. The ethyl acetate layers were combined, and washed with 50 ml of water followed by 50 ml of saturated brine. After drying over anhydrous sodium sulfate, the mixture was concentrated under reduced pressure to obtain 5-fluoro-2-methoxybenzyl alcohol.
$^1$H-NMR ($CDCl_3$) δ: 3.83 (s, 3H), 4.66 (d, 2H , J= 6.4Hz), 6.77-7.07 (m, 3H).
(Step 2) A 25 ml round-bottomed flask was charged with 1.0 g (2.96 mmol) of 3-[bis(4-fluorophenyl)methyl]-4-piperidinone hydrochloride, 508 mg (3.26 mmol) of 5-fluoro-2-methoxybenzyl alcohol, 2.18 g (16.87 mmol) of diisopropylethylamine, 10 ml of dichloromethane and 889 mg (4.44 mmol) of EPPA, and the mixture was stirred at room temperature for about 4 days. To the reaction mixture was added 10 ml of water, and the dichloromethane layer was separated. The aqueous layer was extracted once again with 10 ml of dichloromethane, and then the organic layers were combined and washed with water (10 ml) followed by saturated brine (10 ml). The mixture was dried over an anhydrous magnesium sulfate and concentrated under reduced pressure, and the residue was purified by silica gel column chromatography (50 g, n-hexane-ethyl acetate 4 : 1, V/V) to obtain 642 mg of the title compound (yield: 49.4%).
IR (KBr): 2484, 1729, 1604, 1508, 1224, 1159, 1028, 823, 720, 555cm$^{-1}$.
$^1$H-NMR ($CDCl_3$) δ: 2.38-2.61 (m, 4H), 2.74-2.78 (m, 1H), 2.85-2.90 (m, 1H), 3.26-3.31 (m, 1H), 3.52 (s, 2H), 3.74 (s, 3H), 4.58 (d, 1H, J=11.2Hz), 6.75 (m, 1H), 6.86-6.96 (m, 5H), 7.09-7.12 (m, 3H), 7.23-7.26 (m, 2H).

Example 167

3-[Bis(4-fluorophenyl)methyl]-1-[2-(methylsulfinyl)benzyl]-4-piperidinone

To a solution of 2 g (4.57 mmol) of 3-[bis(4-fluorophenyl)methyl]-1-[2-(methylsulfanyl)benzyl]-4-piperidinone in 10 ml of NMP, was added 452 mg (0.91 mmol) of magnesium monoperoxyphthalate (MMPP) in three portions at intervals of 20 minutes with stirring under ice-cooling. The stirring was continued for 1 hour under ice-cooling and then for 1 day at room temperature. After adding 20 ml of ethyl acetate and 20 ml of water, the mixture was shaken, and the organic layer was separated. The aqueous layer was extracted once again with 20 ml of ethyl acetate, and the ethyl acetate layers were combined and washed twice with 10 ml of water and then twice with 20 ml of saturated brine. After drying over anhydrous magnesium sulfate, the mixture was concentrated under reduced pressure, ethyl acetate was added to the residue, and the precipitated crystals were collected by filtration. The filtrate was dried under reduced pressure to obtain 239 mg of the title compound (yield: 12%).
mp187-189°C.
Elemental analysis: $C_{26}H_{25}F_2\ N_2O_2$ S
Calculated: C, 68.85; H, 5.56; N, 3.09.
Found: C, 68.78; H, 5.66; N, 2.96.
IR(KBr) 3426, 2919, 2809, 2360, 1718, 1603, 1508, 1473, 1417, 1348, 1224, 1159, 1121, 1065, 1028, 910, 830, 760, 732, 580, 560, 468 cm$^{-1}$.

Example 168

3-Benzhydryl-1-[2-methoxy-5-(trifluoromethoxy)benzyl]-4-piperidinone

(Step 1) To a solution of 660 mg (3 mmol) of 2-methoxy-5-trifluoromethoxybenzaldehyde in 3 ml of THF and 3 ml of methanol was added 114 mg (3 mmol) of sodium borohydride with stirring under ice-cooling, and the mixture was stirred for 0.5 hour. A saturated aqueous ammonium chloride and ethyl acetate were added thereto and the mixture was shaken. The ethyl acetate layer was washed with water, and then with saturated brine. After drying over anhydrous

magnesium sulfate, the mixture was concentrated under reduced pressure to obtain 2-methoxy-5-trifluoromethoxybenzyl alcohol as a colorless transparent oil.
$^1$H-NMR ($CDCl_3$) δ: 0.93(t, 1H), 3.87 (s, 3H), 4.68 (d, 2H), 6.85 (d, 2H), 7.11-7.14 (m, 1H), 7.21 (d, 1H).

(Step 2) To a solution of 855 mg (2.84 mmol) of 3-benzhydryl-4-piperidinone hydrochloride, 960 mg (4.8 mmol) of EPPA, 630 mg (2.84 mmol) of 2-methoxy-5-trifluoromethoxybenzyl alcohol in 7 ml of dichloromethane was added 1.55 g (12 mmol) of diisopropylethylamine with stirring, and the mixture was stirred at room temperature for 80 hours. To the reaction solution were added 50 ml of ethyl acetate, 30 ml of hexane and 25 ml of water, the mixture was shaken, and the organic layer was separated and washed with an aqueous sodium bicarbonate followed by water. After concentrating under reduced pressure, the residue was purified by silica gel column chromatography (40 g, n-hexane-ethyl acetate 4 : 1, V/V) to obtain 645.0 mg of the title compound as an oil (yield:49%). This was dissolved in 5 ml of ethanol, 1.5 ml of 1N-HCl was added thereto, and the solvent was distilled off under reduced pressure. The residue was triturated with 2 ml of ethanol, and the crystals were collected by filtration. After washing with small amount of ethanol, the filtrate was dried to obtain 666 mg of the hydrochloride of the title compound as crystals (yield: 46%).
mp 174-175°C.
Elemental analysis: $C_{27}H_{26}F_3NO_3$•HCl (505.956)
Calculated: C, 64.09; H, 5.38; N, 2.77.
Found: C, 64.11; H, 5.53; N, 2.65.
$^1$H-NMR ($CDCl_3$) δ: 2.38-4.96 (m, 13H), 7.06-7.89 (m, 13H), 7.62, 7.89(each s, total 1H).

Example 169

3-[Bis(4-fluorophenyl)methyl]-1-[2-methoxy-5-(trifluoromethoxy)benzyl]-4-piperidinone

(Step 1) A 25 ml round-bottomed flask was charged with 1.0 g (4.85 mmol) of 2-hydroxy-5-(trifluoromethoxy) benzaldehyde, 8ml of dimethylformamide (DMF) and 738 mg (5.34 mmol) of potassium carbonate, the mixture was stirred for about 30 minutes, and then a solution of methyl iodide 758 mg (5.34 mmol)/DMF 2 ml was added thereto under ice-cooling. The mixture was stirred under ice-cooling for 1 hour and then at room temperature for 19 hours. To the reaction mixture was added 20 ml of ethyl acetate and 20 ml of water, the mixture was shaken and the organic layer was separated. The aqueous layer was extracted once again with 20 ml of ethyl acetate, and the organic layers were combined, and washed twice with 20 ml of water and then twice with 20 ml of brine. The organic layer was dried over $MgSO_4$ (anhydrous) and concentrated under reduced pressure to obtain 1.0 g of 2-methoxy-5-(trifluoromethoxy) benzaldehydel (yield: 93%).
$^1$H-NMR ($CDCl_3$) δ: 3.96 (s, 3H), 7.02 (d, 1H, J=8.8Hz), 7.41 (d, 1H, J=9.2Hz), 7.69(1H, s, ), 10.44(1H, s).

(Step 2) A 25 ml round-bottomed flask was charged with 996 mg (4.52 mmol) of 2-methoxy-5-(trifluoromethoxy)benzaldehyde and 8 ml of tetrahydrofuran (THF), and a sodium borohydride 342 mg (9 mmol)/THF 2 ml was added thereto with stirring under ice-cooling. The mixture was stirred under ice-cooling for 1 hour and then at room temperature for 8 hours. Then, 2 ml of methanol was added thereto under ice-cooling, and the mixture was stirred at the same temperature for 30 minutes. To the reaction mixture were added 20 ml of ethyl acetate and 20 ml of water, the mixture was shaken and then the organic layer was separated. The aqueous layer was extracted once again with 20 ml of ethyl acetate, and the organic layers were combined, washed twice with 20 ml of water and then twice with 20 ml of brine. The organic layer was dried over $MgSO_4$ (anhydrous) and concentrated under reduced pressure to obtain 1.0 g of 2-methoxy-5-trifluoromethoxybenzyl alcohol (yield: 85%).
$^1$H-NMR ($CDCl_3$) δ: 3.87 (s, 3H), 4.68 (s, 2H), 6.85 (d, 1H, J=9.2Hz), 7.13 (d, 1H, J=8.8Hz), 7.21 (s, 1H).

(Step 3) A 10 ml round-bottomed flask was charged with 608 mg (1.80 mmol) of 3-[bis(4-fluorophenyl)methyl]-4-piperidinone hydrochloride, 400 mg (1.80 mmol) of 2-methoxy-5-trifluoromethoxybenzyl alcohol, 1.33 g (10.3 mmol) of diisopropylethylamine, 4 ml of dichloromethane, and 540 mg (2.7 mmol) of EPPA, and the mixture was stirred at room temperature for 7 hours. Further, additional 180 mg (0.9 mmol) of EPPA was added, and the mixture was allowed to stand for 2 days. To the reaction mixture was added 20 ml of water, the mixture was shaken, and the dichloromethane layer was separated. The aqueous layer was extracted once again with 20 ml of dichloromethane. The organic layers were combined and washed with water (20 ml) followed by saturated brine (20 ml). After drying over anhydrous magnesium sulfate, the mixture was concentrated under reduced pressure, the residue was subjected to silica gel column chromatography (50 g, n-hexane-ethyl acetate 5 : 1, V/V) to obtain the title compound.
$^1$H-NMR ($CDCl_3$) δ: 2.40-2.45 (m,. 2H), 2.49-2.61 (m, 2H), 3.30 (m, 1H), 3.53 (s, 2H), 3.77 (s, 3H), 4.56 (d, 1H, J=11.2Hz), 6.81 (d, 1H, J=9.2Hz), 6.87(dd, 2H, J=8.6Hz, J=8.8Hz), 6.94(dd, 2H, J=8.6Hz, J=8.8Hz), 7.07-7.12 (m, 3H), 7.22-7.25 (m, 3H).

The title compound was converted into the hydrochloride by a conventional method to obtain 210 mg of the hydrochloride of the title compound.
Elemental analysis: $C_{27}H_{25}F_5NO_3$•HCl•0.5$H_2O$

Calculated: C, 58.86; H, 4.76; N, 2.54.
Found: C, 58.76; H, 4.70; N, 2.42.

Example 170

3-Benzhydryl-1-(2-ethoxy-6-methoxybenzyl)-4-piperidinone

(Step 1) To a mixture of 3.04 g (20 mmol) of 2-hydroxy-6-methoxybenzaldehyde, 25 ml of dimethylformamide (DMF) and 5 ml of ethyl iodide was added 840 mg (21 mmol, washed with hexane) of 60% sodium hydride in oil with stirring at room temperature. The mixture was stirred at room temperature for about 13 hours. To the reaction mixture were added 100 ml of isopropyl ether, 100 ml of ether and 200 ml of water, the mixture was shaken and the organic layer was separated and washed with 20 ml of 0.1N-NaOH. The organic layer was dried over $MgSO_4$ (anhydrous); and then concentrated under reduced pressure to obtain 2.57 g of 2-ethoxy-6-methoxybenzaldehyde (yield: 72%) as a pale yellow oil.
$^1$H-NMR ($CDCl_3$) δ: 1.46(t, 3H), 3.90 (s, 3H), 4.12(q, 2H), 6.56 (d, 2H), 7.42(t, 1H, ), 10.53 (s, 1H).
(Step 2) To a mixture of 2.56 g (14.2 mmol) of 2-ethoxy-6-methoxybenzaldehyde, 20ml of tetrahydrofuran (THF) and 30 ml of methanol was added 380 mg (10 mmol) of sodium borohydride with stirring under ice-cooling. The mixture was stirred under ice-cooling for 4 hours and then at room temperature for 20 hours. The reaction mixture was concentrated under reduced pressure, and 50 ml of ethyl acetate and 20 ml of water were added to the residue. The mixture was shaken and the organic layer was separated, and washed with water. The organic layer was dried over $MgSO_4$ (anhydrous), and then concentrated under reduced pressure to obtain 2.47 g of 2-ethoxy-6-methoxybenzyl alcohol (yield: 96%).
$^1$H-NMR ($CDCl_3$) δ: 1.42(t, 3H), 2.56(t, 1H), 3.84 (s, 3H), 4.06(q, 2H), 4.80 (d, 2H), 6.54 (d, 2H), 7.19(t, 1H).
(Step 3) To 906 mg (3.0 mmol) of 3-benzhydryl-4-piperidinone hydrochloride was added 6 ml of dichloromethane, and then 1.0 g (7.8 mmol) of diisopropylethylamine was added to the mixture with stirring at room temperature. After dissolution, 728 mg (4.0 mmol) of 2-ethoxy-6-methoxybenzyl alcohol and 1 g (5 mmol) of EPPA were added and the mixture was stirred for 120 hours. To the reaction mixture were added 40 ml of isopropyl ether, 40 ml of ethyl acetate and 15 ml of water, the mixture was shaken, and the organic layer was separated, and washed with sodium bicarbonate. After drying over anhydrous magnesium sulfate, the mixture was concentrated under reduced pressure, the residue was washed twice with 20 ml of hexane. After addtion of 10 ml of ethanol, the precipitated crystals were collected by filtration, washed with ethanol and then dried to obtain 0.55 g of the title compound as colorless crystals (yield: 43%).
mp 137-138°C.
$^1$H-NMR ($CDCl_3$) δ: 1.28 (s, 3H), 2.40-4.58 (s, 3H), 2.70-2.74 (m, 1H), 2.85-2.88 (m, 2H), 3.36-3.41 (m, 1H), 3.71 (s, 3H), 3.72 (s, 2H), 3.93(q, 2H), 4.53 (d, 1H), 6.50(t, 2H), 7.09-7.30 (m, 11H).
Elemental analysis: $C_{28}H_{31}NO_3$•$0.1H_2O$
Calculated: C, 77.96; H, 7.29; N, 3.25.
Found: C, 77.77; H, 7.24; N, 3.11.

Example 171

3-[Bis(4-fluorophenyl)methyl]-1-(3,5-dibromo-2-methoxybenzyl)-4-piperidinone

(Step 1) A 35 ml round-bottomed flask was charged with 2.0 g (7.12 mmol) of 3, 5-dibromosalicylaldehyde, 15 ml of dimethylformamide (DMF) and 1.1 g (7.86 mmol) of potassium carbonate, the mixture was stirred for about 30 minutes, and then a methyl iodide 1.1 g (7.86 mmol)/DMF 2 ml solution was added thereto under ice-cooling. The mixture was stirred under ice-cooling for about 1 hour and then at room temperature for 14 hours. To the reaction mixture were added 20 ml of ethyl acetate and 20 ml of water, the mixture was shaken and the organic layer was separated. The aqueous layer was extracted once again with 20 ml of ethyl acetate, and the organic layers were combined, and washed twice with 20 ml of water and twice with 20 ml of brine. The organic layer was dried over $MgSO_4$ (anhydrous) and then concentrated under reduced pressure to obtain 1.4 g of 3, 5-dibromo-2-methoxybenzaldehyde (yield: 65%).
$^1$H-NMR ($CDCl_3$) δ: 3.99 (s, 3H), 7.91 (d, 1H, J=2.4Hz), 7.94 (d, 1H, J=2.4Hz), 10.28(1H, s).
(Step 2) A 25 ml round-bottomed flask was charged with 1.2 g (4.08 mmol) of 3, 5-dibromo-2-methoxybenzaldehyde and 6 ml of tetrahydrofuran (THF), and a sodium borohydride 309 mg (8.16 mmol)/EtOH 6ml was added thereto with stirring under ice-cooling. The mixture was stirred under ice-cooling for about 1 hour and then at room temperature for 3 hours. To the reaction solution were added 20 ml of ethyl acetate and 20 ml of water, the mixture was shaken and the organic layer was separated. The aqueous layer was extracted once again with 20 ml of ethyl acetate, and the organic layers were combined, and washed twice with 20 ml of water and twice with 20 ml of brine.

The organic layer was dried over $MgSO_4$ (anhydrous) and concentrated under reduced pressure. Diisopropyl ether was added to the residue and the precipitated crystals were collected by filtration and dried under reduced pressure to obtain 0.7 g of 3,5-dibromo-2-methoxybenzyl alcohol (yield: 58%).
$^1$H-NMR ($CDCl_3$) $\delta$: 3.85 (s, 3H), 4.72 (s, 2H), 7.51 (d, 1H, J=2.4Hz), 7.64 (d, 1H, J=2.4Hz).

(Step 3) A 10 ml round-bottomed flask was charged with 200 mg (0.68 mmol) of 3, 5-dibromo-2-methoxybenzyl alcohol, 228 mg (0.68 mmol) of 3-[bis(4-fluorophenyl)methyl]-4-piperidinone hydrochloride, 498 mg (3.85 mmol) of diisopropylethylamine and 2 ml of dichloromethane, 203 mg (1.01 mmol) of EPPA was added thereto with stirring and the mixture was stirred at room temperature for 3 days. To the reaction mixture were added 20 ml of dichloromethane and 20 ml of water, the mixture was shaken and the dichloromethane layer was separated. The aqueous layer was extracted once again with 20 ml of dichloromethane and the organic layers were combined and washed with water (20ml) followed by saturated brine (20ml). After drying over anhydrous magnesium sulfate, the mixture was concentrated under reduced pressure, the residue was subjected to silica gel column chromatography (50g, n-hexane-ethyl acetate 4 : 1, V/V) to obtain 0.21 g of the title compound (yield: 54%).
Elemental analysis: $C_{26}H_{23}Br_2F_2NO_2$•$0.3H_2O$
Calculated: C, 53.41; H, 407; N, 2.40.
Found: C, 53.44; H, 401; N, 2.36.
IR (KBr): 2934, 1718, 1604, 1508, 1464, 1419, 1346, 1225, 1158, 999, 826, 552 cm$^{-1}$.

Example 172

1,3-Bis[bis(4-fluorophenyl)methyl]-4-piperidinone

A 10 ml round-bottomed flask was charged with 652 mg (2.96 mmol) of 4, 4'-difluorobenzhydrol, 1.0 g (2.96 mmol) of 3-[bis(4-fluorophenyl)methyl]-4-piperidinone hydrochloride, 2.18 g (16.9 mmol) of diisopropylethylamine and 3 ml of dichloromethane, and EPPA 889 mg (4.44 mmol)/dichloromethane 4 ml was added thereto with stirring and the mixture was stirred for 3 days at room temperature. To the reaction mixture were added 20 ml of dichloromethane and 20 ml of water, the mixture was shaken, and the dichloromethane layer was separated. The aqueous layer was extracted once again with 20 ml of dichloromethane, and the organic layers were combined and washed with water (20 ml) followed by saturated brine (20ml). After drying over anhydrous magnesium sulfate, the mixture was concentrated under reduced pressure, the residue was subjected to silica gel column chromatography (50g, n-hexane-ethyl acetate, 6 : 1, V/V) to obtain 0.24 g of the title compound (yield: 16%).
$^1$H-NMR ($CDCl_3$) $\delta$: 2.26-2.30 (m, 1H), 2.44-2.74 (m, 5H), 3.33-3.38 (m, 1H), 4.33 (s, 1H), 4.54 (d, 1H, J=10.8Hz), 6.82-6.97 (m, 8H), 7.00-7.05 (m, 2H), 7.17-7.20 (m, 2H), 7.23-7.34 (m, 4H), 7.63 (d, 1H, J=2.8Hz).
Elemental analysis: $C_{31}H_{25}F_4NO$•$0.2H_2O$
Calculated: C, 73.42; H, 5.05; N, 2.76.
Found: C, 73.55; H, 5.16; N, 2.57.
IR (KBr) : 1717, 1604, 1508, 1226, 1158, 827, 734, 553 cm$^{-1}$.

Example 173

3-Benzhydryl-1-(3, 5-dichloro-2-methoxybenzyl)-4-piperidinone

(Step 1) A 35 ml round-bottomed flask was charged with 2.0 g (10.5 mmol) of 3, 5-dichloro-2-hydroxybenzaldehyde, 15 ml of dimethylformamide (DMF) and 1.6 g (11.5 mmol) of potassium carbonate, the mixture was stirred for 30 minutes, and a solution of methyl iodide 1.63 g (11.5 mmol)/DMF 5 ml was added thereto under ice-cooling. The mixture was stirred under with cooling in ice for about 1 hour and then at room temperature for 19 hours. To the reaction mixture were added 20 ml of ethyl acetate and 20 ml of water, the mixture was shaken and the organic layer was separated. The aqueous layer was extracted once again with 20 ml of ethyl acetate, and the organic layers were combined, and washed twice with 20 ml of water and twice with 20 ml of brine. The organic layer was dried over $MgSO_4$ (anhydrous) and concentrated under reduced pressure, and ethyl acetate was added to the residue. The precipitated crystals were collected by filtration and dried to obtain 0.89 g of 3,5-dichloro-2-methoxybenzaldehyde (yield: 42%).
$^1$H-NMR ($CDCl_3$) $\delta$: 4.00 (s, 3H), 7.63 (d, 1H, J=2.4Hz), 7.72 (d, 1H, J=2.8Hz), 10.31(1H, s).

(Step 2) A 25 ml round-bottomed flask was charged with 1.0 g (4.88 mmol) of 3, 5-dichloro-2-methoxybenzaldehyde, 5 ml of tetrahydrofuran (THF) and 5 ml of ethanol, and 369 mg (9.75 mmol) of sodium borohydride was added thereto with stirring under ice-cooling in ice. The mixture was stirred under ice-cooling for about 1 hour and then at room temperature for 3 hours. To the reaction mixture were added 20 ml of ethyl acetate and 20 ml of water, the mixture was shaken and the organic layer was separated. The aqueous layer was extracted once again with 20 ml of ethyl acetate, the organic layers were combined, and washed twice with 20 ml of water and twice with 20 ml of brine.

The organic layer was dried over $MgSO_4$ (anhydrous) and concentrated under reduced pressure, and diisopropyl ether was added to the residue. The precipitated crystals were collected by filtration and dried under reduced pressure to obtain 580 mg of 3,5-dichloro-2-methoxybenzyl alcohol (yield: 58%).
$^1$H-NMR ($CDCl_3$) δ: 3.87 (s, 3H), 4.70 (d, 2H, J=6.0Hz), 7.30-7.32 (m, 2H).

(Step 3) A 5 ml round-bottomed flask was charged with 200 mg (0.97 mmol) of 3, 5-dichloro-2-methoxybenzyl alcohol, 292 mg (0.97 mmol) of 3-benzhydryl-4-piperidinone hydrochloride, 712 mg (5.51 mmol) of diisopropylethylamine and 1 ml of dichloromethane, and a solution of EPPA 203 mg (1.01 mmol)/ dichloromethane 1 ml was added thereto with stirring and the mixture was stirred at room temperature for 2 days. To the reaction solution were added 20 ml of dichloromethane and 20ml of a saturated aqueous sodium bicarbonate, the mixture was shaken, and the dichloromethane layer was separated. The aqueous layer was extracted once again with 20 ml of dichloromethane, and the organic layers were combined, and washed with 20 ml of a saturated aqueous sodium bicarbonate, 20 ml of water and then 20 ml of saturated brine. After drying over anhydrous magnesium sulfate, the mixture was concentrated under reduced pressure, the residue was subjected to silica gel column chromatography (50 g, n-hexane-ethyl acetate 4 : 1, V/V) to obtain 0.16 g of the title compound (yield: 35%).
$^1$H-NMR ($CDCl_3$) δ: 2.42-2.57 (m, 4H), 2.77-2.87 (m, 2H), 3.41-3.56 (m, 3H), 3.80 (s, 3H), 4.55 (d, 1H, J=10.8Hz), 7.12-7.31 (m, 12H).
Elemental analysis: $C_{26}H_{25}Cl_2NO_2$
Calculated: C, 68.72; H, 5.55; N, 3.08.
Found: C, 68.40; H, 5.53; N, 3.01.
IR (KBr): 1720, 1494, 1468, 1422, 999, 840, 747, 704, 547 $cm^{-1}$.

Example 174

3-Benzhydryl-1-(3, 5-dibromo-2-methoxybenzyl)-4-piperidinone

A 5 ml round-bottomed flask was charged with 200 mg (0.68 mmol) of 3, 5-dibromo-2-methoxybenzyl alcohol, 204 mg (0.68 mmol) of 3-benzhydryl-4-piperidinone hydrochloride, 498 mg (3.85 mmol) of diisopropylethylamine and 2 ml of dichloromethane, and 203 mg (1.01 mmol) of EPPA was added thereto with stirring and the mixture was stirred at room temperature for 2 days. To the reaction mixture were added 20 ml of dichloromethane and 20 ml of saturated aqueous sodium bicarbonate, the mixture was shaken, and the dichloromethane layer was separated. The aqueous layer was extracted once again with 20 ml of dichloromethane, and then the organic layers were combined and washed successively with 20 ml of saturated aqueous sodium bicarbonate, 20 ml of water and then 20 ml of saturated brine. After drying over anhydrous magnesium sulfate, the mixture was concentrated under reduced pressure, the residue was subjected to silica gel column chromatography (50 g, n-hexane-ethyl acetate 4 : 1, V/V) to obtain 0.18 g of the title compound (yield: 37%).
Elemental analysis: $C_{26}H_{25}Br_2NO_2 \cdot 0.2H_2O$
Calculated: C, 57.10; H, 4.68; N, 2.56.
Found: C, 56.98; H, 4.58; N, 2.64.
IR (KBr): 2937, 1720, 1599, 1494, 1463, 1418, 1345, 1154, 998, 746, 704, 546 $cm^{-1}$.

Example 175

3-Benzhydryl-1-(5-isopropyl-2-methoxybenzyl)-4-piperidinone

(Step 1) A 35ml round-bottomed flask was charged with 2.0 g (11.2 mmol) of 2-methoxy-5-isopropylbenzaldehyde and 15 ml of ethanol, and 849 mg (22.4 mmol) of sodium borohydride was added thereto with stirring under ice-cooling. The mixture was stirred under ice-cooling for about 1 hour and then at room temperature for 3 hours. To the reaction mixture were added 20 ml of ethyl acetate and 20 ml of water, the mixture was shaken and the organic layer was separated. The aqueous layer was extracted once again with 20 ml of ethyl acetate, and the organic layers were combined and washed twice with 20 ml of water and twice with 20 ml of brine. The organic layer was dried over $MgSO_4$ (anhydrous) and concentrated under reduced pressure to obtain 1.70 g of 2-methoxy-5-isopropylbenzyl alcohol (yield: 84%).
$^1$H-NMR ($CDCl_3$) δ: 1.22 (s, 3H), 1.23 (s, 3H), 2.83-2.90 (m, 1H), 3.84 (s, 3H), 4.68 (s, 2H), 6.82 (d, 1H, J=8.0Hz), 7.11-7.14 (m, 2H).

(Step 2) A 25 ml round-bottomed flask was charged 800 mg (4.44 mmol) of 2-methoxy-5-isopropylbenzyl alcohol, 1.34 g (4.44 mmol) of 3-benzhydryl-4-piperidinone hydrochloride, 3.27 g (25.3 mmol) of diisopropylethylamine and 4 ml of dichloromethane, 1.33 g (6.66 mmol) of EPPA was added thereto with stirring and the mixture was stirred at room temperature for 2 days. To the reaction mixture were added 20 ml of dichloromethane and 20 ml of a saturated

aqueous sodium bicarbonate, the mixture was shaken, and the dichloromethane layer was separated. The aqueous layer was extracted once again with 20 ml of dichloromethane, and the organic layers were combined and washed successively with 20 ml of a saturated aqueous sodium bicarbonate, 20 ml of water and 20 ml of saturated brine. After drying over anhydrous magnesium sulfate, the mixture was concentrated under reduced pressure, the residue was subjected to silica gel column chromatography (50 g, n-hexane-ethyl acetate 5 : 1, V/V) to obtain 0.36 g of the title compound (yield: 19%).
$^1$H-NMR ($CDCl_3$) δ: 1.23-1.25 (m, 6H), 2.41-2.52 (m, 3H), 2.64-2.68 (m, 1H), 2.77-2.90 (m, 3H), 3.41-3.42 (m, 1H), 3.56 (s, 2H), 3.74 (s, 3H), 4.59 (d, 1H, J=10.8Hz), 6.79 (d, 1H, J=8.0Hz), 7.09-7.31 (m, 12H).
Elemental analysis: $C_{29}H_{33}NO_2$
Calculated: C, 81.46; H, 7.78; N, 3.28.
Found: C, 81.43; H, 7.80; N, 3.22.
IR (KBr): 3061, 3028, 3002, 2959, 2928, 2869, 2835, 2799, 1717, 1599, 1503, 1452, 1424, 1383, 1363, 1344, 1289, 1252, 1183, 1130, 1089, 1068, 1033, 996, 867, 814, 782, 746, 704, 548 $cm^{-1}$.

Example 176

3-Benzhydryl-1-(2-methoxy-5-nitrobenzyl)-4-piperidinone

A mixture of 9.1 g (30 mmol) of 3-benzhydryl-4-piperidinone hydrochloride, 7.38 g (30.0 mmol) of 2-methoxy-5-nitrobenzyl bromide and 5 g (60 mmol) of sodium hydrogen carbonate in 40 ml of dimethylformamide (DMF) was stirred for 30 hours at room temperature. To the reaction mixture were added 250 ml of ethyl acetate, 400 ml of water and 50 ml of hexane, the mixture was shaken, and the organic layer was separated, and washed with water. The organic layer was dried over $MgSO_4$ (anhydrous) and concentrated under reduced pressure. Ethanol was added to the residue and the precipitated crystals were collected by filtration and dried to obtain 12.0 g of the title compound as crystals (yield:93%).
mp 119-120°C.
$^1$H-NMR ($CDCl_3$) δ: 2.42-4.50 (m, 2H), 2.54-2.67 (m, 2H), 2.78-2.82 (m, 2H), 3.44-3.50 (m, 1H), 3.56 (s, 2H), 3.87 (s, 3H), 4.56 (d, 1H, J=11.2Hz), 6.87 (d, 1H, J=8.8Hz), 7.05-7.32 (m, 10H), 8. 16 (dd, 1H, J=3.2Hz, J=2.8Hz), 8.33 (d, 1H).
Elemental analysis: $C_{26}H_{26}N_2O_4$
Calculated: C, 72.54; H, 6.09; N, 6.51.
Found: C, 72.37; H, 5.92; N, 6.41.

Example 177

1-(5-Amino-2-methoxybenzyl)-3-benzhydryl-4-piperidinone

To a solution of 1.83 g (4.26 mmol) of 3-benzhydryl-1-(2-methoxy-5-nitrobenzyl)-4-piperidinone in 10 ml of THF, was added a suspension of 5%Pd-C 520 mg (dried material)/methanol 15 ml. The mixture was stirred under atmospheric pressure in a hydrogen stream. After the catalyst was filtered off, 0.71 ml of conc. hydrochloric acid, 40 ml of toluene and 50 ml of ethanol were added and the mixture was concentrated under reduced pressure. To the residue was added 50 ml of an ethanol/ethyl acetate (1/4) solvent mixture, and the crystals were collected by filtration, washed with the same solvent and dried to obtain 1.76 g of the hydrochloride of the title compound as colorless crystals (yield: 88%).
mp 198-200°C.
$^1$H-NMR ($CDCl_3$) δ: 2.40-4.60 (m, 13H) , 7.06-7.56 (m, 13H), 10. 30 (br, 2H).
Elemental analysis: $C_{26}H_{28}N_2O_4$•2HCl•0.5$H_2O$
Calculated: C, 64.73; H, 6.48; N, 5.81.
Found: C, 65.01; H, 6.54; N, 5.64.

Example 178

N-[3-[(3-Benzhydryl-4-oxo-1-piperidinyl)methyl]-4-methoxyphenyl]-N-(methylsulfonyl)methanesulfonamide

In 5 ml of dichloromethane, 284 mg (0.6 mmol) of 1-(5-amino-2-methoxybenzyl)-3-benzhydryl-4-piperidinone•2HCl salt was stirred with ice-cooling, and 404 mg (4 mmol) of triethylamine was added thereto. After dissolution, 276 mg (2.4 mmol) of methanesulfonyl chloride was added and the mixture was stirred for 2 hours. To the reaction mixture were added 40 ml of isopropyl ether, 40 ml of ethyl acetate and 30 ml of water, the mixture was shaken, and the organic layer was separated, dried over anhydrous magnesium sulfate and concentrated under reduced pressure,

and then the residue was subjected to silica gel column chromatography (40 g, n-hexane-ethyl acetate 2 : 1-1 : 1, V/V) to obtain 220 mg of the title compound as colorless crystals (yield: 66%).
mp 132-135°C.
$^1$H-NMR ($CDCl_3$) δ :2.38-2.53 (m, 3H), 2.71-2.78 (m, 3H), 3.40-3.48 (m, 7H), 2.56 (s, 2H), 3.79 (s, 3H), 4.58 (d, 1H), 6.89 (d, 1H), 7.13-7.31 (m, 11H), 7.39 (d, 1H).
Elemental analysis: $C_{28}H_{32}N_2O_6S_2$
Calculated: C, 60.41; H, 5.79; N, 5.03.
Found: C, 60.13; H, 5.67; N, 4.97.

Example 179

N-[3-[(3-Benzhydryl-4-oxo-1-piperidinyl)methyl]-4-methoxyphenyl]acetamide

In 2 ml of dimethylformamide, 437 mg (1.0 mmol) of 1-(5-amino-2-methoxybenzyl)-3-benzhydryl-4-piperidinone·2HCl salt was stirred, and 0.5 ml of pyridine and 0.3 ml of acetic anhydride were added thereto at room temperature. After stirring for 0.5 hours, to the reaction mixture were added 60 ml of ethyl acetate and 30 ml of water, the mixture was shaken, and the organic layer was separated. The organic layer was washed with an aqueous sodium bicarbonate, dried over anhydrous magnesium sulfate and then concentrated under reduced pressure. The residue was washed with isopropyl ether and dried to obtain 280 mg of the title compound as a pale yellow solid (yield: 63%).
$^1$H-NMR ($CDCl_3$) δ: 2.19 (s, 3H), 2.37-2.43 (m, 1H), 2.49-2.55 (m, 2H), 2.62-2.66 (m, 1H), 2.72-2.78 (m, 1H), 2.82-2.88 (m, 1H), 3.38-3.43 (m, 1H), 3.74 (s, 3H), 4.62 (d, 1H), 6.79 (d, 1H), 7.10(br, 1H), 7.11-7.42 (m, 12H).

Example 180

3-Benzhydryl-1-[5-(2,5-dimethyl-1H-pyrrol-1-yl)-2-methoxybenzyl]-4-piperidinone

A mixture of 310 mg (0.66 mmol) of 1-(5-amino-2-methoxybenzyl)-3-benzhydryl-4-piperidinone•2HCl salt, 103 mg (0.90 mmol) of acetonylacetone, 107 mg (0.90 mmol) of sodium acetate and 3 ml of acetic acid was stirred at room temperature for 1 hour. The reaction mixture was poured into an excessive amount of an aqueous sodium hydrogen carbonate and the mixture was extracted with ethyl acetate. After drying over anhydrous magnesium sulfate, the mixture was concentrated under reduced pressure, and the residue was subjected to silica gel column chromatography (25 g, n-hexane-ethyl acetate 2 : 1, V/V) to obtain 197 mg of the title compound as a colorless solid (yield: 63%).
$^1$H-NMR ($CDCl_3$) δ: 2.01 (s, 3H), 2.37-2.43 (m, 1H), 2.46-2.55 (m, 2H), 2.63-2.68 (m, 1H), 2.70-2.77 (m, 1H), 2.81-2.85 (m, 1H), 3.39-3.44 (m, 1H), 3.60 (s, 2H), 3.83 (s, 3H), 4.58 (d, 1H), 5.90 (s, 2H), 6.91 (d, 1H), 7.07-7.30 (m, 12H).
Elemental analysis: $C_{32}H_{34}N_2O_2$•$0.1H_2O$
Calculated: C, 80.00; H, 7.18; N, 5.83.
Found: C, 79.76; H, 7.33; N, 5.58.

Example 181

3-[Bis(4-fluorophenyl)methyl]-1-(5-isopropyl-2-methoxybenzyl)-4-piperidinone

A 25 ml round-bottomed flask was charged with 1.5 g (4.44 mmol) of 3-[bis(4-fluorophenyl)methyl]-4-piperidinone hydrochloride, 0.80 g (4.44 mmol) of 5-isopropyl-2-methoxybenzyl alcohol, 3.27 g (25.3 mmol) of diisopropylethylamine, 6 ml of dichloromethane and 1.33 g (6.66 mmol) of EPPA, the mixture was stirred at room temperature for about 2 days. To the reaction mixture were added 20 ml of dichloromethane and 20 ml of saturated brine, the mixture was shaken and the dichloromethane layer was separated. The aqueous layer was extracted once again with 20 ml of dichloromethane, and the organic layers were combined and washed with 20 ml of a saturated aqueous sodium hydrogen carbonate, 20 ml of water and 20 ml of saturated brine. After drying over anhydrous magnesium sulfate, the mixture was concentrated under reduced pressure, the residue was purified by silica gel column chromatography (50 g, n-hexane-ethyl acetate 6 : 1, V/V). The resultant compound was treated with hydrochloric acid to obtain 1.24 g of the hydrochloride of the title compound (yield: 56%).
IR (KBr): 2962, 2369, 1730, 1604, 1509, 1462, 1260, 1224, 1159, 1027, 823, 579, 554$cm^{-1}$.

Example 182

3-[Bis(4-fluorophenyl)methyl]-1-(5-bromo-2-ethoxybenzyl)-4-piperidinone

(Step 1) A 100ml round-bottomed flask was charged with 5.0 g (21.8 mmol) of 5-bromo-2-ethoxybenzaldehyde, 25 ml of ethanol and 25 ml of THF, and 825 mg (21.8 mmol) of sodium borohydride was added thereto with stirring under ice-cooling. The mixture was stirred under ice-cooling for about 1 hour and then at room temperature for 3 hours. The reaction solution was concentrated under reduced pressure, and to the residue were added 25 ml of ethyl acetate and 25 ml of water, the mixture was shaken, and then the organic layer was separated. The aqueous layer was extracted once again with 25 ml of ethyl acetate, and then the organic layers were combined, and then washed with 25 ml of water and 25 ml of saturated brine. The organic layer was dried over $MgSO_4$ (anhydrous) and concentrated under reduced pressure to obtain 2.3 g of 5-bromo-2-ethoxybenzyl alcohol (yield: 46%).
$^1$H-NMR ($CDCl_3$) δ: 1.43(t, J=6.8Hz, 3H), 1.23 (s, 3H), 4.06(q, 2H), 4.65 (d, J=6.8Hz, 2H), 6.73 (d, J=8.8Hz, H), 7.34 (dd, J=2.4Hz, J=8.8Hz), 7.41 (d, J=2.8Hz, 1H).

(Step 2) A 25 ml round-bottomed flask was charged with 1.0 g (2.96 mmol) of 5-bromo-2-ethoxybenzyl alcohol, 752.6 mg (3.26 mmol) of 3-[bis(4-fluorophenyl)methyl]-4-piperidinone hydrochloride, 2.18 g (16.9 mmol) of diisopropylethylamine and 6 ml of dichloromethane, and 889 mg (4.44 mmol) of EPPA dissolved in 4 ml of dichloromethane was added with stirring, and the mixture was stirred at room temperature for 6 days. To the reaction mixture were added 20 ml of dichloromethane and 20 ml of a saturated aqueous sodium bicarbonate, the mixture was shaken, and the dichloromethane layer was separated. The aqueous layer was extracted once again with 20 ml of dichloromethane, and then the organic layers were combined and washed successively with 20 ml of a saturated aqueous sodium bicarbonate, 20 ml of water and 20 ml of saturated brine. After drying over anhydrous magnesium sulfate, the mixture was concentrated under reduced pressure, and the residue was purified by silica gel column chromatography (50 g, n-hexane-ethyl acetate 4 : 1, V/V) to obtain the title compound.
$^1$H-NMR ($CDCl_3$) δ: 1.19-1.35 (m, 3H), 2.40-2.44 (m, 2H), 2.50-2.58 (m, 2H), 2.80-2.88 (m, 2H), 3.30 (m, 1H), 3.45-3.52 (m, 3H), 3.90-3.96(q, J=6.8Hz, 2H), 4.55 (d, 1H, J=11.2Hz), 6.68 (d, 1H, J=8.8Hz), 6.87-6.96 (m, 4H), 7.06-7.10 (m, 2H), 7.23-7.26 (m, 2H), 7.32(dd, 1H, J=2.4Hz, J=8.8Hz), 7.48 (d, 1H, J=2.4Hz).

After treatment with hydrochloric acid, 0.37 g of the hydrochloride of the title compound was obtained (yield: 23%).
Elemental analysis: $C_{27}H_{26}BrF_2NO_2$•HCl
Calculated: C, 58.87; H, 4.94; N, 2.54.
Found: C, 58.73; H, 5.01; N, 2.45.
IR (KBr): 2981, 2444, 2389, 1733, 1605, 1508, 1474, 1454, 1418, 1398, 1341, 1315, 1280, 1262, 1226, 1189, 1161, 1140, 1088, 1044, 1015, 990, 966, 862, 826, 812, 782, 734, 644, 580, 559, 539, 507, 480 $cm^{-1}$.

Example 183

N-[3-[(3-Benzhydryl-4-oxo-1-piperidinyl)methyl]-4-methoxyphenyl] methanesulfonamide

In 5 ml of pyridine, 473 mg (1.0 mmol) of 1-(5-amino-2-methoxybenzyl)-3-benzhydryl-4-piperidinone•2HCl salt was stirred at room temperature, 150 mg (1.3 mmol) of methanesulfonyl chloride was added thereto and the mixture was stirred for 1 hour. To the reaction mixture was added 0.2 ml of water, the mixture was concentrated under reduced pressure, and 40 ml of ethyl acetate and 30 ml of an aqueous sodium hydrogen carbonate were added to the residue. The mixture was shaken, the organic layer was separated, dried over anhydrous magnesium sulfate and concentrated under reduced pressure, and the residue was subjected to silica gel column chromatography (25 g, n-hexane-ethyl acetate 1 : 1, V/V) to obtain 408 mg of the title compound as a colorless solid (yield: 85%).
mp 132-135°C.
$^1$H-NMR ($CDCl_3$) δ: 2.36-2.43 (m, 3H), 2.54-2.63 (m, 3H), 2.71-2.77 (m, 1H), 2.85-2.91 (m, 1H), 2.94 (s, 3H), 3.39-3.44 (m, 1H), 3.51 (d, 1H), 3.57 (d, 1H), 3.77 (s, 3H), 4.64 (d, 1H), 6.20(br, 1H), 6.81 (d, 1H), 7.11-7.31 (m, 12H).
Elemental analysis: $C_{27}H_{30}N_2O_4S$•$0.2H_2O$
Calculated: C, 67.25; H, 6.35; N, 5.81.
Found: C, 67.20; H, 6.26; N, 5.53.

Example 184

3-Benzhydryl-1-(2-hydroxy-5-nitrobenzyl)-4-piperidinone

In 15 ml of dimethylformamide (DMF), 3.02 g (10 mmol) of 3-benzhydryl-4-piperidinone hydrochloride, 1.88

g (10 mmol) of 2-hydroxy-5-nitrobenzyl bromide and 3.36 g (40 mmol) of sodium hydrogen carbonate were stirred at room temperature for 15 hours. To the reaction mixture were added 150 ml of ethyl acetate and 150 ml of water, the mixture was shaken and the organic layer was separated, and washed with water. The organic layer was dried over $MgSO_4$ (anhydrous) and concentrated under reduced pressure, and 10 ml of ethanol was added to the residue. The precipitated crystals were collected by filtration and dried to obtain 2.95 g of the title compound as crystals (yield: 71%).
mp 177-178°C.
$^1$H-NMR ($CDCl_3$) δ: 2.49-4.65 (m, 3H), 2.80-2.84 (m, 1H), 2.91(br, 2H), 3.51-3.56 (m, 1H), 3.78 (s, 2H), 4.41 (d, 1H), 6.95 (d, 1H), 7.11-7.31 (m, 10H), 7.88 (d, 1H), 8.12(dd, 1H).
Elemental analysis: $C_{25}H_{24}N_2O_4$
Calculated: C, 72.10; H, 5.81; N, 6.73.
Found: C, 72.03; H, 5.75; N, 6.46.

Example 185

3-Benzhydryl-1-(2-ethoxy-5-nitrobenzyl)-4-piperidinone

In 15 ml of dimethylformamide (DMF), 852 mg (2 mmol) of 3-benzhydryl-1-(2-hydroxy-5-nitrobenzyl)-4-piperidinone and 552 mg (4 mmol) of anhydrous potassium carbonate were stirred at room temperature for 15 minutes, 377 mg (2.4 mmol) of iodoethane was added thereto, and the mixture was stirred at room temperature further for 6 hours. Ethyl acetate and water were added to the reaction mixture, the mixture was shaken and the organic layer was separated, and washed with water. The organic layer was dried over $MgSO_4$ (anhydrous), concentrated under reduced pressure and dried to obtain 875 mg of the title compound as a pale yellow solid (quantitative).
$^1$H-NMR ($CDCl_3$) δ: 1.40(t, 3H), 2.43-4.50 (m, 2H), 2.54-2.61 (m, 1H), 2.64-2.68 (m, 1H), 2.81-2.92 (m, 2H), 3.45-3.50 (m, 1H), 3.55 (d, 1H), 3.59 (d, 1H), 4.07(q, 2H), 4.56 (d, 1H), 6.83 (d, 1H), 7.05-7.32 (m, 10H), 7.88 (d, 1H), 8.13(dd, 1H), 8.33 (d, 1H).
Elemental analysis: $C_{27}H_{28}N_2O_4$
Calculated: C, 72.95; H, 6.35; N, 6.30.
Found: C, 72.91; H, 6.36; N, 6.03.

Example 186

N-[3-[(3-Benzhydryl-4-oxo-1-piperidinyl)methyl]-4-methoxyphenyl]-N-(2, 2, 2-trifluoroethyl)methanesulfonamide

To a solution of 440 mg (0.92 mmol) of N-[3-[(3-benzhydryl-4-oxo-1-piperidinyl)methyl]-4-methoxyphenyl] methanesulfonamide in 3 ml of dimethylformamide (DMF) were added 280 mg (2.03 mmol) of anhydrous potassium carbonate and 305 mg (1.2 mmol) of 2,2,2-trifluoroethyl p-toluenesulfonate, and the mixture was stirred in a water bath at 125°C for 4 hours. Ethyl acetate and water were added to the reaction solution, the mixture was shaken and the organic layer was separated, and washed with water. The organic layer was dried over $MgSO_4$ (anhydrous) and concentrated under reduced pressure, and then the residue was subjected to silica gel column chromatography (50 g, n-hexane-ethyl acetate 1 : 1, V/V) to obtain 180 mg of the title compound as a pale yellow solid (yield: 32%).
$^1$H-NMR ($CDCl_3$) δ: 2.38-2.53 (m, 3H), 2.63-2.79 (m, 3H), 3.00 (s, 3H), 3.41-3.46 (m, 1H), 3.52 (d, 1H), 3.58 (d, 1H), 3.80 (s, 3H), 4.20 (d, 1H), 4.25 (d, 1H), 4.58 (d, 1H), 6.87 (d, 1H), 7.11-7.31 (m, 11H), 7.38 (d, 1H).
Elemental analysis: $C_{29}H_{31}F_3N_2O_4S$
Calculated: C, 62.63; H, 5.62; N, 4.95.
Found: C, 62.53; H, 5.71; N, 5.01.

Example 187

[3-[(3-Benzhydryl-4-oxo-1-piperidinyl)methyl]-4-methoxyphenyl]acetic acid

(Step 1) According to a conventional method, 3-formyl-4-methoxyphenylacetic acid methyl ester was obtained from 2.0 g (10.3 mmol) of 3-formyl-4-hydroxyphenylacetoc acid methyl ester, 1.57 g (11.3 mmol) of potassium carbonate, methyl iodide and 5 ml of DMF.
$^1$H-NMR ($CDCl_3$) δ: 3.64 (s, 2H), 3.69 (s, 3H), 3.93 (s, 3H), 6.97 (d, 1H, J=8.8Hz), 7.49(dd, 1H, J=8.4Hz, J=2.4Hz), 7.72 (d, 1H, J=2.0Hz).
Without purification, the resultant compound was reduced with 778 mg (20.6 mmol) of sodium borohydride to obtain methyl 3-hydroxymethyl4-methoxyphenylacetate.
$^1$H-NMR ($CDCl_3$) δ: 3.57 (s, 2H), 3.68 (s, 3H), 3.85 (s, 3H), 4.67 (d, 2H, J=6.0Hz), 6.84 (d, 1H, J=8.4Hz), 7.17-7.20

(m, 2H).

(Step 2) To 574.4 mg (1.90 mmol) of 3-benzhydryl-4-piperidinone hydrochloride was added 5 ml of dichloromethane and 1.40 g (10.8 mmol) of diisopropylethylamine was added thereto with stirring at room temperature. Then 400 mg (1.90 mmol) of methyl 3-hydroxymethyl-4-methoxyphenylacetate and 571.2 mg (2.85 mmol) of EPPA were added and the mixture was stirred for 48 hours. To the reaction mixture were added 20 ml of dichloromethane and 20 ml of a saturated aqueous sodium hydrogen carbonate, the mixture was shaken and the organic layer was separated, and dried over anhydrous magnesium sulfate. After concentrating under reduced pressure, the residue was purified by silica gel column chromatography (50 g, n-hexane-ethyl acetate 4 : 1, V/V) to obtain methyl [3-[(3-benzhydryl-4-oxo-1-piperidinyl)methyl]-4-methoxyphenyl]acetate.

$^1$H-NMR ($CDCl_3$) $\delta$: 2.36-2.52 (m, 3H), 2.63-2.87 (m, 3H), 3.37-3.43 (m, 1H), 3.51-3.58 (m, 4H), 3.69 (s, 3H), 3.74 (s, 3H), 4.59 (d, 1H, J=11.2Hz), 6.80 (d, 1H, J=8.4Hz), 7.09-7.32 (m, 12H).

The resultant compound was treated with hydrochloric acid to obtain 310 mg of the hydrochloride (yield: 36%).

Elemental analysis: $C_{29}H_{31}NO_4$•HCl•1.6$H_2O$

Calculated: C, 66.62; H, 6.79; N, 2.68.

Found: C, 66.34; H, 6.90; N, 2.48.

IR (KBr): 2952, 1737, 1717, 1503, 1452, 1254, 1154, 1032, 748, 705, 548cm$^{-1}$.

(Step 3) A solution of 150 mg (0.33 mmol) of [3-[(3-benzhydryl-4-oxo-1-piperidinyl)methyl]-4-methoxyphenyl] acetic acid methyl ester in 1.5 ml of methanol was added 0.33 ml (0.66 mmol) of 2 N NaOH with stirring. After stirring at room temperature for 2 hours, the reaction mixture was concentrated under reduced pressure, 20 ml of ethyl acetate and 20 ml of water were added to the residue, the mixture was adjusted to pH 2-3 with 1 N HCl and then shaken, and then the organic layer was separated and washed with water. The organic layer was dried over $MgSO_4$ (anhydrous) and concentrated under reduced pressure to obtain 73.1 mg of the title compound (yield: 50%).

$^1$H-NMR ($CDCl_3$) $\delta$: 2.40-2.49 (m, 2H), 2.68-3.10 (m, 4H), 3.60 (s, 2H), 3.67 (s, 3H), 3.72 (m, 3H), 4.48 (d, 1H, J=10.4Hz), 5.35(br, 1H), 6.76 (d, 1H, J=8.4Hz), 7.08-7.32 (m, 13H).

Elemental analysis: $C_{28}H_{29}NO_4$•$H_2O$•0.5AcOEt

Calculated: C, 71.27; H, 6.98; N, 2.77.

Found: C, 71.01; H, 6.85; N, 2.61.

IR (KBr): 2934, 1718, 1598, 1506, 1453, 1258, 1136, 1032, 912, 735, 706, 548 cm$^{-1}$.

Example 188

3-Benzhydryl-1-[2-methoxy-5-(1H-pyrrol-1-yl)benzyl]-4-piperidinone

To 473 mg (1 mmol) of 1-(5-amino-2-methoxybenzyl)-3-benzhydryl-4-piperidinone•2HCl salt was added 5 ml of acetic acid, and 164 mg (2 mmol) of sodium acetate was added thereto with stirring at room temperature. After 15 minutes, 158 mg (1.2 mmol) of 2, 5-dimethoxytetrahydrofuran was added and the mixture was stirred at room temperature for 55 hours. To the reaction mixture were added 5.5 g of anhydrous sodium carbonate, 80 ml of water and 100 ml of ethyl acetate with vigorously stirring. The organic layer was separated, and washed with water. The organic layer was dried over $MgSO_4$ (anhydrous) and concentrated under reduced pressure, and then the. residue was subjected to silica gel column chromatography (50 g, n-hexane-ethyl acetate 2 : 1, V/V) to obtain 176 mg of the title compound as a colorless solid (yield: 39%).

$^1$H-NMR ($CDCl_3$) $\delta$: 2.39-2.55 (m, 3H), 2.65-2.69 (m, 1H), 2.78-2.89 (m, 2H), 3.37-3.51 (m, 1H), 3.58 (s, 2H), 3.79 (s, 3H), 4.57 (d, 1H), 6.36(t, 2H), 6.87 (d, 1H), 7.02(t, 2H), 7.08-7.16 (m, 6H), 7.21-7.30 (m, 5H), 7.38 (d, 1H).

Elemental analysis: $C_{30}H_{30}N_2O_2$•0.3$H_2O$

Calculated: C, 79.02; H, 6.76; N, 6.14.

Found: C, 78.93; H, 6.83; N, 5.95.

Example 189

N-[3-[(3-Benzhydryl-4-oxo-1-piperidinyl)methyl]-4-methoxyphenyl]-2, 2, 2-trifluoroacetamide

To 473 mg (1 mmol) of 1-(5-amino-2-methoxybenzyl)-3-benzhydryl-4-piperidinone•2HCl salt was added 5 ml of pyridine, and 300 mg (1.43 mmol) of trifluoroacetic anhydride was added thereto with stirring at room temperature. After stirring for 0.5 hour at room temperature, 50 ml of water, 35 ml of ethyl acetate and 35 ml of hexane were added to the reaction mixture. The mixture was shaken and the organic layer was separated, and washed with water. The organic layer was dried over $MgSO_4$ (anhydrous) and concentrated under reduced pressure to obtain 467 mg of the title compound as a pale yellow solid (yield: 97%).

$^1$H-NMR ($CDCl_3$) $\delta$: 2.40-2.57 (m, 3H), 2.63-2.67 (m, 1H), 2.76-2.87 (m, 2H), 3.41-3.46 (m, 1H), 3.54 (s, 2H), 3.77 (s,

3H), 4.59 (d, 1H), 6.83(t, 2H), 7.09 (m, 10H), 7.44(dd, 1H), 7.50 (d, 1H), 7.79(br, 1H).
Elemental analysis: $C_{28}H_{27}F_3N_2O_3$•0.2$H_2O$
Calculated: C, 67.24; H, 5.52; N, 5.60.
Found: C, 66.94; H, 5.52; N, 5.40.

Example 190

3-Benzhydryl-1-[5-nitro-2-(2,2,2-trifluoroethoxy)benzyl]-4-piperidinone

To 4 ml of dimethylformamide (DMF) were added 624 mg (1.5 mmol) of 3-benzhydryl-1-(2-hydroxy-5-nitrobenzyl)-4-piperidinone, 345 mg (4 mmol) of anhydrous potassium carbonate and 457 mg (1.8 mmol) of 2, 2, 2-trifluoroethyl p-toluenesulfonate, and the mixture was stirred in a water bath at 120°C for 9 hours and then at 115°C for 15 hours. Ethyl acetate and water were added the reaction mixture, the mixture was shaken and the organic layer was separated, and washed with water. The organic layer was dried over $MgSO_4$ (anhydrous) and concentrated under reduced pressure, and then the residue was subjected to silica gel column chromatography (50 g, n-hexane-ethyl acetate 2 : 1, V/V) to obtain 275 mg of the title compound as a pale yellow solid (yield: 37%).
$^1$H-NMR ($CDCl_3$) δ: 2.38-2.53 (m, 2H), 2.56-2.64 (m, 2H), 2.82-2.95 (m, 2H), 3.42-3.49 (m, 1H), 3.53 (d, 1H), 3.64 (d, 1H), 4.38 (d, 1H), 4.53 (d, 1H), 6.84 (d, 1H), 7.03-7.30 (m, 10H), 7.88 (d, 1H), 8.16(dd, 1H), 8.34 (d, 1H).
Elemental analysis: $C_{27}H_{25}F_3N_2O_4$•0.3AcOEt
Calculated: C, 64.52; H, 5.26; N, 5.34.
Found: C, 64.34; H, 4.97; N, 5.04.

Example 191

N-[3-[(3-Benzhydryl-4-oxo-1-piperidinyl)methyl]-4-methoxyphenyl]ethanesulfonamide

A mixture of 473 mg (1.0 mmol) of 1-(5-amino-2-methoxybenzyl)-3-benzhydryl-4-piperidinone·2HCl salt and 5 ml of pyridine was stirred at room temperature, 168 mg (1.3 mmol) of ethanesulfonyl chloride was added thereto and the mixture was stirred for 1 hour. To the reaction mixture was added 0.5 ml of water, the mixture was concentrated under reduced pressure and then ethyl acetate and water were added to the residue. The mixture was shaken and then the organic layer was separated, dried over anhydrous magnesium sulfate and concentrated under reduced pressure to obtain 418 mg of the title compound as a pale yellow solid (yield: 85%).
$^1$H-NMR ($CDCl_3$) δ: 1.39(t, 3H, J=7.2Hz), 2.36-2.42 (m, 1H), 2.53-2.64 (m, 3H), 2.69-2.75 (m, 1H), 2.85-2.81 (m, 1H), 3.05(q, 2H, J=7.2Hz), 3.40-3.44 (m, 1H), 3.53 (d, 1H, J=14Hz), 3.56 (d, 1H, J=14.4Hz), 3.76 (s, 3H), 4.65 (d, 1H, J=11.2Hz), 6.33(br, 1H), 6.69 (d, 1H, J=11.2Hz), 7.11-7.15 (m, 3H), 7.20-7.32 (m, 9H).
Elemental analysis: $C_{28}H_{32}N_2O_4S$•0.1$H_2O$
Calculated: C, 68.02; H, 6.56; N, 5.67.
Found: C, 67.84; H, 6.66; N, 5.61.

Example 192

N-[3-[(3-Benzhydryl-4-oxo-1-piperidinyl)methyl]-4-methoxyphenyl]-1-propanesulfonamide

A mixture of 284 mg (0.6 mmol) of 1-(5-amino-2-methoxybenzyl)-3-benzhydryl-4-piperidinone•2HCl salt and 4 ml of pyridine was stirred at room temperature, 115 mg (0.8 mmol) of n-propanesulfonyl chloride was added thereto, and the mixture was stirred for 1 hour. To the reaction mixture was added 0.5 ml of water, the mixture was concentrated under reduced pressure, and ethyl acetate and water were added to the residue. The mixture was shaken and the organic layer was separated, dried over anhydrous magnesium sulfate and concentrated under reduced pressure to obtain 286 mg of the title compound as a pale yellow solid (yield: 94%).
$^1$H-NMR ($CDCl_3$) δ: 1.13(t, 3H), 1.83-1.92 (m, 2H), 2.34-2.41 (m, 1H), 2.53-2.64 (m, 3H), 2.69-2.75 (m, 1H), 2.85-2.91 (m, 1H), 2.98-3.02 (m, 2H), 3.38-3.43 (m, 1H), 3.52 (d, 1H), 3.56 (d, 1H), 3.78 (s, 3H), 4.65 (d, 1H), 6.26(br, 1H), 6.80 (d, 1H), 7.10-7.14 (m, 1H), 7.20-7.32 (m, 9H).
Elemental analysis: $C_{29}H_{34}N_2O_4S$•0.1$H_2O$
Calculated: C, 68.50; H, 6.78; N, 5.51.
Found: C, 67.27; H, 6.74; N, 5.41.

Example 193

3-Benzhydryl-1-(5-bromo-2-isopropyloxybenzyl)-4-piperidinone

(Step 1) According to a conventional method, 5-bromo-2-isopropoxybenzaldehyde was obtained from 2.0 g (9.95 mmol) of 5-bromosalicylaldehyde, 1.51 g (10.9 mmol) of potassium carbonate, 1.86 g (10.9 mmol) of 2-iodopropane and 5 ml of DMF.
$^1$H-NMR ($CDCl_3$) δ: 1.40-1.41 (m, 6H), 4.64-4.67 (m, 1H), 6.89 (d, 1H, J=8.8Hz), 7.61(dd, 1H, J=2.8Hz, J=9.2Hz), 7.92 (d, 1H, J=2.8Hz), 10.40 (s, 1H).
Without purification, the resultant compound was reduced with 308.9 mg (8.16 mmol) of sodium borohydride to obtain 5-bromo-2-isopropoxybenzyl alcohol.
(Step 2) To 985 mg (3.26 mmol) of 3-benzhydryl-4-piperidinone hydrochloride was added 8 ml of dichloromethane, and further 2.40 g (18.6 mmol) of diisopropylethylamine was added thereto with stirring at room temperature. Then, 800 mg (3.26 mmol) of 5-bromo-2-isopropoxybenzyl alcohol and 979.7 mg (4.90 mmol) of EPPA were added and the mixture was stirred for 72 hours. To the reaction mixture were added 20 ml of dichloromethane and 20 ml of a saturated aqueous sodium hydrogen carbonate, the mixture was shaken, and the organic layer was separated and dried over anhydrous magnesium sulfate. After concentrating under reduced pressure, the residue was purified by silica gel column chromatography (50 g, n-hexane-ethyl acetate 6 : 1, V/V) to obtain the title compound.
$^1$H-NMR ($CDCl_3$) δ: 1.22-1.26 (m, 6H), 2.38-2.64 (m, 4H), 2.77-2.88 (m, 2H), 3.38-3.42 (m, 1H), 3.46 and 3.53(ABq, 2H, J=13.6Hz), 4.41-4.47 (m, 1H), 4.57 (d, 1H, J=11.2Hz), 6.69 (d, 1H, J=8.8Hz), 7.08-7.32 (m, 12H), 7.50 (d, 1H, J=2.4Hz).
The resultant compound was treated with hydrochloric acid to obtain 840 mg of the hydrochloride of the title compound (yield: 48%).
Elemental analysis: $C_{28}H_{30}BrNO_2$•HCl•0.5$H_2O$
Calculated: C, 62.52; H, 6.00; N, 2.60.
Found: C, 62.67; H, 6.12; N, 2.57.
IR (KBr): 3028, 2976, 2799, 1717, 1591, 1484, 1452, 1385, 1247, 1185, 1115, 955, 808, 747 $cm^{-1}$.

Example 194

1-Benzyl-3-[(2-bromophenyl)(phenyl)methyl]-4-piperidinone

According to the same manner as that described in Example 157, the title compound was obtained.
$^1$H-NMR ($CDCl_3$) δ: 2.17-2.96 (m, 6H), 3.27-3.35 (m, 1H), 3.43-3.60 (m, 2H), 5.28 (d, 0.5H, J=11.8Hz), 5.32 (d, 0.5H, J=11.8Hz), 6.94-7.53 (m, 14H).
The title compound was treated with conc. hydrochloric acid and ethanol to obtain the hydrochloride of the title compound.
mp 204-206°C.

Example 195

1-Benzyl-4-[bis(4-fluorophenyl)methyl]-3-piperidinone

A mixture of 9.69 g (42.9 mmol) of 1-benzyl-3-piperidinone hydrochloride•$H_2O$, 150 ml of dichloromethane and 50 ml of a saturated brine was stirred under ice-cooling, 6.08 g (44.1 mmol) of potassium carbonate was added thereto and the mixture was stirred vigorously. The precipitated crystals were filtered off, the filtrate was allowed to stand and the dichloromethane layer was separated. The aqueous layer was extracted once again with 150 ml of dichloromethane. The dichloromethane layers were combined, washed with saturated brine, dried over anhydrous sodium sulfate, and the solvent was distilled off under reduced pressure. The resultant 1-benzyl-3-piperidinone as a dark tan oil was dissolved in 50 ml of dichloromethane, the solution was stirred under ice-cooling, and 18 ml (99.4 mmol) of trimethylsilyl triflate (TMSOTf) and then 9.8 g (44.5 mmol) of 4, 4'-difluorobenzhydrol were added thereto. The mixture was stirred at room temperature for 26 hours, 100 ml of water and 20.3 g of sodium acetate were added under ice-cooling and the mixture was adjusted to pH 9 with saturated sodium hydrogen carbonate and the dichloromethane layer was separated. The dichloromethane layer was dried over anhydrous sodium sulfate, and the solvent was distilled off under reduced pressure. The residue was purified by silica gel column chromatography (150 g, n-hexane-ethyl acetate 10 : 1 - 3 : 1, V/V) to obtain 8.68 g of the title compound as an oil (yield: 51.7%).
$^1$H-NMR ($CDCl_3$) δ: 1.52-1.62 (m, 1H), 1.75-1.81 (m, 1H), 2.48-3.54 (m, 1H), 2.84-2.88 (m, 1H), 2.91 (d, 1H, J=13.2Hz), 3.16 (d, 1H, J=13.8Hz), 3.18-3.24 (m, 1H), 3.58(dd, 2H, J=13.6Hz, J=2.2Hz), 4.39 (d, 1H, J=9.6Hz), 6.94(t, 4H,

J=8.8Hz), 7.09-7.12 (m, 2H), 7.16-7.20 (m, 2H), 7.23-7.33 (m, 5H).

The title compound was dissolved in 10 m of ethanol, and 2.4 ml (4.88 mmol) of 2 N HCl was added. Then, the mixture was concentrated under reduced pressure, 4 ml of ethanol was added to the residue, and the precipitated crystals were collected by filtration to obtain the colorless hydrochloride of the title compound.
mp 177.5-179.0°C.
Elemental analysis: $C_{25}H_{23}F_2NO•HCl•0.25H_2O$ (432.417)
Calculated: C, 69.44; H, 5.71; N, 3.24.
Found: C, 69.51; H, 5.71; N, 3.16.
IR (KBr): 3442, 2922, 2469, 1732, 1603, 1509, 1439, 1226, 1160, 832, 750, 700, 577, 543.$cm^{-1}$.

Example 196

4-[Bis(4-fluorophenyl)methyl]-3-piperidinone

To a solution of 802 mg (1.87 mmol) of 1-benzyl-4-[bis(4-fluorophenyl)methyl]-3-piperidinone in 50 ml of methanol was added 805 mg of 5%Pd-C(50% hydrated), and the mixture was stirred at room temperature for 2 hours in a hydrogen stream. After filtration, the filtrate was distilled off under reduced pressure to obtain 398 mg of the title compound (yield: 62.3%). Then it was dissolved in ethanol, treated with conc. hydrochloric acid to obtain the hydrochloride of the title compound.
Elemental analysis: $C_{18}H_{17}F_2NO•HCl•0.25H_2O$
Calculated: C, 63.16; H, 5.45; N, 4.09.
Found: C, 63.38; H, 5.30; N, 4.32.

Example 197

1-Benzyl-4-(10,11-dihydro-5H-dibenzo[a,d][7]annulen-5-yl)-3-piperidinone

According to the same manner as that descried in Example 195, the hydrochloride of the title compound was obtained.
Elemental analysis: $C_{27}H_{27}NO•HCl•0.1H_2O$
Calculated: C, 77.25; H, 6.77; N, 3.34.
Found: C, 77.15; H, 6.89; N, 3.22.

Example 198

4-(10,11-Dihydro-5H-dibenzo[a,d][7]annulen-5-yl)-3-piperidinone

According to the same manner as that described in Example 196, the hydrochloride of the title compound was obtained.
mp 196-198°C (decomp).
Elemental analysis: $C_{20}H_{21}NO•HCl•0.3H_2O$
Calculated: C, 72.08; H, 6.84; N, 4.20.
Found: C, 71.87; H, 6.88; N, 4.07.

Example 199

1-Acetyl-3-benzhydryl-4-piperidinone

The title compound described in Example 5 can also be produced by the method described below.

(Step 1) To a solution of 1.4 g (0.01 mole) of 1-acetyl-4-piperidinone and 2.2 g of 2, 6-lutidine in 10 ml of dichloromethane was added 5.3 ml of triisopropylsilyl triflate under ice-cooling. After stirring at room temperature for 2 hours, the mixture was washed with water and then with diluted hydrochloric acid. After drying over magnesium sulfate, the solvent was distilled off to obtain 1-acetyl-4-piperidinonetriisopropylsilyl enolate quantitatively.

(Step 2) To a solution of 300 mg of 1-acetyl-4-piperidinonetriisopropylsilyl enolate in 5 ml of dichloromethane were added 250 mg of benzhydryl bromide and 220 mg of zinc bromide under ice-cooling. After allowing to stand overnight, an aqueous solution of sodium bicarbonate was added to the mixture and the mixture was stirred vigorously. The dichloromethane layer was dried over magnesium sulfate, the solvent was distilled off and the residue was purified by silica gel column chromatography (40 g, ethyl acetate). The residue was triturated with a small amount of ether and

collected by filtration to obtain 140 mg of the title compound (yield: 45%).
mp133-135°C

Example 200

3-Benzhydryl-1-benzyl-4-piperidinone

The title compound described in Example 4 can also be produced by the method described below. A 1 L flask was charged with 250 ml of toluene and 73.7 g (389 mmol) of 1-benzyl-4-piperidinone and to the mixture was added dropwise 85.5 ml (467 mmol) of trimethylsilyl triflate with stirring under ice-cooling (internal temperature 5 to 10°C). The cooling bath was removed and the mixture was warmed to room temperature and then stirred for 30 minutes. Then, 87.0 g (429 mmol) of benzhydryl chloride was added thereto and the mixture was stirred at an internal temperature of 78 to 80°C for 4 hours. To the mixture.were added 580 ml of ethyl acetate and a 10% aqueous solution of sodium carbonate with stirring under ice-cooling. After separating into layers, the aqueous layer was washed with 580 ml of ethyl acetate. The organic layers were combined and washed with 580 ml of water. The mixture was decolorized by treating with 7.4 g of an active charcoal SHIRASAGI A. After concentration under reduced pressure, 390 ml of ethanol and 47 ml of conc. hydrochloric acid were added to the residue, and the mixture was ice-cooled, and allowed to stand in a refrigerator overnight. The precipitated crystals were collected by filtration, washed with 200 ml of ethanol and 100 ml of IPE and dried under reduced pressure to obtain 107 g of the hydrochloride of the title compound (yield: 70%).

The compounds obtained in the above Examples are summarized in the following Tables 1 to 3.

Table 1

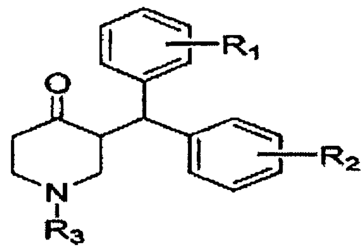

| Example No. | $R^1$ | $R^2$ | $R^3$ |
|---|---|---|---|
| 1 | H | H | Me |
| 5, 199 | H | H | Ac |
| 6 | 4-Cl | 4-Cl | Ac |
| 7 | 4-F | 4-F | Ac |
| 8 | 2-$CH_2CH_2$-2' | | Ac |
| 9 | 2-O-2' | | Ac |
| 10 | H | H | Et |
| 11 | H | H | n-Pr |
| 12 | H | H | $PhCH_2CH_2$ |
| 15 | 4-MeO | 4-MeO | Me |
| 16 | 4-Cl | 4-Cl | Me |
| 17 | 2-O-2' | | Me |
| 19 | 2-S-2' | | Me |
| 20 | 4-F | 4-F | Me |
| 23 | H | 4-Cl | Me |
| 25 | 2-$CH_2CH_2$-2' | | Me |
| 26 | 2-CH=CH-2' | | Me |
| 28 | H | H | H |
| 29 | 4-Cl | 4-Cl | H |
| 30 | 4-F | 4-F | H |

| | | | |
|---|---|---|---|
| 61 | H | H | 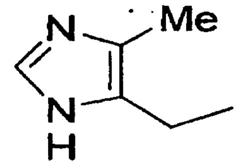 |
| 62 | H | H | 2-MeOPhCH=CH-$CH_2$- |
| 63 | H | H | 2-MeOPh$CH_2$C(=NH)- |
| 64 | 4-Cl | 4-Cl | 2-MeOPh$CH_2$C(=NH)- |
| 65 | 4-F | 4-F | 2-MeOPh$CH_2$C(=NH)- |
| 66 | H | H | 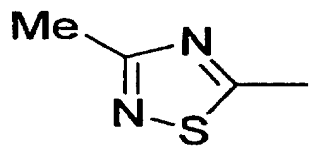 |
| 67 | H | H | PhCONHCS- |
| 68 | H | H | $H_2$NCO- |
| 69 | H | H | $H_2$NCS- |
| 70 | H | H | 2-MeOPhNHCO- |
| 71 | H | H | 2-MeOPhNHCS- |
| 72 | H | H | NC$CH_2CH_2$- |
| 73 | H | H | $NaO_3S$- |
| 74 | H | H | 3-Py-CH=C(NHAc)CO- |
| 84 | 4-F | 4-F | 2-MeOPhCO$CH_2$- |
| 87 | 4-F | 4-F | 3-Py$CH_2$- |
| 89 | 4-F | 4-F | 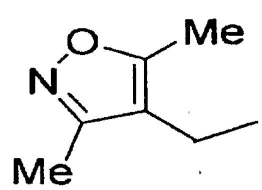 |
| 90 | 4-F | 4-F | 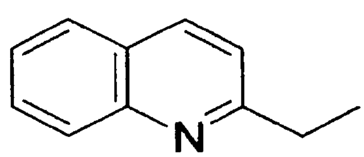 |
| 124 | 4-Me | 4-Me | Ac |

| | | | |
|---|---|---|---|
| 125 | 4-Me | 4-Me | H |
| 131 | 2-CH=CH-2' | | H |
| 133 | 2-O-2' | | H |
| 47 | H | H | 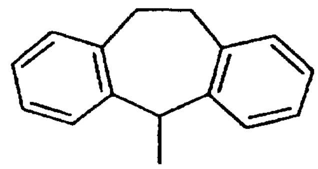 |
| 48 | H | H | 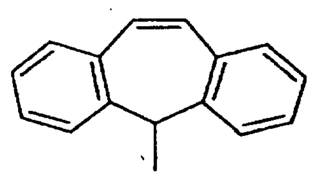 |
| 50 | H | H | 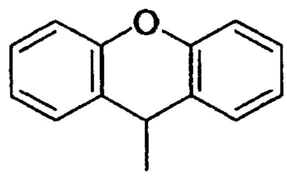 |
| 51 | H | H | 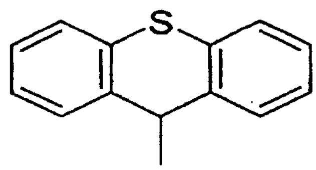 |
| 52 | H | H | $PhCH=CHCH_2-$ |
| 53 | H | H | $3-PyCH_2-$ |
| 54 | H | H | $2-PyCH_2-$ |
| 55 | H | H | 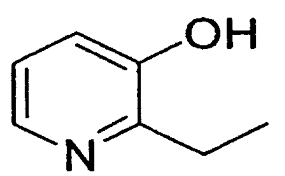 |
| 56 | H | H | 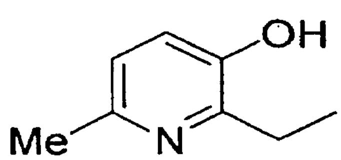 |
| 57 | H | H | $HC\equiv CCH_2-$ |

| | | | |
|---|---|---|---|
| 58 | H | H | 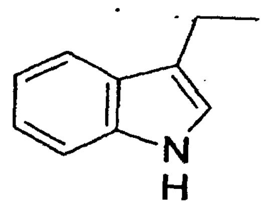 |
| 59 | H | H | 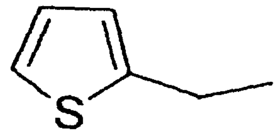 |
| 60 | H | H | 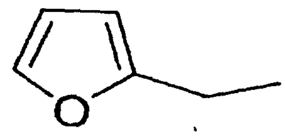 |
| 144 | H | H | 3,5-$(CF_3)_2$PhCO- |
| 145 | 4-Cl | 4-Cl | 3,5-$(CF_3)_2$PhCO- |
| 146 | 4-F | 4-F | EtOCO- |
| 147 | 2-F | H | H |
| 148 | 4-F | 4-F | 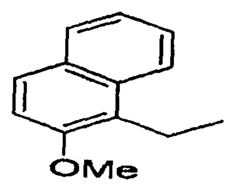 |
| 149 | 4-F | 4-F | 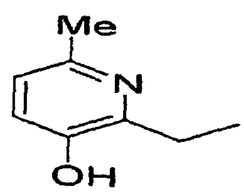 |
| 150 | 4-F | 4-F | 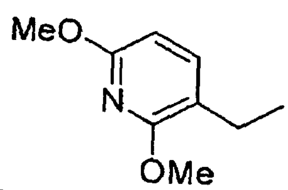 |
| 151 | H | H | 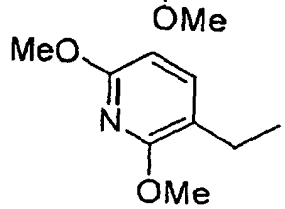 |
| 152 | H | H | 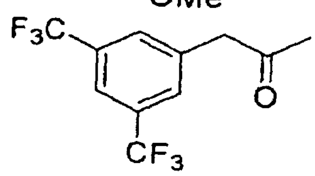 |

| | | | |
|---|---|---|---|
| 153 | H | H | 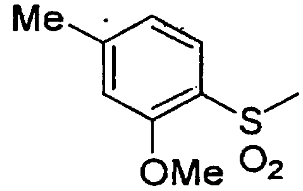 |
| 154 | H | H | 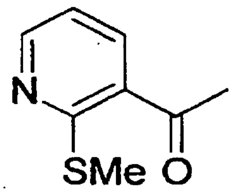 |
| 155 | H | H | 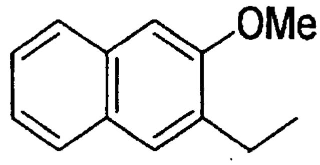 |
| 156 | H | H | 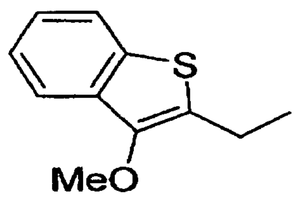 |

Table 2

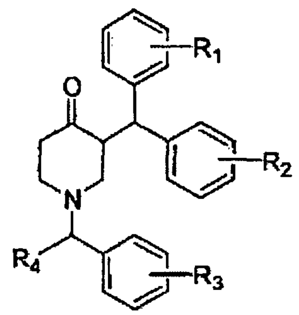

| Ex. No | $R^1$ | $R^2$ | $R^3$ | $R^4$ |
|---|---|---|---|---|
| 4, 200 | H | H | H | H |
| 13 | 2-O-2' | | 2-MeO | H |
| 14 | 2-CH=CH-2' | | 2-MeO | H |
| 18 | 2-O-2' | | H | H |
| 21 | 4-F | 4-F | H | H |
| 22 | 4-Cl | 4-Cl | H | H |
| 24 | H | 4-Cl | H | H |
| 27 | 2-CH=CH-2' | | H | H |
| 32 | H | H | 2-MeO | H |
| 34 | H | H | 2-MeO | Me |
| 35 | 4-Cl | 4-Cl | 2-MeO | H |
| 36 | 4-F | 4-F | 2-MeO | H |
| 37 | H | H | 4-MeO | 4-MeOPh |
| 38 | H | H | 2-OH | H |
| 39 | H | H | 3-OH | H |
| 40 | H | H | 4-OH | H |
| 41 | H | H | 3-MeO,4-OH | H |

| | | | | |
|---|---|---|---|---|
| 42 | H | H | 3,4-$OCH_2O$- | H |
| 43 | H | H | 2,3-$(MeO)_2$ | H |
| 44 | H | H | 2,4-$(MeO)_2$ | H |
| 45 | H | H | 3,4-$(MeO)_2$ | H |
| 46 | H | H | 3,4,5-$(MeO)_3$ | H |
| 49 | H | H | 4-MeO | H |
| 75 | 4-F | 4-F | 2-OH | H |
| 76 | 4-F | 4-F | 2-MeO,5-Br | H |
| 77 | 4-F | 4-F | 3-MeO | H |
| 78 | 4-F | 4-F | 2,3,4-$(MeO)_3$ | H |
| 79 | H | 4-F | 2-MeO | H |
| 80 | 4-F | 4-F | 3,5-$(CF_3)_2$ | H |
| 81 | H | H | 3,5-$(CF_3)_2$ | H |
| 82 | 4-F | 4-F | 2-F | H |
| 83 | H | H | 2-F | H |
| 85 | 4-F | 4-F | 2-$FCH_2O$ | H |
| 86 | 4-F | 4-F | 2-$^i$PrO | H |
| 88 | 4-F | 4-F | 2-$Et_2NCH_2CH_2O$ | H |
| 91 | 4-F | 4-F | 4-$CF_3$ | H |
| 92 | H | H | 4-$CF_3$ | H |
| 93 | 4-F | 4-F | 3-$CF_3$ | H |
| 94 | H | H | 3-$CF_3$ | H |
| 95 | 4-F | 4-F | 2-$CF_3$ | H |
| 96 | H | H | 2-$CF_3$ | H |

| | | | | |
|---|---|---|---|---|
| 97 | 4-F | 4-F | 2-$Me_2CHCH_2O$ | H |
| 98 | 4-F | 4-F | 2-EtO | H |
| 99 | H | H | 2-EtO | H |
| 100 | 4-F | 4-F | 2-OH,5-$NO_2$ | H |
| 101 | 4-F | 4-F | 2-CN | H |
| 102 | 4-F | 4-F | 3-CN | H |
| 103 | 4-F | 4-F | 4-CN | H |
| 104 | 4-F | 4-F | 4-Ph | H |
| 105 | 4-F | 4-F | 2,6-$Cl_2$ | H |
| 106 | 4-F | 4-F | 4-OH | H |
| 107 | 4-Cl | 4-Cl | 4-OH | H |
| 108 | 4-F | 4-F | 3-PhO | H |
| 109 | H | H | 3-PhO | H |
| 110 | 4-F | 4-F | 2,4-$(CF_3)_2$ | H |
| 111 | H | H | 4-Ph | H |
| 112 | 4-F | 4-F | 2-Ph | H |
| 113 | H | H | 2-Ph | H |
| 114 | 4-F | 4-F | 2-$NO_2$ | H |
| 115 | 4-F | 4-F | 3-$NO_2$ | H |
| 116 | 4-F | 4-F | 4-$NO_2$ | H |
| 117 | 4-Me | 4-Me | 2-MeO | H |
| 118 | 4-F | 4-F | 2,6-$(MeO)_2$ | H |
| 119 | 4-F | 4-F | 2-$F_2CHO$ | H |
| 120 | 4-F | 4-F | 2-$CF_3O$ | H |

| | | | | |
|---|---|---|---|---|
| 121 | H | H | 4-MeS | H |
| 122 | H | H | 4-$CO_2$Me | H |
| 123 | H | H | 4-$CO_2$H | H |
| 126 | H | H | 4-(PhCO$CH_2$OCO$CH_2$) | H |
| 127 | H | H | 4-$CH_2CO_2$H | H |
| 128 | H | H | 4-CON$H_2$ | H |
| 129 | 4-Me | 4-Me | 4-OH | H |
| 132 | 2-CH=CH-2' | | 4-OH | H |
| 134 | 2-O-2' | | 4-OH | H |
| 135 | 4-F | 4-F | 2-MeO,5-$NO_2$ | H |
| 136 | 4-F | 4-F | 2-MeO,5-$NH_2$ | H |
| 157 | 2-F | H | H | H |
| 158 | 2-Cl | 4-Cl | H | H |
| 159 | 4-F | 4-F | 2-MeO,3,5-($^t$Bu)$_2$ | H |
| 160 | 4-F | 4-F | 2-MeO,5-MeO | H |
| 161 | H | H | 2-MeO,3,5-($^t$Bu)$_2$ | H |
| 162 | 4-F | 4-F | 2-MeO,5-Br (+)-form | H |
| 163 | 4-F | 4-F | 2-MeO,5-Br (-)-form | H |
| 164 | 4-F | 4-F | 2-MeS | H |
| 165 | H | H | 2-MeS | H |
| 166 | 4-F | 4-F | 2-MeO,5-F | H |
| 167 | 4-F | 4-F | 2-MeSO | H |
| 168 | H | H | 2-MeO,5-$CF_3$O | H |

| | | | | |
|---|---|---|---|---|
| 169 | 4-F | 4-F | 2-MeO,5-$CF_3O$ | H |
| 170 | H | H | 2-MeO,6-EtO | H |
| 171 | 4-F | 4-F | 2-MeO,3,5-$Br_2$ | H |
| 172 | 4-F | 4-F | 4-F | 4-F-Ph |
| 173 | H | H | 2-MeO,3,5-$Cl_2$ | H |
| 174 | H | H | 2-MeO,3,5-$Br_2$ | H |
| 175 | H | H | 2-MeO,5-$^i$Pr | H |
| 176 | H | H | 2-MeO,5-$NO_2$ | H |
| 177 | H | H | 2-MeO,5-$NH_2$ | H |
| 178 | H | H | 2-MeO,5-N($SO_2$Me$)_2$ | H |
| 179 | H | H | 2-MeO,5-NHAc | H |
| 180 | H | H | 2-MeO,5 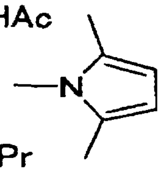 | H |
| 181 | 4-F | 4-F | 2-MeO,5-$^i$Pr | H |
| 182 | 4-F | 4-F | 2-EtO,5-Br | H |
| 183 | H | H | 2-MeO,5-NH$SO_2$Me | H |
| 184 | H | H | 2-OH,5-$NO_2$ | H |
| 185 | H | H | 2-EtO,5-$NO_2$ | H |
| 186 | H | H | 2-MeO,5-N($SO_2$Me)$CH_2CF_3$ | H |
| 187 | H | H | 2-MeO,5-$CH_2CO_2H$ | H |
| 188 | H | H | 2-MeO,5 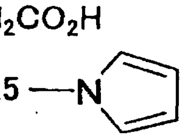 | H |
| 189 | H | H | 2-MeO,5-NHCO$CF_3$ | H |
| 190 | H | H | 2-$CF_3CH_2O$,5-$NO_2$ | H |
| 191 | H | H | 2-MeO,5-NH$SO_2$Et | H |
| 192 | H | H | 2-MeO,5-NH$SO_2$Pr | H |

| | | | | |
|---|---|---|---|---|
| 193 | H | H | 2-$^{i}$PrO,5-Br | H |
| 194 | 2-Br | H | H | H |

Table 3

| EXAMPLE No. | STRUCTURE |
|---|---|
| 2 | 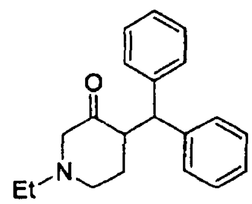 |
| 139 | 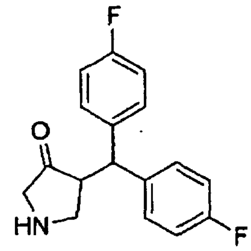 |
| 31 | 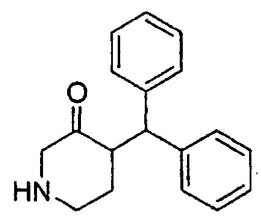 |
| 3 | 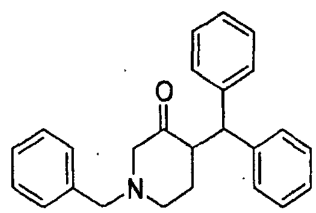 |
| 33 | 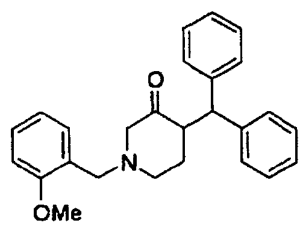 |
| 130 | 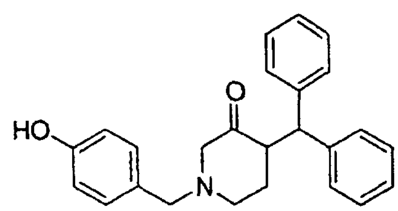 |

137

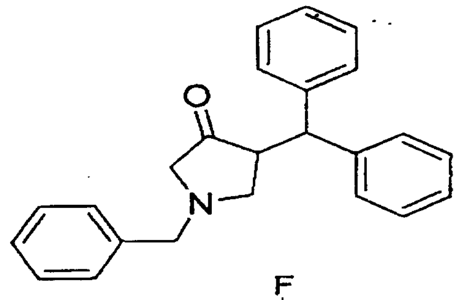

138

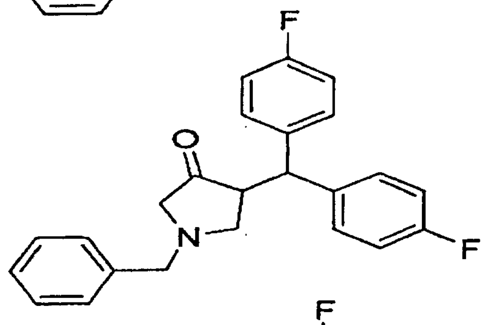

140

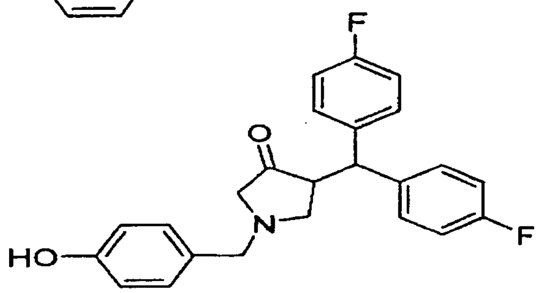

141

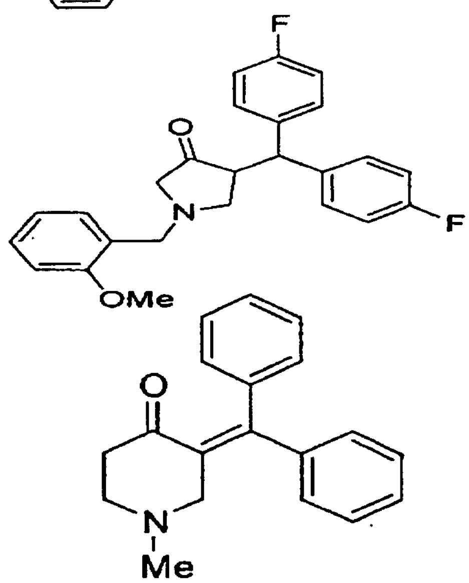

142

143

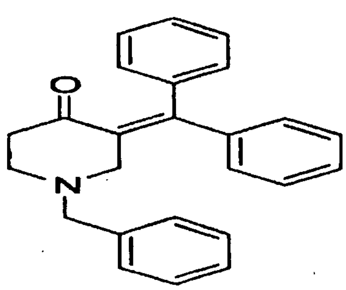

195 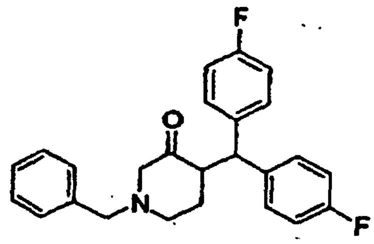

196 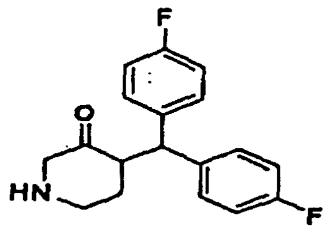

197 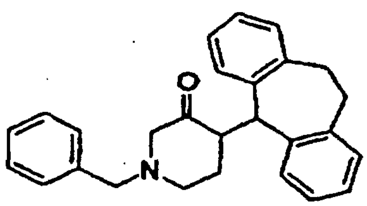

198 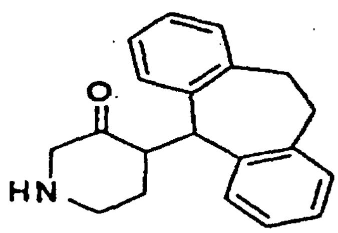

Preparation Example 1

| | | |
|---|---|---|
| (1) | Compound of Example 1 | 10 mg |
| (2) | Lactose | 60 mg |
| (3) | Corn starch | 35 mg |
| (4) | Hydroxypropylmethyl cellulose | 3 mg |
| (5) | Magnesium stearate | 2 mg |

A mixture of 10 mg of the compound obtained in Example 1, 60 mg of lactose and 35 mg of a corn starch is granulated using 0.03 ml of a 10% by weight aqueous solution of hydroxypropylmethyl cellulose (3 mg as hydroxypropylmethyl cellulose), dried at 40°C and sieved. The resultant granules are mixed with 2 mg of magnesium stearate and compressed. The resultant plane tablets are coated with sugar-coating of an aqueous suspension of sucrose, titanium dioxide, and talc and gum arabic. The coated tablets are polished with beeswax to obtain coated tablets.

According to the same manner, coated tablets are obtained except for using any of the compounds of Examples 2 to 200 instead of the compound of Example 1.

Preparation Example 2

| | | |
|---|---|---|
| (1) | Compound of Example 1 | 10 mg |
| (2) | Lactose | 70 mg |
| (3) | Corn starch | 50 mg |
| (4) | Soluble starch | 7 mg |
| (5) | Magnesium stearate | 3 mg |

A mixture of 10 mg of the compound obtained in Example 1 and 3 mg of magnesium stearate is granulated with 0.07 ml of an aqueous solution of a soluble starch (7 mg as soluble starch), dried, and mixed with 70 mg of lactose and 50 mg of corn starch. The mixture is compressed to obtain tablets.

According to the same manner, tablets are obtained except for using any of the compounds of Examples 2 to 200 instead of the compound of Example 1.

Reference Preparation Example 1

| | | |
|---|---|---|
| (1) | Rofecoxib | 5.0 mg |
| (2) | Salt | 20.0 mg |
| (3) | Distilled water | to total 2 ml |

A solution of 5.0 mg of rofecoxib and 20.0 mg of salt in distilled water is made up to 2.0 ml with addition of water. The solution is filtered, and filled in a 2 ml ampoule aseptically. The ampoule is sterilized, sealed to obtain a solution for injection.

Reference Preparation Example 2

| | | |
|---|---|---|
| (1) | Rofecoxib | 50 mg |
| (2) | Lactose | 34 mg |
| (3) | Corn starch | 10.6 mg |
| (4) | Corn starch (gelatinized) | 5 mg |
| (5) | Magnesium stearate | 0.4 mg |
| (6) | Calcium carboxymethyl cellulose | 20 mg |
| | | Total: 120 mg |

According to a conventional manner, the above components (1) to (6) were mixed and compressed with a tabletting machine to obtain tablets.

Preparation Example 4

The preparation obtained in the Preparation Example 1 or 2 is combined with the preparation obtained in the Reference Preparation Example 1 or 2.

Experiment 1

Radioligand receptor binding inhibiting activity (binding inhibitory activity using receptor from human lymphoblast cell (IM-9))

A modified method by M.A. Cascieri et al (Molecular Pharmacology, Vol.42, p.458 (1992)) was employed. The receptor was prepared from a human lymphoblast cell (IM-9). After inoculation of IM-9 cells ($2 \times 10^5$ cells/ml), the cells were cultured for 3 days (1L), and then the culture was centrifuged at 500 × G for 5 minutes to obtain a cell pellet. The resultant pellet was washed once with a phosphate buffer (FLOW LABORATORIES, CAT.No.28-103-05), fractured in 30 ml of a 50 mM Tris-HCl buffer (pH7.4) containing 120 mM sodium chloride, 5 mM potassium chloride, 2 μg/ml chymostatin, 40 μg/ml bacitracin, 5 μg/ml phosphoramidone, 0.5 mM phenylmethylsulfonyl fluoride and 1 mM ethylenediamine tetraacetic acid using a polytron homogenizer (Kinematika, Germany), and centrifuged at 40,000 × G for 20 minutes. The separated material was washed twice with 30 ml of the buffer solution described above and stored frozen as a receptor standard preparation (-80°C).

This preparation was suspended in a reaction buffer [50 mM Tris-HCl buffer (pH7.4), 0.02% bovine serum albumin, 1 mM phenylmethylsulfonyl fluoride, 2 μg/ml of chymostatin, 40 μg/ml bacitracin, 3 mM manganese chloride] at a protein concentration of 0.5 mg/ml and a 100 μl portion was used for the reaction. The sample, 125I-BHSP (0.46 KBq) was also added and the mixture was reacted in a 0.2 ml reaction buffer at 25°C for 30 minutes. A non-specific binding level was measured by adding substance P at $2 \times 10^{-6}$ M.

After the reaction, a Cell Harvester [290PHD manufactured by Cambridge Technology, Inc, USA] was used to conduct a rapid filtration over a glass filter [GF/B manufactured by Whatman, USA] whereby terminating the reaction, followed by washing three times with 250 μl of a 50 mM Tris-HCl buffer solution (pH7.4) containing 0.02% bovine serum albumin. Then, the radioactivity remaining on the filter was counted with a gamma counter. The filter was used after immersing in a 0.1% polyethylene imine over a whole day and night followed by drying in air.

The antagonistic activity of the compound obtained in each Example was calculated as a drug level required for accomplishing a 50% inhibition under the conditions specified above ($IC_{50}$ value), and the results are shown in Table 4.

Table 4

| Example No. | $IC_{50}$ value (nM) |
|---|---|
| 98 | 0.85 |
| 99 | 0.44 |
| 163 | 0.62 |
| 168 | 0.34 |
| 175 | 0.42 |
| 183 | 0.26 |
| 185 | 0.29 |

The radioligand means [$^{125}$I]-labeled substance P.

As seen from Table 4, each compound of the present invention was revealed to have excellent substance P receptor antagonistic activity.

Industrial Applicability

The compound (I) of the present invention or its salt or prodrug has high tachykinin receptor antagonistic activity, especially high substance P receptor antagonistic activity, and low toxicity, thus being employed safely as a medicine. Accordingly, the compound (I) of the present invention or its salt or prodrug is useful as a pharmaceutical composition, for example, a tachykinin receptor antagonist, an abnormal urination improving agent and the like.

**Claims**

1. A compound represented by the formula (I):

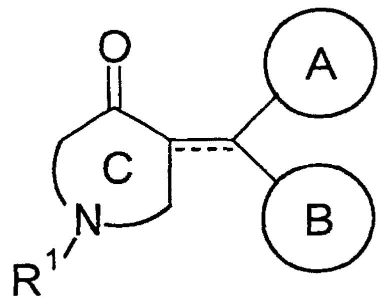

(I)

wherein each of rings A and B represents an optionally substituted aromatic ring, or rings A and B may be bonded to each other through linking between bonds or substituents thereof to form a ring; ring C represents a nitrogenous saturated heterocyclic ring optionally having one or more substituents besides the oxo (provided that 2,3-dioxopyrrolidine ring is excluded); $R^1$ represents a hydrogen atom, an optionally substituted hydrocarbon group or an optionally substituted heterocyclic group; and

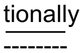

represents a single bond or a double bond, or a salt thereof.

**2.** The compound according to claim 1, wherein each of rings A and B represents an optionally substituted benzene ring, or the substituents on rings A and B may be taken together to form a dibenzotricyclic ring.

**3.** The compound according to claim 1, which is represented by the formula (Ia):

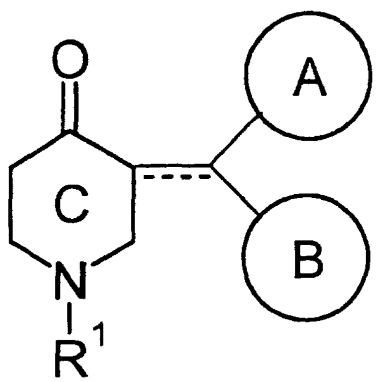

(Ia)

wherein each symbol is as defined in claim 1, or a salt thereof.

**4.** The compound according to claim 1, which is represented by the formula (Ib):

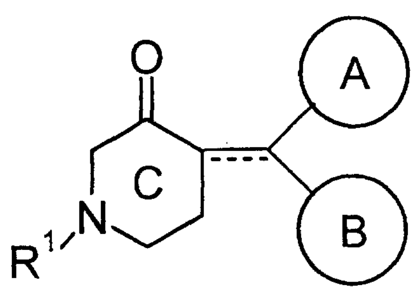

(Ib)

wherein each symbol is as defined in claim 1 or a salt thereof.

**5.** The compound according to claim 1, which is represented by the formula (Ic):

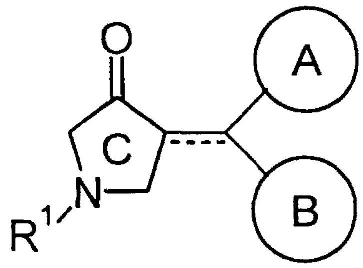

(Ic)

wherein each symbol is as defined in claim 1, or a salt thereof.

**6.** The compound according to claim 1, wherein each of rings A and B is a benzene ring which may have one to three substituents selected from a halogen atom, a $C_{1-6}$ alkyl group and a $C_{1-6}$ alkoxy group, or the ring formed by binding between the bonds or substituents of rings A and B is a dibenzsuberane ring, dibenzosuberene ring, xanthene ring or thioxanthene ring.

**7.** The compound according to claim 1, wherein $R^1$ is an optionally substituted benzyl.

**8.** A prodrug of the compound according to claim 1, or a salt thereof.

**9.** A process for producing the compound according to claim 1, which comprises reacting a compound represented by the formula (II):

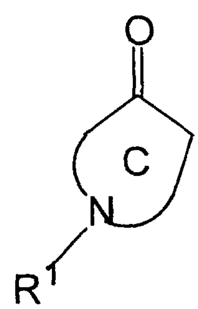

(II)

wherein each symbol is as defined in claim 1, or a salt thereof with a compound represented by the formula (III):

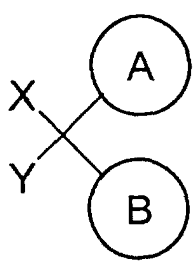

(III)

wherein each of X and Y represents a hydrogen atom, a hydroxyl group or a halogen atom, and other symbols are as defined in claim 1, provided that, when X is a hydrogen atom, then Y represents a hydroxyl group or a halogen atom, while X is a halogen atom, then Y is a halogen atom, or a reactive derivative or a salt thereof.

**10.** A pharmaceutical composition comprising the compound according to claim 1, or a salt or a prodrug thereof.

**11.** The pharmaceutical composition according to claim 10, which is a tachykinin receptor antagonist.

**12.** The pharmaceutical composition according to claim 11, which is an agent for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder.

**13.** A method for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder, which comprises administering an effective amount of the compound according to claim 1, or a salt or a prodrug to thereof to a mammal.

**14.** Use of the compound according to claim 1, or a salt or a prodrug thereof for manufacturing an agent for preventing or treating pollakiuria, incontinence of urine, asthma, rheumatoid arthritis, osteoarthritis, pain, cough, itching, chronic obstructive pulmonary disease, irritable bowel disease, vomiting, depression, anxiety neurosis, obsessive-compulsive neurosis, panic disorder, manic-depressive psychosis, schizophrenia, mania, migraine, cancer, HIV infection, cardiovascular disorder, solar dermatitis, hypogonadism, ataxia, cognitive disorder or circadian rhythm disorder.

## INTERNATIONAL SEARCH REPORT

International application No.
PCT/JP02/13581

A. CLASSIFICATION OF SUBJECT MATTER
Int.Cl$^7$ C07D207/24, 211/74, 401/06, 405/04, 405/06, 409/04, 409/06, 413/06, 417/04, 211/86, A61K31/4015, 31/45, 31/451, 31/4525, 31/453, 31/4535, 31/454, 31/4545, 31/4709, A61P1/00, 1/08, 9/00, 11/00,
According to International Patent Classification (IPC) or to both national classification and IPC

B. FIELDS SEARCHED
Minimum documentation searched (classification system followed by classification symbols)
Int.Cl$^7$ C07D207/24, 211/74, 401/06, 405/04, 405/06, 409/04, 409/06, 413/06, 417/04, 211/86, A61K31/4015, 31/45, 31/451, 31/4525, 31/453, 31/4535, 31/454, 31/4545, 31/4709

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X | WO 99/43323 A (FOX CHASE CANCER CENTER), 02 September, 1999 (02.09.99), Page 11 & US 6248752 B | 1,2,6,9,10 |
| A | JP 4-103570 A (Pfizer Inc.), 06 April, 1992 (06.04.92), Full text & EP 436334 A | 1-12,14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 February, 2003 (07.02.03) | 25 February, 2003 (25.02.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13581

**Box I Observations where certain claims were found unsearchable (Continuation of item 2 of first sheet)**

This international search report has not been established in respect of certain claims under Article 17(2)(a) for the following reasons:

1. [X] Claims Nos.: 13
because they relate to subject matter not required to be searched by this Authority, namely:
Claim 13 pertains to a method for treatment of the human body by therapy.

2. [X] Claims Nos.: 8, 10, and 14
because they relate to parts of the international application that do not comply with the prescribed requirements to such an extent that no meaningful international search can be carried out, specifically:
(See extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/13581

Continuation of A. CLASSIFICATION OF SUBJECT MATTER
(International Patent Classification (IPC))

Int.Cl$^7$ 11/06, 11/14, 13/02, 15/00, 15/10, 17/04, 17/06, 17/16, 19/02, 25/00, 25/02, 25/04, 25/06, 25/14, 25/18, 25/20, 25/22, 25/24, 29/00, 31/18, 35/00, 43/00, 25/28

(According to International Patent Classification (IPC) or to both national classification and IPC)

Continuation of Box No.I-2 of continuation of first sheet(1)

The term "prodrug" used in the claims is unclear as to what structure is implied, even when the statements in the description are investigated. This term hence makes the scope of the compounds and medicines of the invention unclear.

Consequently, claims 8, 10, 13, and 14 and the description do not comply with the given requirements to such a degree that a meaningful international search can be made.

In this international search report, a search was hence made through prior art documents with respect to the compounds specified in the description.

Form PCT/ISA/210 (extra sheet) (July 1998)